(19) Europäisches Patentamt
European Patent Office
Office européen des brevets

(11) Publication number: 0 341 713 A2

# (12) EUROPEAN PATENT APPLICATION

(21) Application number: 89108487.3

(22) Date of filing: 11.05.89

(51) Int. Cl.4: C07C 69/92 , C09K 19/20 , G02F 1/13 , C07C 69/82 , C07C 69/76 , C07D 239/34 , C07C 69/96 , C09K 19/12 , C09K 19/30 , C09K 19/34 , C07C 43/315

(30) Priority: 12.05.88 JP 115619/88
01.06.88 JP 136256/88

(43) Date of publication of application:
15.11.89 Bulletin 89/46

(84) Designated Contracting States:
CH DE FR GB LI

(71) Applicant: HITACHI, LTD.
6, Kanda Surugadai 4-chome
Chiyoda-ku Tokyo 101(JP)

Applicant: TAKEDA CHEMICAL INDUSTRIES, LTD.
3-6, Doshomachi 2-chome Chuo-ku
Osaka(JP)

(72) Inventor: Matsumura, Koichi
12-9, Teradacho
Ibaraki-shi(JP)
Inventor: Kawada, Mitsuru
25-3, Tsukaguchicho-4-chome
Amagasaki-shi(JP)
Inventor: Iida, Kouichi
C29-903, 1, Takemidai-3-chome
Suita-shi(JP)
Inventor: Yamashita, Toshiro
2-103, 21, Kofudai Toyonocho
Toyono-guni Osaka(JP)
Inventor: Kondo, Katsumi
19-21, Aobacho
Katsuta-shi(JP)
Inventor: Mukoh, Akio
498-21, Kasaharacho
Mito-shi(JP)
Inventor: Kitamura, Teruo
3600-234, Nakane,
Katsuta-shi(JP)

(74) Representative: Patentanwälte Beetz sen. - Beetz jun. Timpe - Siegfried - Schmitt-Fumian- Mayr
Steinsdorfstrasse 10
D-8000 München 22(DE)

## (54) Optically active compounds, liquid crystal compositions comprising said compounds, and liquid crystal optical modulators using said compositions.

(57) The invention relates to optically active compounds represented by the general formula I

$$R_1-Q_1-M-Q_2-(CH_2)_m-\overset{*}{\underset{\displaystyle R_2}{CH}}-\overset{*}{\underset{\displaystyle R_3}{CH}}-(CH_2)_n-Q_3-R_4 \qquad [I]$$

wherein m, n, $R_1$, $R_2$, $R_3$, $R_4$, $Q_1$, $Q_2$, $Q_3$, and M are defined as in the specification, methods and intermediates for their preparation, liquid crystal compositions comprising at least one optically active

EP 0 341 713 A2

compound of formula I and their use in electrooptical display, switching and modulation devices.

# OPTICALLY ACTIVE COMPOUNDS, LIQUID CRYSTAL COMPOSITIONS COMPRISING SAID COMPOUNDS, AND LIQUID CRYSTAL OPTICAL MODULATORS USING SAID COMPOSITIONS

## BACKGROUND OF THE INVENTION

### FIELD OF THE INVENTION

The present invention relates to optically active compounds and liquid crystal compositions comprising these compounds. The compounds and compositions of the present invention show a ferroelectric liquid crystal phase, and accordingly are useful as electrooptic switching elements such as liquid crystal display devices or the like, and can be used in liquid crystal optical modulators.

### RELATED ART STATEMENT

Liquid crystal display devices have various excellent features such as low-voltage operability, low power consumption, being thin and light-weight, being a non-emissive type and easy on the eye, etc. Accordingly, they are in wide use as various display devices.

Liquid crystal display devices using a nematic liquid crystal operating in the so-called twisted nematic mode (TN mode) are in use currently. However, display devices using this kind of nematic liquid crystals have the drawback of being very slow in response as compared to luminescent type display devices such as CRT, EL and the like. Consequently, when such display devices are applied in a display device, particularly a large scale display device capable of displaying a large amount of information, it is impossible to obtain a display of good contrast. Thus the liquid crystal display devices using a nematic liquid crystal have only a very limited applicability. There has recently been developed a liquid crystal display device using a nematic liquid crystal operating in the so-called super twisted nematic mode (STN mode) or SBE and capable of giving a display of improved contrast. Even in this STN mode liquid crystal display device, however, the response is not sufficient, and therefore said device finds also only a very limited application, because it cannot be used for displays capable of displaying a still larger amount of information. Hence, various attempts have been made to develop a new liquid crystal display system giving an excellent response.

Ferroelectric liquid crystals have a memory characteristics and give a high speed response, and accordingly their application to large scale displays is highly expected. As liquid crystals having ferroelectric properties, there are known those showing a chiral smectic C phase, a chiral smectic H phase, a chiral smectic J phase, etc. Of these ferroelectric liquid crystals, those showing a chiral smectic C phase are thought to have highest practical utility.

Ferroelectric liquid crystals showing a chiral smectic C phase were first synthesized in 1975 by R. B. Meyer et al.; one typical example thereof is 2-methylbutyl 4-(4′-n-decyloxybenzylideneamino)cinnamate (hereinafter abbreviated to DOBAMBC) [J. Physique, 36, L69 (1975)].

A thin film liquid crystal cell was prepared using DOBAMBC and was found to have a high speed response in the order of $\mu$s [N.A. Clark et al., Appl. Phys. Lett., 36, 89 (1980)]. Since that time, there was started the development of optical modulation devices (e.g. liquid crystal devices, photo-printer heads) using a ferroelectric liquid crystal showing a chiral smectic C phase (hereinafter may be referred to simply as "ferroelectric liquid crystal").

As a result, a number of ferroelectric liquid crystal compounds showing a chiral smectic C phase have been developed since then, and various ferroelectric liquid crystal compounds are already known. However, no ferroelectric liquid crystal compound is found yet which has satisfactory reliability and capability for use in display devices, particularly large scale displays, etc.

In order for a ferroelectric liquid crystal to be practically used in a liquid crystal display device, etc., the liquid crystal must be superior in high speed response, orientation, memory characteristics, characteristics of threshold voltage, temperature dependences of these properties, etc. Also, the ferroelectric liquid crystal is required to show a chiral smectic C phase over a wide temperature range so that it can operate within a sufficiently wide temperature range including room temperature, and further to have excellent physical and chemical stability.

In order for a ferroelectric liquid crystal to have, in particular, excellent physical and chemical stability, good high speed response and good memory characteristics, the liquid crystal must have a large

spontaneous polarization. Further, in order to be able to reliably operate within a wide temperature range, the liquid crystal must show a chiral smectic C phase over a sufficiently wide temperature range.

Among the so far developed ferroelectric liquid crystals, no compound is found yet which satisfies all the above requirements. For example, the above mentioned DOBAMBC shows a chiral smectic C phase over a relatively wide temperature range but, being a liquid crystal of Schiff's base type, is insufficient in chemical stability to water, light, etc., and moreover, has a small spontaneous polarization of 4 $nC/cm^2$ or below.

Ester type liquid crystals are reported as being ferroelectric liquid crystals which are chemically stable. However, these liquid crystals are not satisfactory because they have no sufficiently large spontaneous polarization and no sufficiently wide temperature range of chiral smectic C phase.

In order to obtain a large spontaneous polarization, there were synthesized compounds having two asymmetric carbon atoms as an optically active group essential for the expression of a chiral smectic C phase.

These compounds include, for example, liquid crystal compounds having a dichiral epoxide side chain [David M. Walba et al., Journal of American Chemical Society, 108, 7424 (1986)], and liquid crystal compounds having a halogen atom and a methyl group on two adjacent asymmetric carbon atoms [cf. e.g. JP-A-168780/1985, 218358/1985, 68449/1986, 40/1987, 46/1987, 103043/1987, 111950/1987, 142131/1987, 175443/1987].

A typical example of the above liquid crystal compounds is 4′-octylcarbonyloxy-4-biphenyl (s)-3-methyl-2-chloropentanoate [JP-A-68449/1986]. This liquid crystal compound has a very large spontaneous polarization of 180 $nC/cm^2$, but has no sufficiently wide temperature range of chiral smectic C phase and moreover, being an aliphatic chloro compound, has poor chemical stability. Hence, there was synthesized 4′-octylcarbonyloxy-4-[(s)-2-methoxy-(s)-3-methylpentyloxycarbonyl]biphenyl [JP-A-228036/1987]. This compound has excellent chemical stability and has a relatively large spontaneous polarization of 17 $nC/cm^2$, but has no sufficiently wide temperature range of chiral smectic C phase.

## SUMMARY OF THE INVENTION

The present inventors made investigation in order to find out a ferroelectric liquid crystal compound having excellent physical and chemical stabilities and a large spontaneous polarization and have reached the present invention. That is, the present inventors made investigation on liquid crystal compounds wherein a chemically stable ester compound and an optically active group having two asymmetric carbon atoms are combined, and have reached the present invention. Specifically, the present inventors found that when an optically active group having two asymmetric carbon atoms is bonded to a six-membered ring (e.g. benzene ring) constituting the skeleton of a conventional liquid crystal compound, the pattern of the bonding has a close connection to the spontaneous polarization of the resulting liquid crystal compound, and consequently the present invention has been achieved.

## BRIEF DESCRIPTION OF DRAWING

Fig. 1 is a schematic illustration of an example of a liquid crystal display device using the liquid crystal compositions of the present invention.

## DETAILED DESCRIPTION OF PREFERRED EMBODIMENTS

The first aspect of the present invention is directed to optically active compounds represented by the general formula I:

$$R_1-Q_1-M-Q_2-(CH_2)_m-\underset{\underset{R_2}{|}}{\overset{*}{C}H}-\underset{\underset{R_3}{|}}{\overset{*}{C}H}-(CH_2)_n-Q_3-R_4 \qquad [I]$$

[$R_1$ is an alkyl group of 3-14 carbon atoms; $R_2$ and $R_3$, which may be the same or different, are independently a lower alkyl group of 1-3 carbon atoms or a lower alkoxy group of 1-3 carbon atoms; $R_4$ is an alkyl group of 1-10 carbon atoms which may be substituted with a phenyl group which may be substituted with a lower alkyl or alkoxy group of 1-4 carbon atoms; m and n are independently 0 or 1; $Q_1$ is a single bond, an ether group, a carbonyl group, a carbonyldioxy group or a carboxylic acid ester group; $Q_2$ is a single bond, an ether group, a carbonyl group or a carboxylic acid ester group; $Q_3$ is an ether group, a carbonyl group or a carboxylic acid ester group; M is

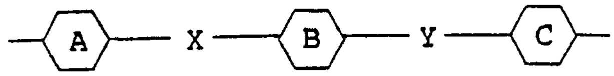 or

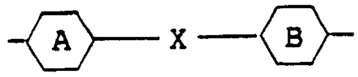

(X and Y are independently a single bond, a carboxylic acid ester group, a methyleneoxy group or an ethylene group, and

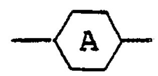 , 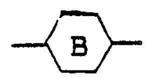 and

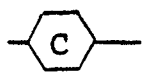

are independently a homocyclic or heterocyclic six-membered ring-1,4-diyl group which may contain 1-2 oxygen or nitrogen atoms as ring-forming atoms); when m is 0, $R_2$ is a lower alkoxy group of 1-3 carbon atoms, $R_4$ is an alkyl group of 1-10 carbon atoms, and $Q_2$ is a single bond, a carbonyl group or a carboxylic acid ester group represented by

$-O-\overset{O}{\overset{\|}{C}}-$ ; the carbon atoms with the asterisk (*) denote asymmetric carbon atoms]; preferably to optically active compounds of the general formula I wherein $R_2$ is a lower alkoxy group of 1-3 carbon atoms, $R_4$ is an alkyl group of 1-10 carbon atoms, m and n are both 0, $Q_1$ is a single bond, an ether group, a carbonyl group or a carboxylic acid ester group, and $Q_2$ is a carbonyl group or a carboxylic acid ester group represented by

$-O-\overset{O}{\overset{\|}{C}}-$ ; or, m is 1, n is 0 or 1, $Q_2$ and $Q_3$, which may be the same or different, are independently an ether group, a carbonyl group or a carboxylic acid ester group, M is

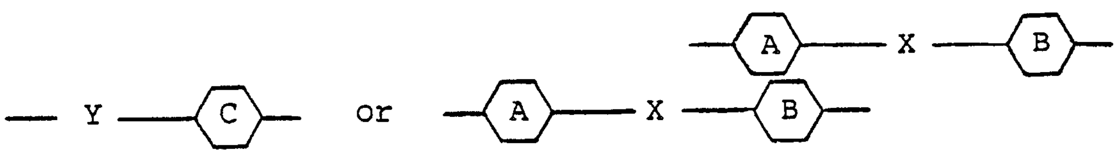

(X and Y are independently a single bond, a carboxylic acid ester group, a methyleneoxy group or an ethylene group, and

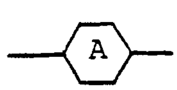 , 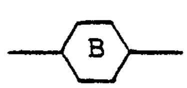 and 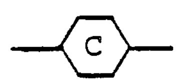

are independently a homocyclic or heterocyclic six-membered ring-1,4-diyl group which may contain 1-2 oxygen or nitrogen atoms as ring-forming atoms), and the carbon atoms with the asterisk (*) denote asymmetric carbon atoms; more preferably to optically active compounds of the general formula I wherein m is 1, $R_2$ is a lower alkyl or alkoxy group of 1-3 carbon atoms, $R_3$ is a lower alkyl group of 1-3 carbon atoms and $Q_3$ is an ether group, or, m is 1, $R_2$ and $R_3$ are independently a lower alkoxy group of 1-3 carbon atoms, n is 1 and $Q_2$ is an ether group, or, m is 0, $R_3$ is a lower alkyl or alkoxy group or 1-3 carbon atoms, $Q_2$ is a carboxylic acid ester group represented by

$-O-\overset{O}{\overset{\|}{C}}-$ , and $Q_3$ is an ether group when $R_3$ is an alkyl group, and is a carboxylic acid ester group when $R_3$

is an alkoxy group.

The second and third aspects of the present invention are directed to liquid crystal compositions comprising at least one of the optically active compounds of the general formula I and liquid crystal optical modulators using at least one of the liquid crystal compositions, respectively.

The compounds I according to the present invention can be classified into the following compounds I′ and I″ depending upon the basic skeleton M.

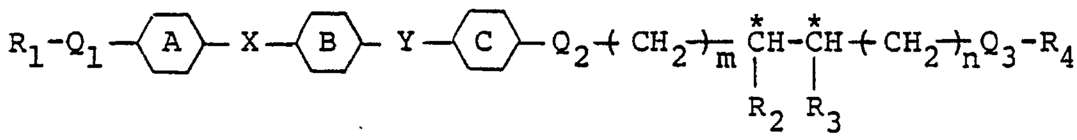

[I']

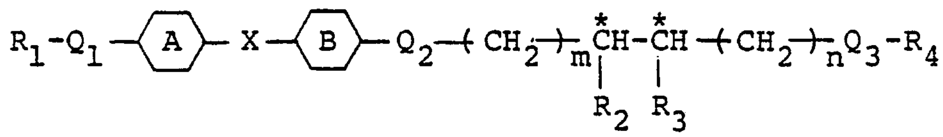

[I"]

In the above compounds I, I′ and I″, the alkyl group of 3-14 carbon atoms represented by $R_1$ can be of straight chain or branched chain. Specifically, there can be mentioned straight chain alkyl groups such as propyl, butyl, pentyl, hexyl, heptyl, octyl, nonyl, decyl, undecyl, dodecyl, tridecyl, tetradecyl and the like (hereinafter, the straight chain alkyl groups are sometimes expressed only by the common names without using the prefix "n-") as well as branched chain alkyl groups such as isopropyl, isobutyl, sec-butyl, tert-butyl, isopentyl, neopentyl, tert-pentyl, isohexyl, 1-methylpentyl, 2-methylpentyl, 5-methylhexyl, 2,3,5-trimethylhexyl, 2,7,8-trimethyldecyl, 4-ethyl-5-methylnonyl and the like. Of these, preferable are straight chain alkyl groups of 6-12 carbon atoms, such as hexyl, heptyl, octyl, decyl, undecyl, dodecyl and the like. As the lower alkyl groups of 1-3 carbon atoms represented by $R_2$ and $R_3$, there can be mentioned straight or branched chain alkyl groups such as methyl, ethyl, propyl, isopropyl and the like. Of these, methyl is preferable. Further as the lower alkoxy groups of 1-3 carbon atoms represented by $R_2$ and $R_3$, there can be mentioned straight or branched chain alkoxy groups such as methoxy, ethoxy, propoxy, isopropoxy and the like. Of these, methoxy is preferable. The alkyl group of 1-10 carbon atoms represented by $R_4$ can be of straight chain or branched chain. Specifically, there can be mentioned straight chain alkyl groups such as methyl, ethyl, propyl, butyl, pentyl, hexyl, heptyl, octyl, nonyl, decyl and the like, as well as branched chain alkyl groups such as isopropyl, isobutyl, sec-butyl, tert- butyl, isopentyl, neopentyl, tert-pentyl, isohexyl, 1-methylpentyl, 2-methylpentyl, 5-methylhexyl, 4-ethylhexyl, 2,3,5-trimethylhexyl, 4-ethyl-5-methylhexyl and the like. Of these, preferable are straight chain alkyl groups of 1-8 carbon atoms such as methyl, ethyl, propyl, butyl, pentyl, heptyl and octyl. The alkyl groups represented by $R_4$ may be substituted with a phenyl group. The phenyl group may be substituted with a lower alkyl or alkoxy group of 1-4 carbon atoms. As such a phenyl group, there can be mentioned, for example, 4-methylphenyl, 4-ethylphenyl, 4-propylphenyl, 4-isopropylphenyl, 4-butylphenyl, 4-isobutylphenyl, 4-tert-butylphenyl, 4-methoxyphenyl, 4-ethoxyphenyl, 4-propoxyphenyl, 4-isopropoxyphenyl, 4-butoxyphenyl and 4-isobutoxyphenyl. As the carboxylic acid ester groups represented by $Q_1$, $Q_2$ and $Q_3$, there can be mentioned an ester group represented by $-\overset{O}{\overset{\|}{C}}-O-$ and an ester group represented by $-O-\overset{O}{\overset{\|}{C}}-$.

With respect to the various bond and groups which can be taken by $Q_1$, $Q_2$ and $Q_3$, it is preferable that $Q_1$ be an ether group, $Q_2$ be a $-\overset{O}{\overset{\|}{C}}-O-$ ester group or an ether group when m is 1, and be a $-O-\overset{O}{\overset{\|}{C}}-$ ester group when m is 0, and $Q_3$ be an ether bond or a carboxylic acid ester group.

As the carboxylic acid ester groups represented by X and Y, there can be mentioned a $-\overset{O}{\overset{\|}{C}}-O-$ester group and a

$-O-\overset{O}{\overset{\|}{C}}-$ ester group. As the methyleneoxy group represented by X and Y, there can be mentioned $-CH_2-O-$ and $-OCH_2-$.

As the six-membered ring-1,4-diyl groups represented by

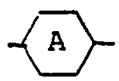 , 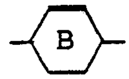 and 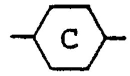 ,

there can be specifically mentioned, for example, p-phenylene, 1,4-cyclohexylene, 2,5-(1,3-dioxane)diyl, 2,5-pyridinediyl, 2,5-pyrimidinediyl, 2,5-(1,4-pyrazine)diyl and 3,6-(1,2-pyridazine)diyl. These rings may be substituted with a halogen, cyano, methyl and or methoxy group, etc.

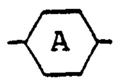 , 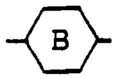 and 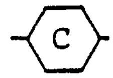

may be the same or different.

2,5-(1,3-Dioxane)diyl can be

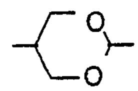 or

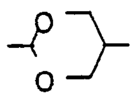 ; 2,5-pyridinediyl can be 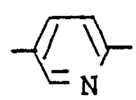 or 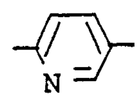 ;

2,5-pyrimidinediyl can be 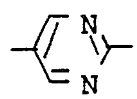 or 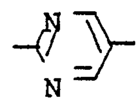 .

When M is

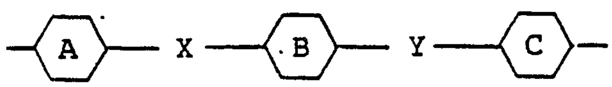 ,

preferable combinations of A, B, C, X and Y include the case where one of X and Y is a single bond, the other of them is a carboxylic acid ester bond, and all of

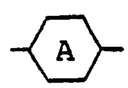 , 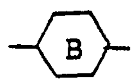 and 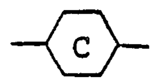

and are p-phenylene. When M is

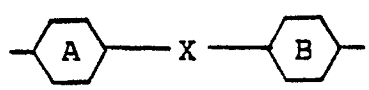 ,

preferable combinations of A, B and X include the case where X is a single bond and both of

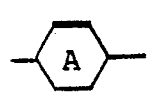 and 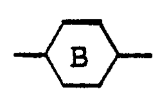 ,

and are p-phenylene.

The compounds I have two asymmetric carbon atoms within the molecule and therefore have four

different optical isomers, that is, (R,R) type, (R,S) type, (S,R) type and (S,S) type.

The optically active compounds I of the present invention can be produced according to, for example, the following processes.

Process 1 Compounds of the general formula I wherein $Q_2$ is a $-\overset{O}{\overset{\|}{C}}-O-$ ester group.

Scheme 1

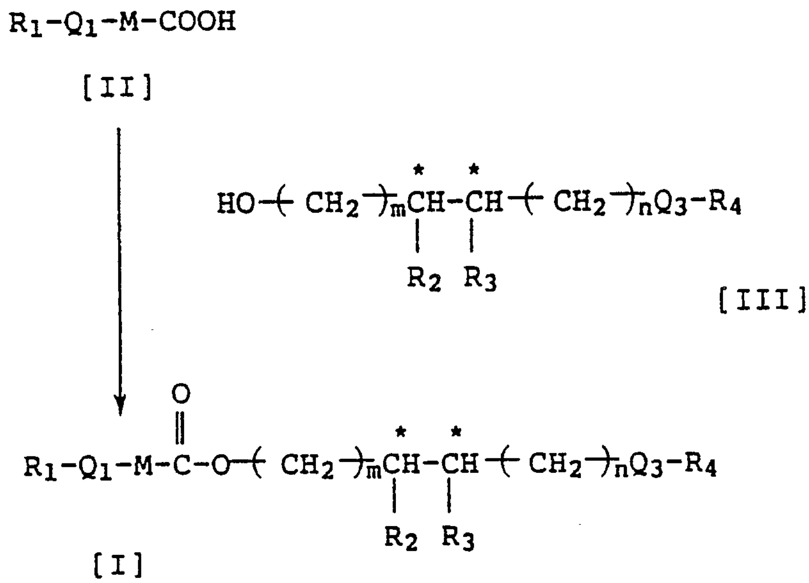

[II] [III] [I]

(In the above scheme, $R_1$ to $R_4$, m, n, $Q_1$, $Q_3$ and M have the same definitions as given above. The same applies hereinafter.)

As shown in the above scheme 1, the compound I can be obtained by subjecting a carboxylic acid II and an optically active dichiral alcohol III to a condensation reaction. This condensation reaction per se is known and can be effected according to a conventional method. For example, the carboxylic acid II and the dichiral alcohol III are condensed in an organic solvent in the presence of a proton acid. As the proton acid, there can be used, for example, inorganic acids such as sulfuric acid, hydrochloric acid, perchloric acid and the like; organic sulfonic acids such as p-toluenesulfonic acid, benzenesulfonic acid, trifluoromethanesulfonic acid, methanesulfonic acid and the like; and strongly acidic ion exchange resins such as Amberlist® and the like. As the organic solvent, there can be mentioned, for example, hydrocarbons such as hexane, benzene, toluene and the like, halogenated hydrocarbons such as chloroform, methylene chloride, carbon tetrachloride, 1,2-dichloroethane and the like, ethers such as diethyl ether, tetrahydrofuran, dioxane and the like, ethyl acetate, acetonitrile and dimethylformamide. It is possible that the carboxylic acid II be converted to an acid halide with, for example, a halogenating agent such as phosphorus pentachloride, thionyl chloride, thionyl bromide or the like and the halide be reacted with the dichiral alcohol III in the above mentioned organic solvent in the presence of, for example, a tertiary amine such as pyridine, triethylamine or the like. It is further possible that the alcohol III be converted to a trimethylsilyl ether derivative

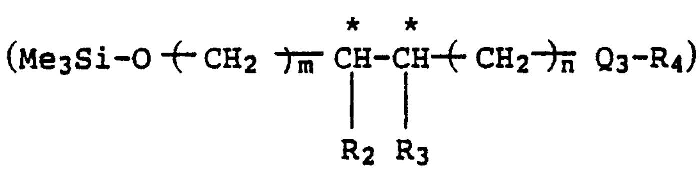

and the ether be condensed with an acid halide derivative of the carboxylic acid II in the presence of a Lewis acid such as zinc chloride or the like.

It is furthermore possible that the carboxylic acid II and the alcohol III be reacted with an activating agent such as N,N′-dicyclohexylcarbodiimide (DCC), Mukaiyama's reagent illustrated by a 1-methyl-2-halopyridium iodide, diethyl azodicarboxylate (DEAD) and triphenylphosphine ($Ph_3P$) (Mitsunobu's reagent), triphenylphosphine dibromide and the like.

These methods are described in, for example, J. Org. Chem., 27, 4675 (1962); Tetrahedron Lett., 1978, 4475; Chemistry Lett., 1975, 1045; Chemistry Lett., 1976, 13; Bull. Chem. Soc. Japan, 50, 1863 (1977); Bull. Chem. Soc. Japan, 40, 2380 (1967); Syn. Commun., 16, 1423 (1986); and Syn. Commun., 16, 659 (1986).

Process 2 Compounds of the general formula I wherein $Q_2$ is an ether group.

Scheme 2

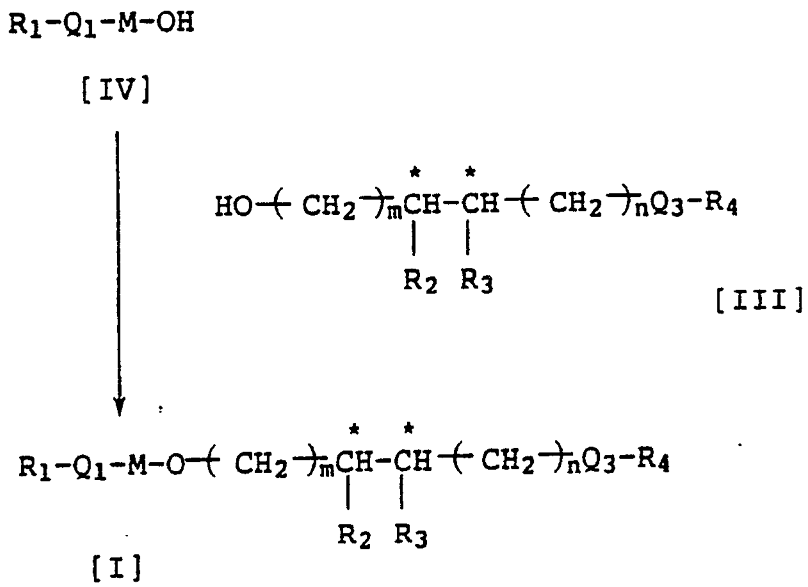

[IV]

[III]

[I]

As shown in the above scheme 2, the compound I can be obtained by subjecting a hydroxyl group-containing compound IV and an optically active dichiral alcohol III to an etherification reaction. This etherification reaction per se is known and can be effected according to a conventional method. For example, the reaction can be effected with diethyl azodicarboxylate (DEAD) and triphenylphosphine ($Ph_3P$) (S. Bittner et al., Chem. & Ind., 1975, 281).

It is also possible that the dichiral alcohol III and an organic sulfonyl chloride be reacted in an organic solvent in the presence of an organic base (e.g. pyridine, triethylamine) or an inorganic base (e.g. sodium hydride) to obtain a corresponding organic sulfonic acid ester and the ester be reacted with the hydroxyl gorup-containing compound IV. This reaction is conducted in an organic solvent in the presence of an inorganic base (e.g. potassium carbonate, sodium hydride) or an organic base (e.g. pyridine, triethylamine). As the organic sulfonyl chloride usable in the reaction, there can be mentioned, for example, aromatic sulfonyl chlorides such as p-toluenesulfonyl chloride, o-toluenesulfonyl chloride, p-chlorobenzenesulfonyl chloride, benzenesulfonyl chloride, α-naphthalenesulfonyl chloride, β-naphthalenesulfonyl chloride and the like, as well as aliphatic sulfonyl chlorides such as methanesulfonyl chloride, trifluoromethanesulfonyl chloride and the like.

As the organic solvent usable in the etherification reaction, there can be mentioned, for example, aliphatic hydrocarbons (e.g. hexane, cyclohexane), halogenated hydrocarbons (e.g. chloroform, methylene chloride, carbon tetrachloride, 1,2-dichloroethane), ethers (e.g. diethyl ether, tetrahydrofuran, dioxane), aromatic hydrocarbons (e.g. benzene, toluene), ethyl acetate, acetonitrile, dimethylformamide (DMF), dimethyl sulfoxide (DMSO) and hexamethylphosphoric triamide (HMPA).

In the etherification reaction, the activation of the dichiral alcohol III can be effected not only by converting the dichiral alcohol III to the above mentioned organic sulfonic acid ester but also by converting the alcohol III to a halogen derivative. The conversion to a halogen derivative can be effected by, for example, reacting the organic sulfonic acid ester with a metal halide (e.g. sodium iodide, potassium iodide). Or, the dichiral alcohol III can be directly reacted with a halogenating agent such as phosphorus pentachloride, thionyl chloride, thionyl bromide or the like. The thus obtained halogen derivative can be reacted with the hydroxyl group-containing compound IV in an organic solvent in the presence of the above mentioned inorganic or organic base.

<u>Process 3</u> Compounds of the general formula I wherein $Q_2$ is a

$-O-\overset{O}{\overset{\|}{C}}-$ ester group.

Scheme 3

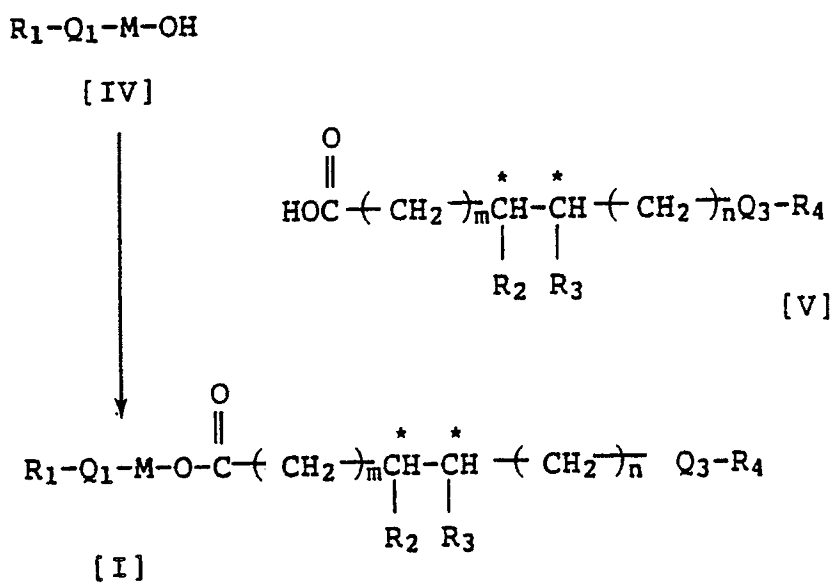

[IV] [V] [I]

The optically active compound I can be synthesized from a hydroxyl group-containing compound IV and an optically active dichiral carboxylic acid V. This reaction is a condensation reaction and the reaction per se can be effected according to a known method. For example, the compound IV and the dichiral carboxylic acid V are reacted in an organic solvent inert to the reaction, in the presence of a proton acid at a low temperature, room temperature or an elevated temperature. As the solvent usable in the reaction, there can be mentioned the same solvents as in the Process 1, for example, hydrocarbons (e.g. hexane, benzene, toluene) and halogenated hydrocarbons.

The optically active compound I can also be produced by converting the optically active dichiral carboxylic acid V to an acid halide with a halogenating agent such as phosphorus pentachloride, thionyl chloride, thionyl bromide or the like and then subjecting the acid halide to an esterification reaction with the hydroxyl group-containing compound IV in an organic solvent in the presence of organic base (e.g. pyridine, triethylamine) at a low temperature, room temperature or an elevated temperature for a few minutes to a few hours, optionally a few days. The condensation reaction of the Process 3 can also be effected by appropriately selecting a method other than those mentioned above, such as a method using an activating agent [e.g. N,N′-dicyclohexylcarbodiimide (DCC), a Mukaiyama's reagent, i.e. a 1-methyl-2-halopyridium iodide, diethyl azodicarboxylate (DEAD) and triphenylphosphine (Mitsunobu's reagent), triphenylphosphine dibromide] and a method comprising converting the optically active carboxylic acid V to an acid halide with a halogenating agent (e.g. phosphorus pentachloride, thionyl chloride, thionyl bromide) and then condensing the halide with a trimethylsilyl ether derivative of the compound IV in the presence of a catalytic amount of

a Lewis acid (e.g. zinc chloride).

As in the case of the Process 1, these methods can be effected as per described in, for example, J. Org. Chem., 27, 4675 (1962); Tetrahedron Lett., 1978, 4475; Chemistry Lett., 1975, 1045; Chemistry Lett., 1976, 13; Bull. Chem. Soc. Japan, 50, 1863 (1977); Bull. Chem. Soc. Japan, 40, 2380 (1967); Syn. Comm., 16, 1423 (1986); and Syn. Comm., 16, 659 (1986).

Process 4 Compounds of the general formula I wherein $Q_2$ is a carbonyl group.

Scheme 4

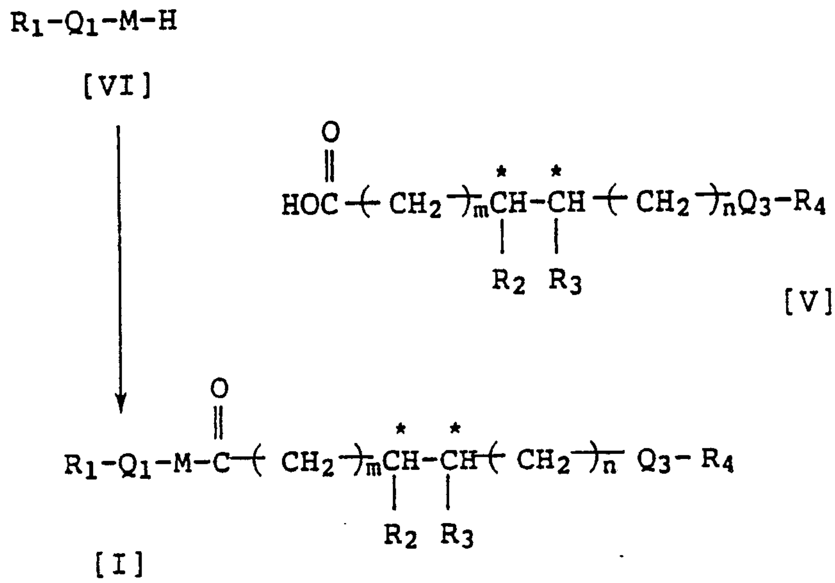

[VI] [V] [I]

The optically active compound I can be synthesized from an aromatic compound represented by the formula VI and an optically active dichiral carboxylic acid V. This reaction is ordinarily effected in the presence of a proton acid. As the proton acid, there can be mentioned, for example, hydrofluoric acid, sulfuric acid and polyphosphoric acid. When the aromatic compound is subjected to an acylation reaction, a Friedel-Crafts reaction can be used. That is, the carboxylic acid is reacted with a halogenating agent such as phosphorus pentachloride, thionyl chloride, thionyl bromide or the like to convert to an acid halide, and then the acid halide is reacted with the aromatic compound VI in the presence of a Lewis acid (e.g. aluminum chloride, titanium tetrachloride, boron trifluoride, tin tetrachloride, zinc chloride) in an organic solvent inert to the reaction at a low temperature, room temperature or an elevated temperature. As the organic solvent usable in the reaction, there can be mentioned, for example, carbon disulfide, halogenated hydrocarbons (e.g. methylene chloride, 1,2-dichloroethane, 1,1,2,2-tetrachloroethane), nitromethane, nitrobenzene and chlorobenzene. The thus produced optically active compound I can be isolated from the reaction mixture and purified according to ordinary separation and purification methods (e.g. extraction, solvent operation, column chromatography, liquid chromatography, recrystallization, fractional crystallization).

In the above, there were described the typical processes for production of the optically active compounds I of the present invention. The compounds I can also be produced by other processes. For example, the compounds I can be produced by condensing a basic skeletal compound having an optically active dichiral compound with other basic skeletal compound.

The desired compounds I obtained by the above processes can be separated from the reaction mixture and purified by ordinary separation and purification methods such as extraction, solvent operation, column chromatography, liquid chromatography, recrystallization and the like.

All of the starting materials II, III, IV, V and VI for production of the optically active compounds I of the present invention are known substances or can be easily derived from known substances. For exmaple, the optically active dichiral compounds III and V can be derived from known optically active dichiral compounds, and can also be obtained by, for example, a chemical asymmetric synthesis [J. D. Morrison et al., Asymmetric Synthesis, vol. 1 (1983) to vol. 5 (1985); B. Bosnich et al., Asymmetric Catalysis (1986); M. A. Sutter et al., Ann., 1983, 939], a biological asymmetric synthesis using an enzyme or a microorganism [J. B. Jones et al., "Applications of Biochemical Systems in Organic Chemistry", John Wiley, New York (1976); G. Frater et al., Tetrahedron, 40, 1269 (1984); R. W. Hoffman et al., Chem. Ber., 114, 2786 (1981); K. Nakamura et al., Tetrahedron Lett., 27, 3155 (1986)], and an optical resolution [J. Jacques et al., "Enantiomers, Racemates and Resolutions", John Wiley & Sons (1981); A. W. Ingersoll, Org. Synth., Coll. vol., 2, 506 (1943); H. Nohira et al., Chemistry Lett., 1981, 875, 951].

The thus obtained dichiral compound III can be subjected to inversion of configuration on asymmetric carbon by a chemical or biological method to convert it into other optical isomer(s). As the typical methods for inverting the hydroxyl group of optically active secondary alcohol, there are known, for example, a method in which the hydroxyl group is converted into an organic sulfonic acid ester and then subjected to an intramolecular nucleophilic substitution reaction to effect inversion [E. J. Corey et al., Tetrahedron Lett., 1975, 3183; D. T. Sawyer and M. J. Gibian, Tetrahedron, 35, 1471 (1979); Hong-Ming Shieh et al., J. Org. Chem., 46, 4321 (1981); J. W. Huffman and R. C. Desai, Syn. Commun., 13, 553 (1983)], a method in which an optically active secondary alcohol is activated by N,N′-dicyclohexylcarbodiimide (DCC) in the presence of cuprous chloride and then reacted with an appropriate carboxylic acid to effect inversion [J. Kaulen, Angew. Chem., 99, 800 (1987)], and a method in which an optically active secondary alcohol is reacted with diethyl azodicarboxylate (DEAD), triphenylphosphine ($Ph_3P$) and an appropriate carboxylic acid to effect inversion [O. Mitsunobu and E. Eguchi, Bull. Chem. Soc. Japan, 44, 3427 (1971); O. Mitsunobu, Synthesis, 1981, 1].

As typical examples of the optically active dichiral alcohol III which is a constituent of the dichiral portion of the optically active compounds I of the present invention, there can be mentioned the alcohols listed in the following cases (1), (2), (3) and (4). The alcohols listed in the cases (1), (2) and (4) represent the alcohols III wherein m is 1 and n is 0, and the alcohols listed in the case (3) represent the alcohols III wherein m and n are both 1.

(1) Alcohols III wherein m is 1, n is 0, $R_2$ and $R_3$ are $CH_3$, $R_4$ is an alkyl group of 1-10 carbon atoms and $Q_3$ is an ether group:

3-Alkyloxy-2-methylbutanols

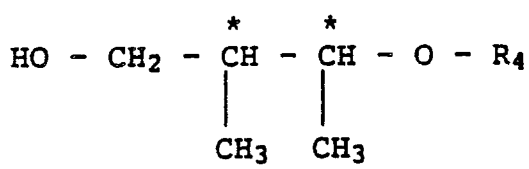

Specific examples of these alcohols include 3-methoxy-2-methylbutanol, 3-ethoxy-2-methylbutanol, 3-propoxy-2-methylbutanol, 3-butoxy-2-methylbutanol, 3-pentyloxy-2-methylbutanol, 3-hexyloxy-2-methylbutanol, 3-heptyloxy-2-methylbutanol, 3-octyloxy-2-methylbutanol, 3-nonyloxy-2-methylbutanol, 3-decyloxy-2-methylbutanol, 3-isopropoxy-2-methylbutanol, 3-isobutoxy-2-methylbutanol, 3-tert-butoxy-2-methylbutanol, 3-(2-methylpentyloxy)-2-methylbutanol and 3-(3-methylpentyloxy)-2-methylbutanol.

(2) Alcohols III wherein m is 1, n is 0, $R_3$ is $CH_3$, $R_2$ is a lower alkoxy group of 1-3 carbon atoms, $R_4$ is an alkyl group of 1-10 carbon atoms and $Q_3$ is an ether group.

2-Alkoxy-3-alkyloxybutanols

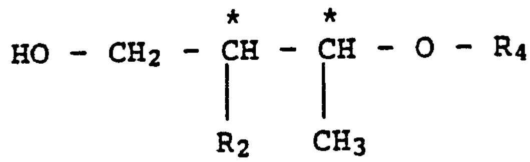

Specific examples of these alcohols include 2,3-dimethoxybutanol, 2,3-diethoxybutanol, 2,3-dipropoxybutanol, 2,3-diisopropoxybutanol, 2-methoxy-3- ethoxybutanol, 2-methoxy-3-propoxybutanol, 2-methoxy-3-butoxybutanol,2-methoxy-3-pentyloxybutanol, 2-methoxy-3-hexyloxybutanol, 2-methoxy-3-heptyloxybutanol, 2-methoxy-3-octyloxybutanol, 2-methoxy-3-nonyloxybutanol, 2-methoxy-3-isopropoxybutanol, 2-methoxy-3-isobutoxybutanol, 2-methoxy-3-tert-butoxybutanol, 2-methoxy-3-(2-methylpentyloxy)butanol and 2-methoxy-3-(3-methylpentyloxy)butanol.

(3) Alcohols III wherein m is 1, n is 1, $R_2$ and $R_3$ are independently a lower alkoxy group of 1-3 carbon atoms, $R_4$ is an alkyl group of 1-10 carbon atoms and $Q_3$ is an ether group:

2,3,4-Trialkoxybutanols

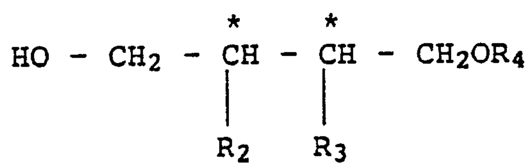

Specific examples of these alcohols include 2,3,4-trimethoxybutanol, 2,3,4-triethoxybutanol, 2,3,4-tripropoxybutanol, 2,3,4-triisopropoxybutanol, 2,3-dimethoxy-4-ethoxybutanol, 2,3-dimethoxy-4-propoxybutanol, 2,3-dimethoxy-4-butoxybutanol, 2,3-dimethoxy-4-pentyloxybutanol, 2,3-dimethoxy-4-hexyloxybutanol, 2,3-dimethoxy-4-heptyloxy-butanol, 2,3-dimethoxy-4-octyloxybutanol, 2,3-dimethoxy-4- nonyloxybutanol, 2,3-dimethoxy-4-decyloxybutanol, 2,3-dimethoxy-4-benzyloxybutanol and 2,3-dimethoxy-4-(4-methoxybenzyloxy)butanol.

4) Alcohols III wherein m is 1, n is 0, $R_2$ and $R_3$ are both $CH_3$, $R_4$ is an alkyl group of 1-10 carbon atoms and Q3 is a carboxylic acid ester group:

3-Acyloxy-2-methylbutanols

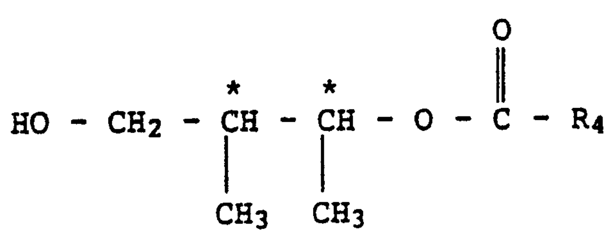

Secific examples of these alcohols include 3-acetyloxy-2-methylbutanol, 3-propionyloxy-2-methylbutanol, 3-butyryloxy-2-methylbutanol, 3-pentanoyloxy-2-methylbutanol, 3-hexanoyloxy-2-methylbutanol, 3-heptanoyloxy-2-methylbutanol, 3-octanoyloxy-2-methylbutanol, 3-nonanoyloxy-2-methylbutanol, 3-decyloxy-2-methylbutanol, 3-isobutyryloxy-2-methylbutanol, 3-isovaleryloxy-2-methylbutanol and 3-pyvaroyloxy-2-methylbutanol.

The optically active dichiral carboxylic acid V which is a raw material for the compounds I of the present invention can be derived from easily available optically active compounds (e.g. L-threonine, L-serine, L-alanine, L-aspartic acid, L-lactic acid, L-tartaric acid, D-tartaric acid, L-malic acid, D-malic acid) and can also be obtained by, for example, a chemical asymmetric synthesis [J. D. Morrison et al., Asymmetric Synthesis, vol. 1 (1983) to vol. 5 (1985); B. Bosnich et al., Asymmetric Catalysis (1986); M. A. Sutter et al., Ann., 1983, 939], a biological asymmetric synthesis using an enzyme or a microorganism [J. B. Jones et al., "Applications of Biochemical Systems in Organic Chemistry", John Wiley, New York (1976); G. Frater et al., Tetrahedron, 40, 1269 (1984); R. W. Hoffman et al., Chem. Ber., 114, 2786 (1981); K. Nakamura et al., Tetrahedron Lett., 27, 3155 (1986)], and an optical resolution [J. Jacques et al., "Enantiomers, Racemates and Resolutions", Joh Wiley & Sons (1981); A. W. Ingersoll, Org. Synth., Coll. vol. 2, 506 (1943); H. Nohira et al., Chemistry Lett., 1981, 875, 951].

Also as optically active dichiral carboxylic acids V wherein m and n are both 0, there can be mentioned the followings.

(1) Acids V wherein m and n are both 0, $R_2$ is $OCH_3$ and $R_3$ is $CH_3$, $Q_3$ is an ether group and $R_4$ is an alkyl group of 1-10 carbon atoms:

3-Alkoxy-2-methoxybutyric acids

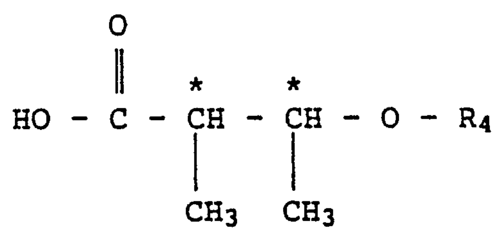

These alcohols specifically include 2,3-dimethoxybutyric acid, 3-ethoxy-2-methoxybutyric acid, 2-methoxy-3-propoxybutyric acid, 3-butoxy-2- methoxybutyric acid, 2-methoxy-3-pentyloxybutyric acid, 3-hexyloxy-2-methoxybutyric acid, 3-heptyloxy-2-methoxybutyric acid, 2-methoxy-3-octyloxybutyric acid, 2-methoxy-3-nonyloxybutyric acid, 3-decyloxy-2-methoxybutyric acid, 3-isopropoxy-2-methoxybutyric acid, 3-isobutoxy-2-methoxybutyric acid, 3-tert-butoxy-2-methoxybutyric acid and the like.

(2) Acids V wherein m and n are both 0, $R_2$ is $OCH_3$ and $R_3$ is $CH_3$, $Q_3$ is a $-\overset{O}{\overset{\|}{C}}-$ group and $R_4$ is an alkyl group of 1-10 carbon atoms:

3-Acyl-2-methoxybutyric acids

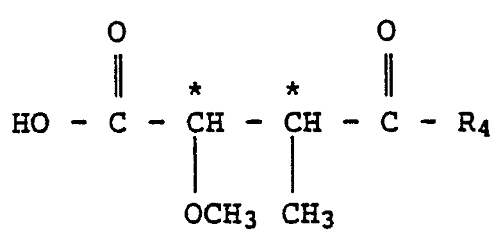

These compounds specifically include 3-acetyl, propionyl, butyryl, pentanoyl, hexanoyl, heptanoyl, octanoyl, nonanoyl, decynoyl, isobutyryl or isovaleryl 2-methoxybutyric acid.

(3) Acids V wherein m and n are both 0, $R_2$ is $OCH_3$ and $R_3$ is $CH_3$, $Q_3$ is a

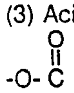 - ester group and $R_4$ is an alkyl group of 1-10 carbon atoms:

3-Acyloxy-2-methoxybutyric acids

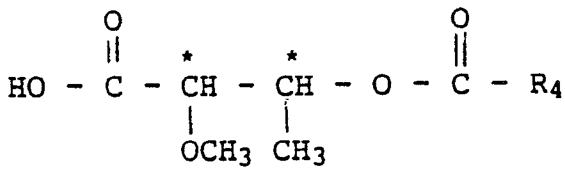

These compounds specifically include 3-acetyloxy-2-methoxybutyric acid, 2-methoxy-3-propionyloxybutyric acid, 3-butyryloxy-2-methoxybutyric acid, 2-methoxy-3-pentanoyloxybutyric acid, 3-hexanoyloxy-2-methoxybutyric acid, 3-heptanoyloxy-2-methoxybutyric acid, 2-methoxy-3-octanoyloxybutyric acid and the like.

Next, the carboxylic acid II, the alcohol or phenolic hydroxyl group-containing compound IV and the compound VI, all of which are a constituent of the skeletal portion of the optically active compounds I of the present invention, are specifically explained by mentioning their typical examples.

The carboxylic acid II can be classified into two type compounds represented by the following general formulas.

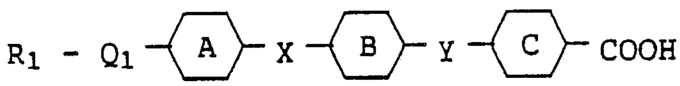 [II']

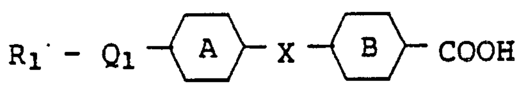 [II"]

As typical examples of these compounds, there can be mentioned 4-(4′-alkyloxy or alkyl-4-biphenyl carbonyloxy)benzoic acids, 4-(4′-alkyloxy or alkyl-4-biphenyloxycarbonyl)benzoic acids, 4′-(4-alkyloxy or alkylphenylcarbonyloxy)-4-biphenylcarboxylic acids, 4′-(4-alkyloxy or alkylphenyloxycarbonyl) 4-biphenylcarboxylic acids, 4′-alkyloxy or alkyl-4-biphenylcarboxylic acids, 4′-alkyloxycarbonyloxy-4-biphenylcarboxylic acids, 4′-alkyloxy(or alkyl)-4-terphenylcarboxylic acids, 4′-(trans-4-alkyloxy or alkylcyclohexylcarbonyloxy)-4-biphenylcarboxylic acids, trans-4-(4′-alkyloxy or alkyl-4-biphenylcarbonyloxy)-cyclohexanecarboxylic acids, 2-[4-(4-alkyloxy or alkylphenylcarbonyloxy)phenyl]pyridimidinyl-5-carboxylic acids, 2-(4′-alkyloxy or alkyl-4-biphenyl)pyrimidinyl-5-carboxylic acids, 4′-(5-alkyloxy or alkylpyrimidinyl-2-oxycarbonyl)biphenyl-4-craboxylic acids, 4′-[2-(5-alkyloxy or alkyl)-2-(pyridyl)ethyl]biphenyl-4-carboxylic acis, 4-[4-(trans-5-alkyloxy or alkyl-1,3-dioxane-2-yl)phenylcarbonyloxyzbenzoic acids, 4′-[4-(trans-5-alkyloxy or alkyl-1,3-dioxane-2-yl)biphenyl-4-carboxylic acids, 2-[4-(4-alkyloxy or alkylphenylcarbonyloxy)phenyl]-pyrazinyl-5-carboxylic acids, 2-(4′alkyloxy or alkyl-4-biphenyl)pyrazinyl-5-carboxylic acids, 4′-(5-alkyloxy or alkylpyrazinyl-2-oxycarbonyl)biphenyl-4-carboxylic acids and 4′-(6-alkyloxy or alkyl-3-pyridazinyl)biphenyl-4-carboxylic acids.

The alcohol IV or the compound IV having a phenolic hydroxy group can be classified into two type compounds represented by the following general formulas.

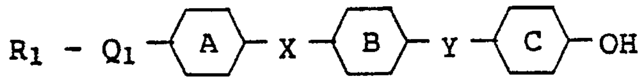

[IV']

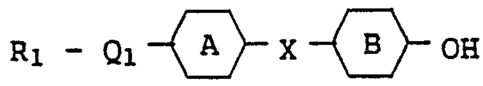

[IV"]

As typical examples of these compounds, there can be mentioned 4-hydroxyphenyl esters of 4'-alkyloxy or alkylbiphenyl-4-carboxylic acids, 4'-alkyloxy or alkyl-4-biphenyl esters of 4-hydroxybenzoic acids, 4'-hydroxy-4-biphenyl esters of 4-alkyloxy or alkylbenzoic acids, 4-alkyloxy or alkylphenyl esters of 4'-hydroxybiphenyl-4-carboxylic acids, 4'-hydroxy-4-biphenyl esters of trans-4-alkyloxy or alkylcyclohexanecarboxylic acids, trans-4-hydroxycyclohexyl esters of 4'-alkyloxy or alkyl-4-biphenylcarboxylic acis, 4-(5-hydroxy-2-pyrimidinyl)phenyl esters of 4-alkyloxy or alkylbenzoic acids, 2-(4'-alkyloxy or alkyl-4-biphenyl)-pyrimidine-5-ol, 5-alkyloxy or alkyl-2-pyridinyl esters of 4'-hydroxy-4-biphenylcarboxylic acids, 4'-[2-(5-alkyloxy or alkyl-2-pyridyl)ethyl]biphenyl-4-ol, 4-hydroxyphenyl esters of 4-[4-(trans-5-alkyloxy or alkyl)-1,3-dioxane-2-yl]benzoic acids, 5-alkyloxy or 5-alkyl-2-pyrazinyl esters of 4'-hydroxy-4-biphenylcarboxylic acids, 4'-alkyloxy or alkyl-4-biphenol, 4'-alkyloxycarbonyloxy-4-biphenol, 4"-alkyloxy or alkyl-4-terphenol.

The compound VI is easily available commercially as a reagent, or can be synthesized from commercially available precursors.

The optically active compounds I of the present invention have a structure in which each asymmetric carbon atom is present close to oxygen or carbonyl, and therefore the compounds generally show high spontaneous polarization. In addition, most of the compounds I show a chiral smectic C (Sc*) phase which is a liquid crystal phase suitable for display methods utilizing the ferroelectric properties of liquid crystals, and the temperature range of the chiral smectic C phase is low and wide.

The optically active compounds of the present invention are very stable to heat, light, water and air. Accordingly, in putting the compounds to practical use as or in liquid crystal materials, there can be eliminated inconveniences such as arrangements of an apparatus for prevention of overheating, a glass frit seal for prevention of moisture absorption or permeation, etc.

The optically active compounds I of the present invention have excellent compatibility with conventionally known liquid crystal compounds such as those of Schiff's base type, biphenyl type, phenylcyclohexane type, heterocyclic type and the like. Therefore, the compounds can be made into liquid crystal compositions having excellent properties, by incorporating them into said liquid crystal compounds.

As the liquid crystal compounds into which the optically active compounds I of the present invention can be incorporated, there can be mentioned, for example, ferroelectric liquid crystal compounds as well as liquid crystal compounds showing a smectic C phase. The ferroelectric liquid crystal compounds include, for example, biphenyl type liquid crystals described in JP-A-118744/1984 and 13729/1985, ester type liquid crystals described in JP-A-128357/1984, 51147/1985, 22051/1986 and 249953/1986, and pyrimidine type liquid crystals described in JP-A-260564/1985, 24756/1986, 85368/1986 and 215373/1986. The liquid crystal compounds showing a smectic C phase include, for example, ester type liquid crystal compounds described in JP-A-228036/1987, and cyclohexane type liquid crystals and heterocyclic type liquid crystals described in the materials of the l6th Freiburg Liquid Crystal Forum (Mar. 21, 1986) and the materials of the First International Symposium on Ferroelectric Liquid Crystals (Sept. 21, 1987).

The optically active compounds of the present invention can also be incorporated into the nematic or cholesteric liquid crystals described in "Flüssige Kristalle in Tabellen" I & II, VEB-Verlag, Leipzig, and further can be mixed with commercially available nematic liquid crystal compounds. When the optically active compounds of the present invention are incorporated into nematic liquid crystals, the twisting direction of the cholesteric pitch and the pitch length of the nematic liquid crystal compositions obtained can be freely controlled via the amount added.

The present invention is described more specifically by way of Examples, Application Examples and Examples of Preparation of Liquid Crystal Compositions. Firstly, Examples 1 to 31 show cases of synthesis of compounds I wherein m is 1.

Example 1

Preparation of 4-[(2S,3S)-3-methoxy-2-methylbutoxycarbonyl]phenyl ester of 4'-octyloxy-4-biphenylcar-

boxylic acid (a compound of the general formula I′ in which $R_1$ is n-$C_8H_{17}$, $R_2$, $R_3$ and $R_4$ are all $CH_3$, $Q_1$ and $Q_3$ are both -O-, X is a single bond, Y and $Q_2$ are both $-\overset{\overset{O}{\|}}{C}-O-$, n is 0, and

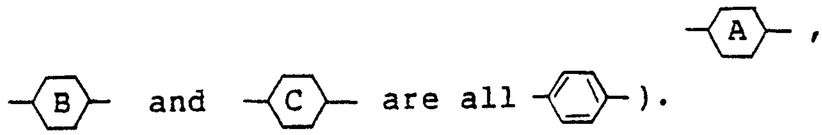

and

are all

).

i) Preparation of (2S,3S)-3-methoxy-2-methylbutanol

This compound was prepared according to the following scheme

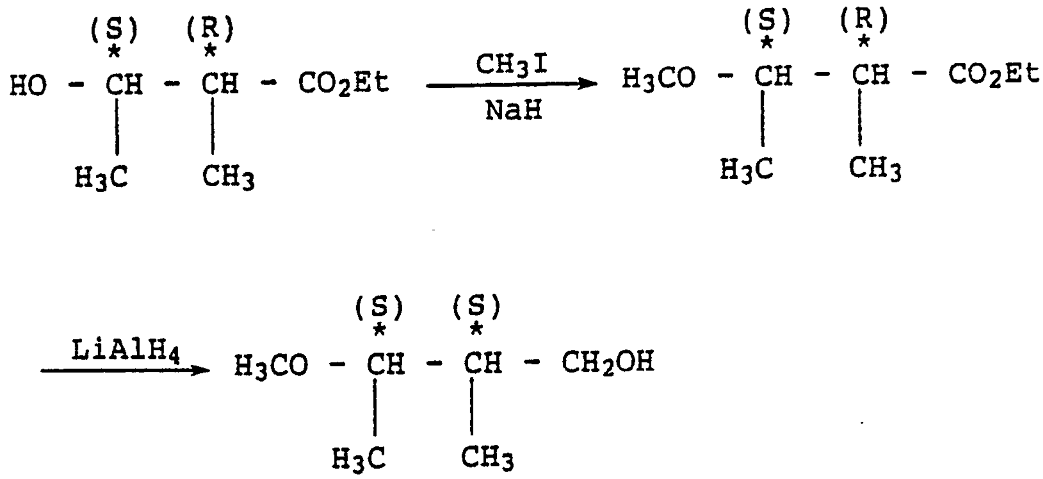


The starting material, i.e. ethyl ester of (2R,3S)-3-hydroxy-2-methylbutyric acid can be obtained by a known method [R. W. Hoffman et al., Chem Ber., 114, 2786 (1981)]. In this method, ethyl ester of 2-methyl-3-oxobutyric acid was subjected to asymmetric reduction using baker's yeast, whereby the desired compound and its diastereomer, i.e. ethyl ester of (2S,3S)-3-hydroxy-2-methylbutyric acid was obtained at a ratio of 82:18 to 87:13. In the following, the mixture of these two compounds was used, and the final product was subjected to purification procedures such as column chromatography, recrystallization and the like to obtain a single diastereomer.

8.20 g of the above ethyl ester of (2R,3S)-3-hydroxy-2-methylbutyric acid and 78.0 g of methyl iodide were dissolved in 150 ml of N,N-dimethylformamide. To the solution was added in small portions about 60% oily sodium hydride with ice cooling. The mixture was stirred for 1 hour with ice cooling. The reaction mixture was poured into 300 ml of ethyl acetate and the mixture was stirred for 10 minutes. The insoluble materials were removed by filtration and the filtrate was washed with water three times. The organic layer was dried and concentrated. The residue was subjected to distillation under reduced pressure (b.p. 80 °C/30 mmHg) to obtain 6.87 g of ethyl ester of 2R,3S)-3-methoxy-2-methylbutyric acid.

3.0 g of this methyl ether compound was dissolved in 20 ml of dry ether. To the solution was added in small portions 0.57 g of lithium aluminum hydride with ice cooling. The mixture was stirred for about 2 hours with ice cooling, then overnight at room temperature. To the reaction mixture were added 0.57 ml of water, 0.57 ml of an aqueous 15% NaOH solution and 0.57 ml of water in this order. The mixture was stirred at room temperature. The insoluble materials were removed by filtration. The filtrate was concentrated under reduced pressure. The residue was subjected to distillation under reduced pressure (b.p. 65-70 °C/30 mmHg) to obtain 1.42 g of (2S,3S)-3-methoxy-2-methylbutanol.

The $^1$H-NMR spectral data of this compound are shown below.

$^1$H-NMR (90 MHz, $CDCl_3$)

δ: 0.87 (3H, d), 1.23 (3H, d), 2.0 (1H, m), 2.7 (1H, b), 3.34 (3H, s), 3.4-3.7 (3H, m)

ii) Esterification

2.0 g of 4-(4′-octyloxy-4-biphenylcarbonyloxy)benzoic acid was mixed with 35 ml of thionyl chloride. The mixture was refluxed for 8 hours. Excess thionyl chloride was removed by distillation to obtain a corresponding acid chloride. The acid chloride was dissolved in 40 ml of 1,2-dimethoxyethane. To the solution were added 0.52 g of the (2S,3S)-3-methoxy-2-methylbutanol obtained in the above i) and 0.70 g of pyridine, and the mixture was stirred overnight at room temperature. After the completion of the reaction, the insoluble materials were removed by filtration and the filtrate was concentrated under reduced pressure. The residue was subjected to rough purification by column chromatography (silica gel, developing solvent: chloroform) and then to separation and purification by column chromatography [silica gel, developing solvent: chloroform-n-hexane (1:1)] and further to recrystallization from ethyl acetate-n-hexane to obtain 0.63 g of the title compound.

The ¹H-NMR and IR spectral data and elemental analysis of the compound are shown below.

¹H-NMR (90 MHz, $CDCl_3$)

δ: 0.91 (3H, t), 1.05 (3H, d), 1.20 (3H, d), 1.35 (10H, b), 1.85 (3H, b), 3.49 (3H, s), 3.43 (1H, m), 4.04 (2H, t), 4.02-4.5 (2H, m), 6.95-8.28 (12H, m)

IR $\nu_{max}^{KBr}$ $cm^{-1}$ : 1610, 830, 770

Elemental analysis

| | | |
|---|---|---|
| Calcd. for $C_{34}H_{42}O_6$: | C, 74.70; | H, 7.74 |
| Found : | C, 74.44; | H, 7.72 |

Example 2

Preparation of 4′-octyloxy-4-biphenyl ester of 4-[(2S,3S)-3-methoxy-2-methylbutoxycarbonyl]benzoic acid (a compound of the general formula I′ in which $R_1$ is n-$C_8H_{17}$, $R_2$, $R_3$ and $R_4$ are all $CH_3$, $Q_1$ and $Q_3$ are both -O-, X is a single bond, Y is

$-O-\overset{O}{\overset{\|}{C}}-$, n is 0, $Q_2$ is $-\overset{O}{\overset{\|}{C}}-O-$, and

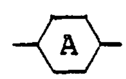 ,

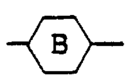 and 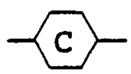 are all 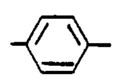 ).

The same procedure as in Example 1 ii) was carried out using 0.52 g of the (2S,3S)-3-methoxy-2-methylbutanol prepared in Example 1 i) and 2.0 g of 4-(4′-octyloxy-4-biphenyloxycarbonyl)benzoic acid, to obtain 1.09 g of the title compound.

The elemental analysis of the compound are shown below.

Elemental analysis

| | | |
|---|---|---|
| Calcd. for $C_{34}H_{42}O_6$: | C, 74.70; | H, 7.74 |
| Found : | C, 74.81; | H, 7.72 |

Example 3

Preparation of 4′-octyloxy-4-bipheny ester of 4-[(2S,3S)-3-methoxy-2-methylbutoxy]benzoic acid (a compound of the general formula I′ in which $R_1$ is n-$C_8H_{17}$, $R_2$, $R_3$ and $R_4$ are all $CH_3$, $Q_1$, $Q_2$ and $Q_3$ are all -O-, X is a single bond, Y is

O
||
-O- C -, n is 0, and

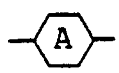 , 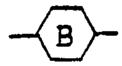 and 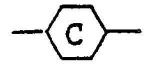 are all 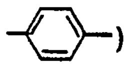 ).

In 50 ml of dry tetrahydrofuran were dissolved 0.84 g of the (2S,3S)-3-methoxy-2-methylbutanol obtained in Example 1 i), 2.0 g of 4′-octyloxy-4-biphenyl ester of 4-hydroxybenzoic acid and 2.5 g of triphenylphosphine. Thereto was dropwise added 1.66 g of diethyl azodicarboxylate. The mixture was stirred for 1 hour at room temperature. The solvent was removed by distillation. The residue was subjected to rough purification by column chromatography (silica gel, developing solvent: dichloromethane), then to separation and purification by column chromatography [silica gel, developing solvent: n-hexane-ethyl acetate (95:5)] and further to recrystallization from ethyl acetate-methanol to obtain 0.66 g of the title compound.

The $^1$H-NMR and IR spectral data and elemental analysis of the compound are shown below.

$^1$H-NMR (90 MHz, $CDCl_3$)

δ: 0.8-1.22 (9H, m), 1.33 (10H, b), 1.70-1.90 (2H, m), 2.15 (1H, m), 3.33 (3H, s), 3.55 (1H, m), 3.8-4.2 (4H, m), 6.9-8.18 (12H, m)

IR $\nu_{max}^{KBr}$ $cm^{-1}$ : 1730, 1610, 1580, 1510

Elemental analysis

| | | |
|---|---|---|
| Calcd. for $C_{33}H_{42}O_5$: | C, 76.42; | H, 8.16 |
| Found : | C, 76.56; | H, 8.16 |

Example 4

Preparation of 4-[(2S,3S)-3-methoxy-2-methylbutoxy]phenyl ester of 4′-octyloxy-4-biphenylcarboxylic acid (a compound of the general formula I′ in which $R_1$ is n- $C_8H_{17}$, $R_2$, $R_3$ and $R_4$ are all $CH_3$, $Q_1$, $Q_2$ and $Q_3$ are all -O-, X is a single bond, Y is

O
||
- C -O-, n is 0, and

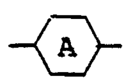 , 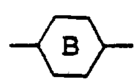 and 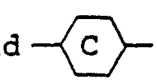

are all 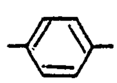 ).

The same procedure as in Example 3 was repeated using 0.5 g of the (2S,3S)-3-methoxy-2-methylbutanol obtained in Example 1 i) and 1.0 g of 4-hydroxyphenyl ester of 4′-octyloxy-4-biphenylcarboxylic acid, to obtain 0.58 g of the title compound.

The elemental analysis of the compound is shown below.

Elemental analysis

| | | |
|---|---|---|
| Calcd. for $C_{33}H_{42}O_5$: | C, 76.42; | H, 8.16 |
| Found : | C, 76.44; | H, 8.16 |

Example 5

Preparation of 4′-[(2S,3S)-3-methoxy-2-methylbutoxy]-4-biphenyl ester of 4-octyloxybenzoic acid (a compound of the general formula I′ in which $R_1$ is n-$C_8H_{17}$, $R_2$, $R_3$ and $R_4$ are all $CH_3$, $Q_1$, $Q_2$ and $Q_3$ are all -O-, X is $-\overset{\underset{\|}{O}}{C}-O-$, Y is a single bond, n is 0, and

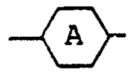 , 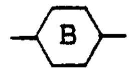 and 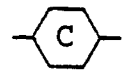 are all 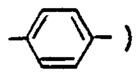 ).

The same procedure as in Example 3 was repeated using 0.5 g of the (2S,3S)-3-methoxy-2-methylbutanol obtained in Example 1 i) and 1.0 g of 4-octyloxyphenyl ester of 4′-hydroxy-4-biphenylcarboxylic acid, to obtain 0.46 g of the title compound.

The elemental analysis of the compound is shown below.

Elemental analysis

| | | |
|---|---|---|
| Calcd. for $C_{33}H_{42}O_5$: | C, 76.42; | H, 8.16 |
| Found : | C, 76.50; | H, 8.20 |

Example 6

Preparation of 4-octyloxyphenyl ester of 4′-[(2S,3S)-3-methoxy-2-methylbutoxy]-4-biphenylcarboxylic acid (a compound of the general formula in which $R_1$ is n-$C_8H_{17}$, $R_2$, $R_3$ and $R_4$ are all $CH_3$, $Q_1$, $Q_2$ and $Q_3$ are all -O-, X is $-O-\overset{\underset{\|}{O}}{C}-$, Y is a single bond, n is 0 and

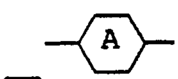 , 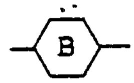 and 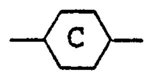 are all 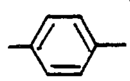 ).

The same procedure as in Example 3 was repeated using 0.42 g of the (2S,3S)-3-methoxy-2-methylbutanol obtained in Example 1 i) and 1.0 g of 4-octyloxyphenyl ester of 4′-hydroxy-4-biphenylcarboxylic acid, to obtain 0.60 g of the title compound.

The elemental analysis of the compound is shown below.

Elemental analysis

| | | |
|---|---|---|
| Calcd. for $C_{33}H_{42}O_5$: | C, 76.42; | H, 8.16 |
| Found : | C, 76.32; | H, 8.23 |

Example 7

Preparation of 4′-[(2S,3S)-3-butoxy-2-methylbutoxy]-4-biphenyl ester of 4-octyloxybenzoic acid (a compound of the general formula I′ in which $R_1$ is n-$C_8H_{17}$, $R_2$ and $R_3$ are both $CH_3$, $R_4$ is n-$C_4H_9$, $Q_1$, $Q_2$ and $Q_3$ are all -O-, X is $-\overset{\underset{\|}{O}}{C}-O-$, Y is a single bond, n is 0, and

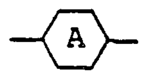 , 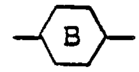

and 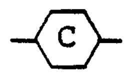 are all 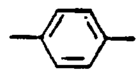 ).

i) Preparation of (2S,3S)-3-butoxy-2-methylbutanol

The compound was prepared in accordance with the following scheme.

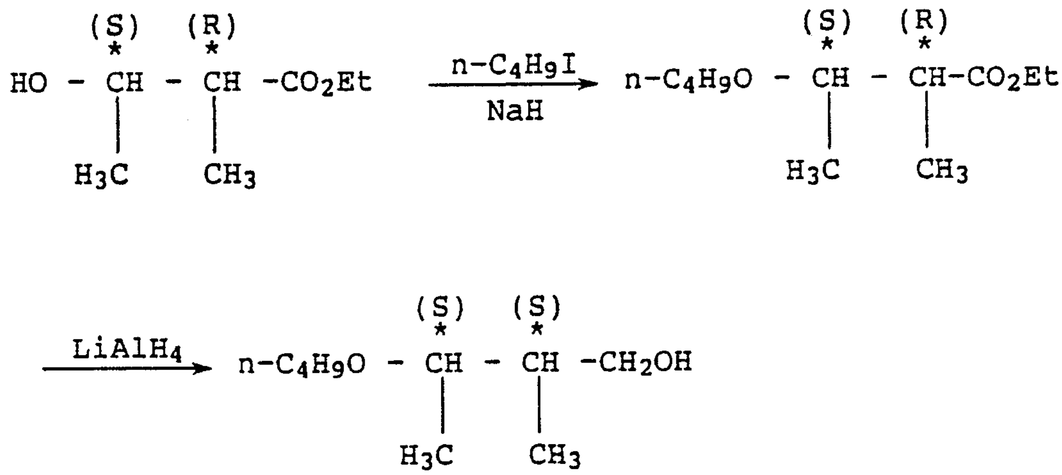


In 80 ml of N,N-dimethylformamide were dissolved 7.0 g of ethyl ester of (2R,3S)-3-hydroxy-2-methylbutyric acid (see Example 1 i)) and 45 g of n-butyl iodide. To the solution was added by small portions about 60% oily sodium hydride with ice cooling. The mixture was stirred for 1 hour with ice cooling. The reaction mixture was poured into 300 ml of ether, and the mixture was stirred for 10 minutes. The insoluble materials were removed by filtration, and the filtrate was washed with water three times. The organic layer was dried and concentrated and then subjected to distillation under reduced pressure (b.p. 90-95 °C/mmHg) to obtain 4.17 g of ethyl ester of (2R,3S)-3-butoxy-2-methylbutyric acid.

2.0 g of the butyl ether compound was dissolved in 20 ml of dry ether. To the solution was added by small portions 0.3 g of lithium aluminum hydride with ice cooling. The mixture was stirred for about 2 hours with ice cooling and then overnight at room temperature.

To the reaction mixture were added 0.3 ml of water, 0.3 ml of an aqueous 15% NaOH solution and 0.9 ml of water in this order. The mixture was stirred at room temperature. The insoluble materials were removed by filtration. The filtrate was concentrated under reduced pressure. The residue was subjected to Kugel-rohr distillation to obtain 0.54 g of (2S,3S)-3-butoxy-2-methylbutanol.

The $^1$H-NMR spectral data of the compound are shown below.

$^1$H-NMR (90 MHz, $CDCl_3$)

δ: 0.82-1.50 (13H, m), 1.95 (1H, m), 2.95 (1H, b), 3.25-3.7 (5H, m)

ii) Etherification

In 50 ml of dry tetrahydrofuran were dissolved 0.5 g of the (2S,3S)-3-butoxy-2-methylbutanol obtained in the above i), 1.0 g of 4-hydroxyphenyl ester of 4′-octyloxy-4-biphenylcarboxylic acid and 1.25 g of triphenylphosphine. To the solution was dropwise added 1.3 g of diethyl azodicarboxylate, and the mixture was stirred for 3 hours at room temperature. The solvent was removed by distillation. The residue was subjected to rough purification by column chromatography (silica gel, developing solvent: dichloromethane), then to separation and purification by column chromatography [silica gel, developing solvent: n-hexane-ethyl acetate (95:5)] and further to recrystallization from ethanol to obtain 0.19 g of the title compound.

The $^1$H-NMR and IR spectral data and elemental analysis of the compound are shown below.

$^1$H-NMR (90 MHz, $CDCl_3$)

δ: 0.80-1.60 (28H, m), 1.70-2.20 (3H, m), 3.20- 4.20 (7H, m), 6.92-8.18 (12H, m)

IR $\nu_{max}^{KBr}$ $cm^{-1}$ : 1735, 1610, 1510, 1500

Elemental analysis

| | | |
|---|---|---|
| Calcd. for $C_{36}H_{48}O_5$: | C, 77.11; | H, 8.63 |
| Found : | C, 76.98; | H, 8.59 |

Example 8

Preparation of 4-[(2S,3S)-3-butoxy-2-methylbutoxy]phenyl ester of 4′-octyloxy-4-biphenylcarboxylic acid (a compound of the general formula I′ in which $R_1$ is n-$C_8H_{17}$, $R_2$, $R_3$ are both $CH_3$, $R_4$ is n-$C_4H_9$, $Q_1$, $Q_2$ and $Q_3$ are all -O-, X is a single bond, Y is

$-\overset{O}{\overset{\|}{C}}-O-$, n is 0, and

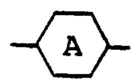 , 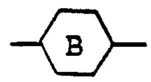 and 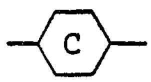 are all 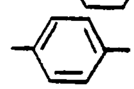 ).

The same procedure as in Example 7 ii) was repeated using 0.5 g of the (2S,3S)-3-butoxy-2-methylbutanol obtained in Example 7 i) and 1.0 g of 4-hydroxyphenyl ester of 4′-octyloxy-4-biphenylcarboxylic acid, to obtain 0.38 g of the title compound.

The elemental analysis of the compound is shown below.

Elemental analysis

| | | |
|---|---|---|
| Calcd. for $C_{36}H_{48}O_5$: | C, 77.11; | H, 8.63 |
| Found : | C, 76.15; | H, 8.56 |

Example 9

Preparation of 4-octyloxyphenyl ester of 4′-[(2S,3S)-3-butoxy-2-methylbutoxy]-4-biphenylcarboxylic acid (a compound of the general formula I′ in which $R_1$ is n-$C_8H_{17}$, $R_2$, $R_3$ are both $CH_3$, $R_4$ is n-$C_4H_9$, $Q_1$, $Q_2$ and $Q_3$ are all -O-, X is a

$-O-\overset{O}{\overset{\|}{C}}-$, Y is a single bond, n is 0, and

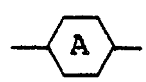 , 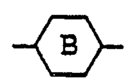 and 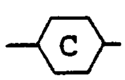 are all 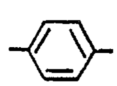 ).

0.34 g of the title compound was obtained using 0.5 g of the (2S,3S)-3-butoxy-2-methylbutanol obtained in Example 7 i) and 1.0 g of 4-octyloxyphenyl ester of 4′-hydroxy-4-biphenylcarboxylic acid.

The elemental analysis of the compound is shown below.

Elemental analysis

| | | |
|---|---|---|
| Calcd. for $C_{36}H_{48}O_5$: | C, 77.11; | H, 8.63 |
| Found : | C, 76.18; | H, 8.56 |

Example 10

Preparation of 4-[(2S,3S)-3-butoxy-2-methylbutoxy]phenyl ester of 4′-octyloxy-4-biphenylcarboxylic acid (a compound of the general formula I′ in which $R_1$ is n-$C_8H_{17}$, $R_2$ and $R_3$ are both $CH_3$, $R_4$ is n-$C_4H_9$, $Q_1$, $Q_2$ and $Q_3$ are all -O-, X is a single bond, Y is
$-\overset{\overset{O}{\|}}{C}-O-$, n is 0, and

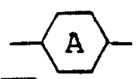 , 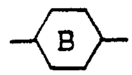 and

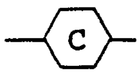 are all 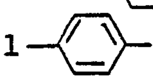 ).

The same procedure as in Example 7 ii) was repeated using 0.5 g of the (2S,3S)-3-butoxy-2-methylbutanol obtained in Example 7 i) and 1.0 g of 4-hydroxy phenyl ester of 4′-octyloxy-4-biphenylcarboxylic acid, to obtain 0.19 g of the title compound.

The elemental analysis of the compound is shown below.

Elemental analysis

| | | |
|---|---|---|
| Calcd. for $C_{36}H_{48}O_5$: | C, 77.11; | H, 8.63 |
| Found : | C, 77.22; | H, 8.59 |

Example 11

Preparation of 4-[(2R,3R)-2,3-dimethoxybutoxycarbonyl]phenyl ester of 4′-octyloxy-4-biphenylcarboxylic acid (a compound of the general formula I′ in which $R_1$ is n-$C_8H_{17}$, $R_2$ is $OCH_3$, $R_3$ and $R_4$ are both $CH_3$, $Q_1$ and $Q_2$ are both -O-, X is a single bond, Y and $Q_2$ are both
$-\overset{\overset{O}{\|}}{C}-O-$, n is 0 and

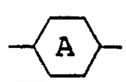 , 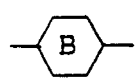 and

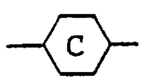 are all 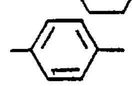 ).

i) Preparation of (2S,3R)-2,3-dimethoxy-1-butanol

This optically active alcohol was prepared in accordance with the following scheme.

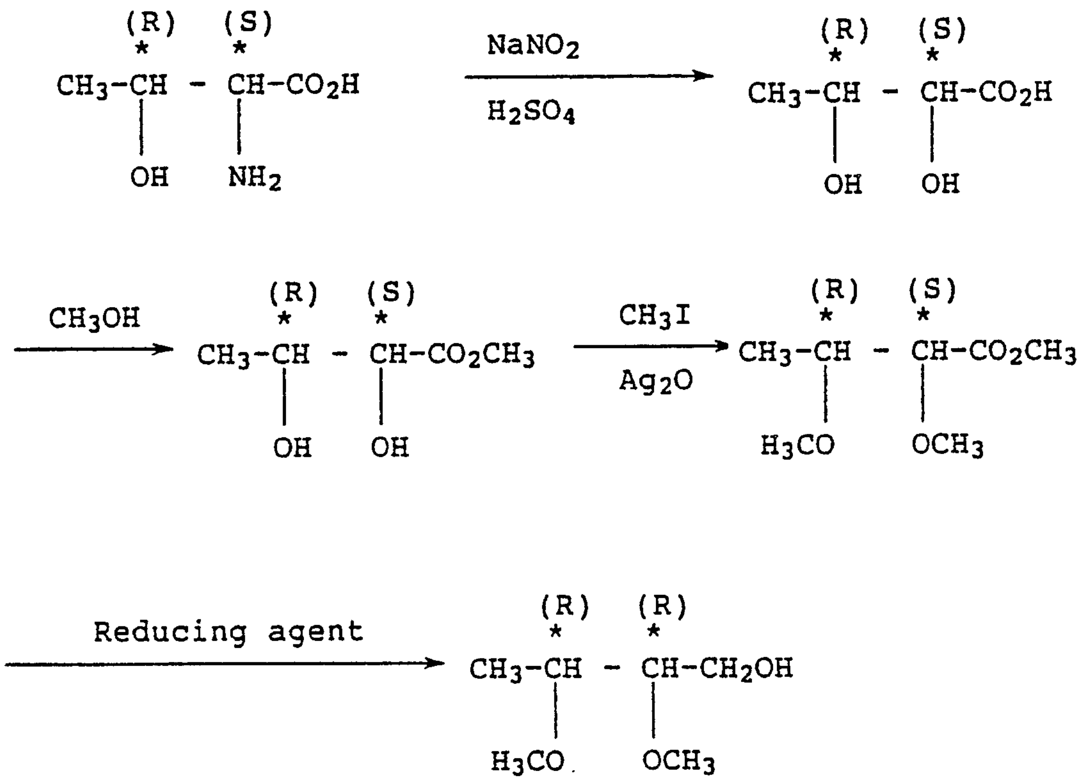


119.1 g of (2S,3R)-2-amino-3-hydroxybutyric acid was dissolved in 1.5 liters of 1 N sulfuric acid and subjected to a diazotization reaction using sodium nitrite in accordance with the known method. After the completion of the reaction, the solvent was removed by distillation under reduced pressure. The residue was mixed with 3 liters of methanol. The insoluble materials were removed by filtration. The filtrate was refluxed for 5 hours. After cooling, the reaction mixture was concentrated under reduced pressure. The residue was subjected to distillation under reduced pressure (b.p. 98-102 °C/8 mmHg) to obtain 53.3 g of methyl ester of (2S,3R)-2,3-dihydroxybutyric acid. 5.4 g of this methyl ester was dissolved in 200 ml of acetonitrile. To the solution were added 119 g of methyl iodide and 27.8 g of silver oxide. The mixture was stirred for 5 days at room temperature. The insoluble materials were removed by filtration. The filtrate was concentrated under reduced pressure. The residue was subjected to distillation under reduced pressure (b.p. 91-95 °C/25 mmHg) to obtain 3.2 g of methyl ester of (2S,3R)-2,3-dimethoxybutyric acid.

3.2 g of the methyl ester was dissolved in 50 ml of dry ether. To the solution was added by small portions 0.60 g of lithium aluminum hydride with cooling. The mixture was stirred overnight at room temperature. After the completion of the reaction, 0.6 ml of $H_2O$, 0.6 ml of 15% aqueous NaOH and 1.8 ml of $H_2O$ were added sequentially dropwise. The insoluble materials were removed by filtration and the filtrate was concentrated under reduced pressure. The residue was subjected to distillation under reduced pressure (b.p. 90 °C/30 mmHg) to obtain 1.9 g of (2R,3R)-2,3-dimethoxy-1-butanol.

The $^1$H-NMR and IR spectral data of the compound are shown below.

$^1$H-NMR (90 MHz, $CDCl_3$)

δ: 1.15 (3H, d), 2.30 (1H, t), 3.04-3.30 (2H, m), 3.38 (3H, s), 3.48 (3H, s), 3.56-3.85 (2H, m)

IR $\nu_{max}^{neat}$ $cm^{-1}$ : 3150-3650

ii) Esterification

2.00 g of 4-(4′-octyloxy-4-biphenylcarbonyloxy)benzoic acid was mixed with 30 ml of thionyl chloride. The mixture was refluxed for 6 hours. Excess thionyl chloride was removed by distillation to obtain a corresponding acid chloride. The acid chloride was dissolved in 80 ml of dry tetrahydrofuran. To the solution was added 0.66 g of the (2R,3R)-2,3-dimethoxy-1-butanol obtained in the above i). 0.71 g of pyridine was added dropwise. The mixture was stirred overnight at room temperature. The reaction mixture

was concentrated under reduced pressure. The residue was subjected to separation and purification by column chromatography (silica gel, developing solvent: chloroform) and then to recrystallization from ethyl acetate-methanol to obtain 0.32 g of the title compound.

The $^1$H-NMR and IR spectral data and elemental analysis of the compound are shown below.

$^1$H-NMR (90 MHz, $CDCl_3$)

δ: 0.90 (3H, t), 1.19-1.33 (13H, b), 1.70-1.90 (2H, m), 3.40 (3H, s), 3.54 (5H, s), 4.02 (2H, t), 4.50 (2H, t), 6.95-8.28 (12H, m)

IR $\nu_{max}^{KBr}$ $cm^{-1}$ : 1730, 1705, 1600, 810, 760

Elemental analysis

| | | |
|---|---|---|
| Calcd. for $C_{34}H_{42}O_7$: | C, 72.57; | H, 7.52 |
| Found : | C, 72.50; | H, 7.62 |

Example 12

Preparation of 4′-octyloxy-4-biphenyl ester of 4-[(2R,3R)-2,3-dimethoxybutoxycarbonyl]benzoic acid (a compound of the general formula I′ in which $R_1$ is n-$C_8H_{17}$, $R_2$ is $OCH_3$, $R_3$ and $R_4$ are both $CH_3$, $Q_1$ and $Q_3$ are both -O-, X is a single bond, Y is

$-O-\overset{O}{\overset{\|}{C}}-$, $Q_2$ is $-\overset{O}{\overset{\|}{C}}-O-$, n is 0, and

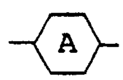 , 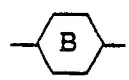 and 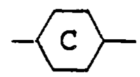 are all 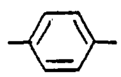 ).

The same procedure as in Example 1 ii) was repeated using 0.66 g of the (2R,3R)-2,3-dimethoxy-1-butanol obtained in Example 1 i) and 2.23 g of 4-(4′-octyloxy-4-biphenyloxycarbonyl)benzoic acid, to obtain 0.44 g of the title compound.

The elemental analysis of the compound is shown below.

Elemental analysis

| | | |
|---|---|---|
| Calcd. for $C_{34}H_{42}O_7$: | C, 72.57; | H, 7.52 |
| Found : | C, 72.30; | H, 7.56 |

Example 13

Preparation of 4′-octyloxy-4-biphenyl ester of 4-[(2R,3R)-2,3-dimethoxybutoxy]benzoic acid (a compound of the general formula I′ in which $R_1$ is n-$C_8H_{17}$, $R_2$ is $OCH_3$, $R_3$ and $R_4$ are both $CH_3$, $Q_1$, $Q_2$ and $Q_3$ are all -O-, X is a single bond, Y is

$-O-\overset{O}{\overset{\|}{C}}-$, n is 0, and

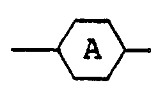 , 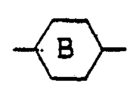 and 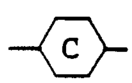 are all 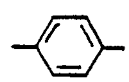 ).

In 100 ml of dry tetrahydrofuran were dissolved 1.00 g of the (2R,3R)-2,3-dimethoxy-1-butanol obtained

in Example 1 i), 2.0 g of 4′-octyloxy-4-biphenyl ester of 4-hydroxybenzoic acid and 1.97 g of triphenylphosphine. To the solution was dropwise added 1.30 g of diethyl azodicarboxylate, and the mixture was stirred for 1 hour at room temperature. The solvent was removed by distillation. The residue was subjected to separation and purification by column chromatography (silica gel, developing solvent: dichloromethane) and then to recrystallization from methanol to obtain 1.90 g of the title compound.

The $^1$H-NMR and IR spectral data and elemental analysis of the compound are shown below.

$^1$H-NMR (90 MHz, $CDCl_3$)

δ: 0.89 (3H, t), 1.20-1.34 (13H, b), 1.70-1.90 (2H, m), 3.39 (3H, s), 3.55 (5H, s), 4.00 (2H, t), 4.20 (2H, t), 6.91-8.20 (12H, m)

IR $\nu_{max}^{KBr}$ $cm^{-1}$ : 1725, 1605, 1510, 1490

Elemental analysis

| | | |
|---|---|---|
| Calcd. for $C_{33}H_{42}O_6$: | C, 74.13; | H, 7.92 |
| Found : | C, 74.32; | H, 7.87 |

Example 14

Preparation of 4-[(2R,3R)-2,3-dimethoxybutoxy]phenyl ester of 4′-octyloxy-4-biphenylcarboxylic acid (a compound of the general formula I′ in which $R_1$ is n-$C_8H_{17}$, $R_2$ is $OCH_3$, $R_3$ and $R_4$ are $CH_3$, $Q_1$, $Q_2$ and $Q_3$ are -O-, X is a single bond, Y is $-\overset{O}{\overset{\|}{C}}-O-$, n is 0, and

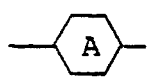 , 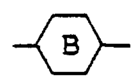

and 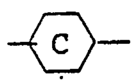 are all 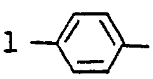 ).

The same procedure as in Example 3 was repeated using 0.50 g of the (2R,3R)-2,3-dimethoxy-1-butanol obtained in Example 1 i) and 1.04 g of 4-hydroxyphenyl ester of 4′-octyloxy-4-biphenylcarboxylic acid, to obtain 0.25 g of the title compound.

The elemental analysis of the compound is shown below.

Elemental analysis

| | | |
|---|---|---|
| Calcd. for $C_{33}H_{42}O_6$: | C, 74.13; | H, 7.92 |
| Found : | C, 74.32; | H, 7.87 |

Example 15

Preparation of 4-octyloxyphenyl ester of 4′-[(2R,3R)-2,3-dimethoxybutoxy]-4-biphenylcarboxylic acid (a compound of the general formula I′ in which $R_1$ is n-$C_8H_{17}$, $R_2$ is $OCH_3$, $R_3$ and $R_4$ are both $CH_3$, $Q_1$, $Q_2$ and $Q_3$ are all -O-, X is $-O-\overset{O}{\overset{\|}{C}}-$, Y is a single bond, n is 0, and

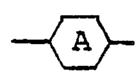 , 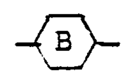

and 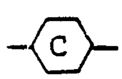 are all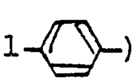 ).

The same procedure as in Example 3 was repeated using 0.67 g of the (2R,3R)-2,3-dimethoxy-1-butanol and 1.04 g of 4-octyloxyphenyl ester of 4′-hydroxy-4-biphenylcarboxylic acid, to obtain 0.60 g of the title compound.

The elemental analysis of the compound is shown below.

Elemental analysis

| | | |
|---|---|---|
| Calcd. for $C_{33}H_{42}O_6$: | C, 74.13; | H, 7.92 |
| Found : | C, 74.03; | H, 7.90 |

Example 16

Preparation of 4′-[(2R,3R)-2,3-dimethoxybutoxy]-4-biphenyl ester of 4-octyloxybenzoic acid (a compound of the general formula I′ in which $R_1$ is n-$C_8H_{17}$, $R_2$ is $OCH_3$, $R_3$ and $R_4$ are both $CH_3$, $Q_1$, $Q_2$ and $Q_3$ are all -O-, X is

$-\overset{O}{\overset{\|}{C}}-O-$, Y is a single bond, n is 0, and

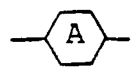 , 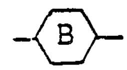 and 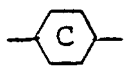 are all 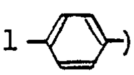).

The same procedure as in Example 3 was repeated using 0.67 g of the (2R,3R)-2,3-dimethoxy-1-butanol obtained in Example 1 i) and 1.04 g of 4-hydroxyphenyl ester of 4′-octyloxy-4-biphenylcarboxylic acid, to obtain 0.64 g of the title compound.

The elemental analysis of the compound is shown below.

Elemental analysis

| | | |
|---|---|---|
| Calcd. for $C_{33}H_{42}O_6$: | C, 74.13; | H, 7.92 |
| Found : | C, 74.17; | H, 7.91 |

Example 17

Preparation of (2S,3S)-4-(4-methoxybenzyloxy)-2,3-dimethoxybutyl ester of 4-(4′-octyloxy-4-biphenylcarbonyloxy)benzoic acid (a compound of the general formula I′ in which $R_1$ is n-$C_8H_{17}$, $R_2$ and $R_3$ are both $OCH_3$, $R_4$ is

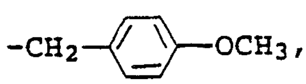,

$Q_1$ is -O-, $Q_2$ is

$-\overset{O}{\overset{\|}{C}}-O-$, $Q_3$ is -O-, X is a single bond, Y is

$-\overset{O}{\overset{\|}{C}}-O-$, n is 0, and

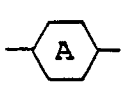 , 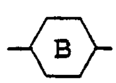 and 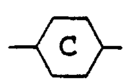 are all 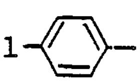).

i) Preparation of (2S,3S)-4-(4-methoxybenzyloxy)-2,3-dimethoxybutanol

Said compound is prepared by converting (2S,3S)-2,3-dimethoxy-1,4-butanediol [this compound is derived from (2R,3R)-2,3-dihydroxysuccinic acid (common name:L-(+)-tartaric acid) and mentioned in Seebach et al., Helv. Chim. Acta, 60, 301 (1977)] into its mono(4-methoxybenzyl) form.

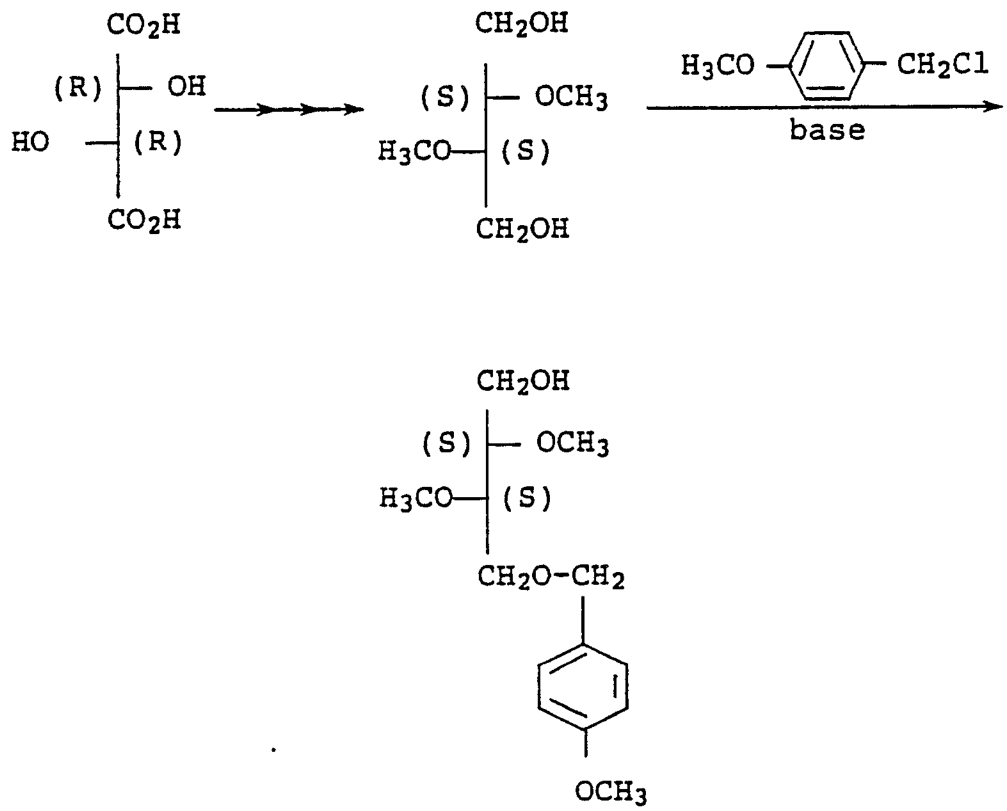


4.55 g of (2S,3S)-2,3-dimethoxy-1,4-butanediol was dissolved in 20 ml of dry tetrahydrofuran. The solution was added to a solution of 1.26 g of 60% oily sodium hydride dissolved in 50 ml of dry tetrahydrofuran, at 0° C with stirring. The mixture was stirred for 5 minutes at 0° C and further overnight at room temperature. The reaction mixture was cooled to 5-7° C and filtered using a filtering aid (High Flow Super Cell®). The filtrate was concentrated under reduced pressure. The residue was subjected to column chromatography using a silica gel (250 g) and ethyl acetate-carbon tetrachloride (2:1). 5.81 g of the title compound was obtained as a colorless oily material from the relevant fractions.

IR $\nu_{max}^{neat}$ $cm^{-1}$ : 3450, 1610, 1510, 1245
$^1$H-NMR (90 MHz, $CDCl_3$)
δ: 3.458 (6H, s, CH-$OCH_3$), 3.3-3.9 (m), 3.80 (3H, s, aromatic $OCH_3$), 4.476 (2H, s, benzylic $CH_2$), 6.75-7.35 (4H, m, aromatic H)

ii) Esterification

2.25 g of 4-(4′-octyloxy-4-biphenylcarbonyloxy)benzoic acid was dissolved in 30 ml of tetrahydrofuran at 60-65° C. The solution was concentrated under reduced pressure. The residue was mixed with 20 ml of thionyl chloride. The mixture was heated at 90° C for 2.5 hours with stirring. The reaction mixture was concentrated under reduced pressure. The residue was mixed with toluene and the mixture was concentrated under reduced pressure. This proceudre was repeated several times to obtain, as a residue, an acid chloride. The acid chloride was dissolved in 30 ml of dry tetrahydrofuran. To the solution were added at room temperature, with stirring, a solution of 2.0 g of the (2S,3S)-4-(4-methoxybenzyloxy)-2,3-dimethoxybutanol obtained in the above i) and 2.0 ml of pyridine in 40 ml of dry tetrahydrofuran. The mixture was stirred overnight at room temperature. The insoluble materials were removed by filtration and

the filtrate was concentrated under reduced pressure. The residue was subjected to column chromatography using a silica gel (150 g ) and carbon tetrachloride-ethyl acetate (5:1). The relevant fractions were concentrated and the residue was recrystallized from ethanol to obtain 0.83 g of the title compound as a colorless needles.

The IR and $^1$H-NMR spectral data and elemental analysis of the compound are shown below.

IR $\nu_{max}^{KBr}$ $cm^{-1}$ : 1730, 1710, 1600, 1515
$^1$H-NMR (90 MHz, $CDCl_3$)
δ: 0.895 (3H, t, J=6Hz, $CH_2CH_3$), 1.1-2.0 (12H, m, $CH_2$), 3.492 (3H, s, $OCH_3$), 3.507 (3H, s, $OCH_3$), 3.3-3.9 (m), 3.797 (3H, s, aromatic $\bar{O}CH_3$), 4.019 (2H, t, J=6Hz, $OC\underline{H}_2CH_2$), 4.35-4.6 (4H, m), 6.7-8.3 (16H, m, aromatic)
Elemental analysis

| | | |
|---|---|---|
| Calcd. for $C_{42}H_{50}O_8$ (698.854): | C, 72.18; | H, 7.21 |
| Found : | C, 72.13; | H, 7.20 |

Example 18

Preparation of (2S,3S)-4-(4-methoxybenzyloxy)-2,3-dimethoxybutyl ester of 4-(4′-octyloxy-4-biphenyloxycarbonyl)benzoic acid (a compound of the general formula I′ in which $R_1$ is n-$C_8H_{17}$, $R_2$ and $R_3$ are both $OCH_3$, $R_4$ is

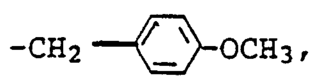

$Q_1$ is -O-, $Q_2$ is $-\overset{O}{\overset{||}{C}}-O-$, $Q_3$ is -O-, X is a single bond, Y is $-O-\overset{O}{\overset{||}{C}}-$, n is 0, and

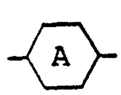 , 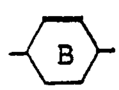 and 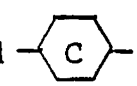 are all 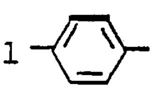 ).

2.02 g of 4-(4′-octyloxy-4-biphenyloxycarbonyl)benzoic acid was dissolved in 100 ml of tetrahydrofuran at 90°C. The solution was concentrated under reduced pressure. The residue was mixed with 30 ml of thionyl chloride. The mixture was heated at 90°C for 2.5 hours with stirring. The reaction mixture was concentrated under reduced pressure. To the residue was added toluene, and the mixture was concentrated under reduced pressure. This procedure was repeated several times. The acid chloride obtained as the residue was dissolved in 35 ml of dry tetrahydrofuran. To the solution were added at room temperature, with stirring, a solution of 1.35 g of the (2S,3S)-4-(4-methoxybenzyloxy)-2,3-dimethoxybutanol obtained in Example 17 i) and 2.0 ml of pyridine in 35 ml of dry tetrahydrofuran. The mixture was stirred for 3 days at room temperature. The insoluble materials were removed by filtration. The filtrate was concentrated under reduced pressure. The residue was subjected to column chromatography using a silica gel (150 g) and carbon tetrachloride-ethyl acetate (5:1). The relevant fractions were concentrated and the residue was recrystallized from ethanol to obtain 0.68 g of the title compound as a colorless needles.

The elemental analysis of the compound is shown below.

Elemental analysis

| | | |
|---|---|---|
| Calcd. for $C_{42}H_{50}O_9$ (698.854): | C, 72.18; | H, 7.21 |
| Found : | C, 71.92; | H, 7.12 |

Example 19

Preparation of 4′-octyloxy-4-biphenyl ester of 4-[(2S,3S)-4-(4-methoxybenzyloxy)-2,3-dimethoxybutoxy]-benzoic acid (a compound of the general formula I′ in which $R_1$ is n-$C_8H_{17}$, $R_2$ and $R_3$ are both $OCH_3$, $R_4$ is

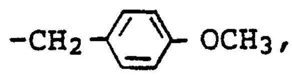

$Q_1$ is -O-, $Q_2$ is -O-, $Q_3$ is -O-, X is a single bond, Y is $-O-\overset{\overset{O}{\|}}{C}-$, n is 1, and

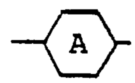 ,

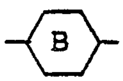 and 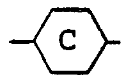 are all 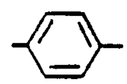 ).

To 0.93 g of the (2S,3S)-4-(4-methoxybenzyloxy)-2,3-dimethoxybutanol obtained in Example 17 i) and a solution of 1.43 g of 4′-octyloxy-4-biphenyl ester of 4-hydroxybenzoic acid dissolved in 50 ml of dry tetrahydrofuran were added at room temperature, with stirring, 0.97 g of triphenylphosphine and then a solution of 0.65 g of diethyl azodicarboxylate dissolved in 15 ml of dry tetrahydrofuran. The mixture was stirred overnight at room temperature. The reaction mixture was concentrated under reduced pressure. The residue was mixed with carbon tetrachloride. The insoluble materials were removed by filtration. The filtrate was concentrated under reduced pressure. The residue was subjected to column chromatography using a silica gel (200 g) and chloroform-ethyl acetate (30:1). The relevant fractions were subjected to column chromatography using a silica gel (50 g) and n-hexane-ethyl acetate (3:1) for separation and purification. The relevant fractions were concentrated and the residue was recrystallized from isopropanol to obtain 0.222 g of the title compound as a colorless needles.

The IR and $^1$H-NMR spectral data and elemental analysis of the compound are shown below.

IR $\nu_{max}^{KBr}$ $cm^{-1}$ : 1725, 1605, 1510, 1495, 1470

$^1$H-NMR (90 MHz, $CDCl_3$)

δ: 0.893 (3H, t, J=6Hz, $CH_2CH_3$), 1.1-2.0 (m), 3.3-4.3 (m), 3.474 (3H, s, $OCH_3$), 3.517 (3H, s, $OCH_3$), 3.802 (3H, s, aromatic $OCH_3$), 4.498 (2H, s,benzylic $CH_2$), 6.6-8.2 (16H, m, aromatic H)

Elemental analysis

| | | |
|---|---|---|
| Calcd. for $C_{41}H_{50}O_8$ (670.843): | C, 73.41; | H, 7.51 |
| Found : | C, 73.35; | H, 7.47 |

Example 20

Preparation of 4′-octyloxy-4-biphenyl ester of 4-[(2S,3S)-4-butoxy-2,3-dimethoxybutoxy]benzoic acid (a compound of the general formula I′ in which $R_1$ is $C_8H_{17}$, $R_2$ and $R_3$ are both $OCH_3$, $R_4$ is $C_4H_9$, $Q_1$ and $Q_2$ are both -O-, $Q_3$ is -O-, X is a single bond, Y is

O

-O- C -, n is 1, and A , B and C are all ).

i) Preparation of (2S,3S)-4-butoxy-2,3-dimethoxybutanol

Said compound was prepared from the (2S,3S)-2,3-dimethoxy-1,4-butanediol mentioned in Example 17 i), in accordance with the following scheme.

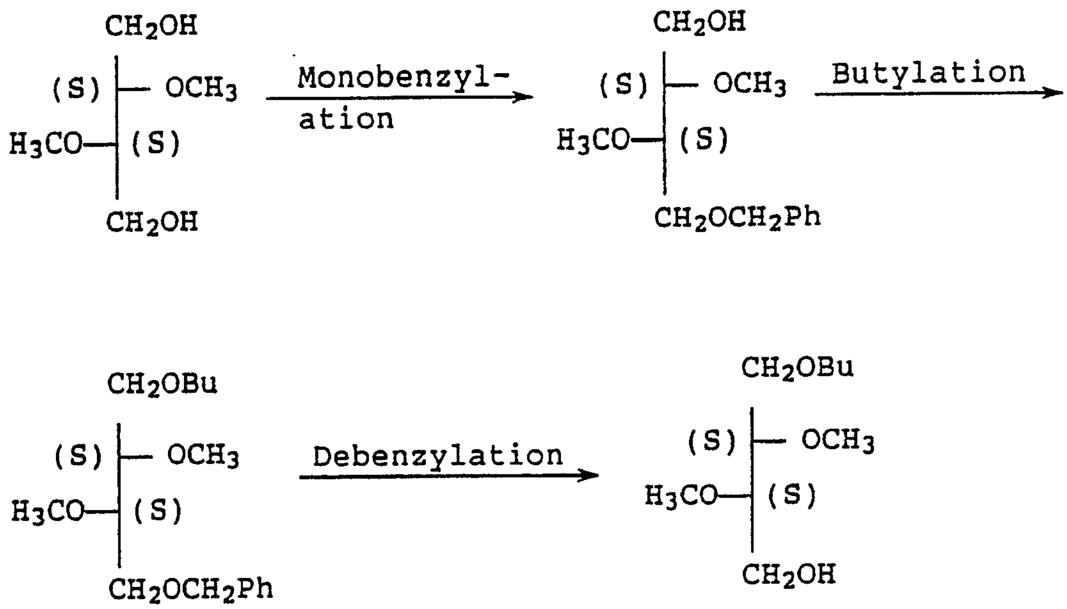


A solution of 18.59 g of (2S,3S)-2,3-dimethoxy-1,4-butanediol dissolved in 100 ml of dry tetrahydrofuran was added to a suspension of 4.99 g of 60% oily sodium hydride in 150 ml of dry tetrahydrofuran, at 0° C with stirring. To the mixture were added at 0° C, with stirring, 3.44 g of tetrabutylammonium iodide and a solution of 21.25 g of benzyl bromide in 80 ml of dry tetrahydrofuran. The mixture was stirred overnight at room temperature. The insoluble materials were removed by filtration using a filtering aid (High Flow Super Cell®). The filtrate was concentrated under reduced pressure. The residue was subjected to column chromatography using a silica gel (300 g) and ethyl acetate-carbon tetrachloride (2:1). 20.58 g of (2S,3S)-4-benzyloxy-2,3-dimethoxybutanol was obtained as a colorless oil from the relevant fractions.

IR $\nu_{max}^{neat}$ $cm^{-1}$ : 3450
$^1$H-NMR (90 MHz, $CDCl_3$)
δ: 3.456 (6H, s, $OCH_3$), 3.25-3.9 (m), 4.543 (2H, s, benzylic $CH_2$), 7.318 (5H, m, aromatic H)

14.29 g of the above (2S,3S)-4-benzyloxy-2,3-dimethoxybutanol was suspended in 150 ml of dry tetrahydrofuran at 0° C with stirring. To the mixture were added at 0° C, with stirring, 3.44 g of tetrabutylammonium iodide and a solution of 21.25 g of benzyl bromide in 80 ml of dry tetrahydrofuran. The mixture was stirred overnight at room temperature. The insoluble materials were removed by filtration using a filtering aid (High Flow Super Cell®). The filtrate was concentrated under reduced pressure. The residue was subjected to column chromatography using a silica gel (300 g) and ethyl acetate-carbon tetrachloride (2:1). 20.58 g of (2S,3S)-4-benzyloxy-2,3-dimethoxybutanol was obtained as a colorless oil from the relevant fractions.

IR $\nu_{max}^{neat}$ $cm^{-1}$ : 3450
$^1$H-NMR (90 MHz, $CDCl_3$)
δ: 3.456 (6H, s, $OCH_3$), 3.25-3.9 (m), 4.543 (2H, s, benzylic $CH_2$), 7.318 (5H, m, aromatic H)

14.29 g of the above (2S,3S)-4-benzyloxy-2,3-dimethoxybutanol was dissolved in 150 ml of dry tetrahydrofuran. Thereto was added 21.9 g of butyl iodide. Further there was added by small portions 3.60 g of 60% oily sodium hydride at 0° C with stirring. The mixture was stirred overnight at room temperature. 22.12 g of butyl iodide was added, and then 3.61 g of 60% oily sodium hydride was added by small portions at room temperature with stirring. The mixture was stirred overnight at room temperature. Thereafter, 1.19 g of 60% oily sodium hydride was added by small portions at room temperature with stirring. The mixture was stirred for 3 days at room temperature. The insoluble materials were removed by filtration using a filtering aid (High Flow Super Cell®). The filtrate was concentrated under reduced pressure.

The residue was mixed with carbon tetrachloride. The insoluble materials were removed by filtration. The filtrate was concentrated under reduced pressure. The residue was subjected to column chromatography using a silica gel (300 g) and carbon tetrachloride-ethyl acetate (4:1). 14.65 g of (2S,3S)-1-benzyloxy-4-butoxy-2,3-dimethoxybutane was obtained as a colorless oil from the relevant fractions.

$^1$H-NMR (90 MHz, $CDCl_3$)

δ: 0.903 (3H, t, J=6Hz, $CH_2$-$CH_3$), 1.1-1.7 (m), 3.2-3.7 (m), 3.435 (3H, s, $OCH_3$), 3.465 (3H, s, $OCH_3$), 4.539 (2H, s, benzylic $CH_2$), 7.317 (5H, s, aromatic H)

20 g of the above (2S,3S)-1-benzyloxy-4-butoxy-2,3-dimethoxybutane was dissolved in 400 ml of ethyl acetate. To the solution were added 4.08 g of 5% Pd-C and 1.0 ml of concentrated hydrochloric acid, and the butane derivative was subjected to catalytic hydrogen reduction at room temperature under atmospheric pressure. The catalyst was removed by filtration, and the filtrate was concentrated under reduced pressure. The residue was subjected to distillation under reduced pressure (b.p. 124-125 °C/10 mmHg) to obtain 12.37 g of the title compound as a colorless oil.

IR $\nu_{max}^{neat}$ $cm^{-1}$ : 3450, 1465, 1100

$^1$H-NMR (90 MHz, $CDCl_3$)

δ: 0.916 (3H, t, J=6Hz, $CH_2$-$CH_3$), 1.1-1.75 (m), 3.2-3.8 (m)

ii) Etherification

In 30 ml of dry tetrahydrofuran were dissolved 1.41 g of 4′-octyloxy-4-biphenyl ester of 4-hydroxybenzoic acid and 0.69 g of the (2S,3S)-4-butoxy-2,3-dimethoxybutanol obtained in the above i). To the solution was added 1.32 g of triphenylphosphine. Then, there was added a solution of 0.89 g of diethyl azodicarboxylate in 20 ml of dry tetrahydrofuran, at room temperature with stirring. Further, 0.18 g of triphenylphosphine was added. The mixture was stirred overnight at room temperature. The reaction mixture was concentrated under reduced pressure. The residue was mixed with isopropyl ether. The resulting crystal was removed by filtration, and the filtrate was concentrated under reduced pressure. The residue was subjected to column chromatography using a silica gel (200 g) and carbon tetrachloride-ethyl acetate (5:1). The relevant fractions were subjected to column chromatography using a silica gel (150 g) and chloroform. The relevant fractions were further subjected to column chromatography using a silica gel (150 g) and n-hexane-ethyl acetate (4:1) for separation and purification. The relevant fractions were concentrated and the residue was recrystallized from ethanol to obtain 0.77 g of the title compound as a colorless crystal.

The IR and $^1$H-NMR spectral data and elemental analysis of the compound are shown below.

IR $\nu_{max}^{KBr}$ $cm^{-1}$ : 1725, 1600, 1505, 1490, 1465

$^1$H-NMR (90 MHz, $CDCl_3$)

δ: 0.85-1.05 (6H, m, $CH_2CH_3$), 1.1-2.0 (m), 3.3-4.4 (m), 3.479 (3H, s, $OCH_3$), 3.544 (3H, s, $OCH_3$), 6.8-8.25 (12H, m, aromatic H)

Elemental analysis

| | | |
|---|---|---|
| Calcd. for $C_{37}H_{50}O_7$ (606.807): | C, 73.24; | H, 8.31 |
| Found : | C, 73.39; | H, 8.42 |

Example 21

Preparation of 4-octyloxyphenyl ester of 4′-[(2S,3S)-4-butoxy-2,3-dimethoxybutoxy]-4-biphenylcarboxylic acid (a compound of the general formula I′ in which $R_1$ is $C_8H_{17}$, $R_2$ and $R_3$ are both $OCH_3$, $R_4$ is $C_4H_9$, $Q_1$ and $Q_2$ are both -O-, $Q_3$ is -O-, X is

$-O-\overset{O}{\overset{\|}{C}}-$, Y is a single bond, n is 1 and

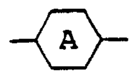 , 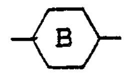 and 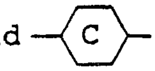

are all 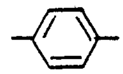 ).

In 30 ml of dry tetrahydrofuran were dissolved 0.71 g of the (2S,3S)-4-butoxy-2,3-dimethoxybutanol obtained in Example 20 i) and 1.40 g of 4-octyloxyphenyl ester of 4′-hydroxy-4-biphenylcarboxylic acid. To the solution was added 1.31 g of triphenylphosphine. Thereto was added a solution of 0.89 g of diethyl azodicarboxylate in 10 ml of dry tetrahydrofuran, at room temperature with stirring. 0.18 g of triphenylphosphine was further added. The mixture was stirred overnight at room temperature. Then, 0.13 g of diethyl azodicarboxylate and 0.09 g of triphenylphosphine were added, and the mixture was stirred overnight at room temperature. The reaction mixture was concentrated under reduced pressure. The residue was mixed with isopropyl ether. The resulting crystal was removed by filtration and the filtrate was concentrated under reduced pressure. The residue was subjected to column chromatography using a silica gel (300 g) and n-hexane-ethyl acetate (3:1). The relevant fractions were subjected to column chromatography using a silica gel (50 g) and dichloroethane and then carbon tetrachlorideethyl acetate (5:1) for separation and purification. The relevant fractions were concentrated and the residue was recrystallized from ethanol to obtain 0.62 g of the title compound as a colorless needles.

The elemental analysis of the compound is shown below.
Elemental analysis

| | | |
|---|---|---|
| Calcd. for $C_{37}H_{50}O_7$ (606.807): | C, 73.24; | H, 8.31 |
| Found : | C, 73.44; | H, 8.38 |

Examples 22-26 and 28-31

The same procedure as in Example 3 was repeated using the (2S,3S)-3-methoxy-2-methylbutanol obtained in Example 1 i) and various skeletal component compounds, to obtain corresponding compounds of these Examples.

Example 27

The same procedure as in Example 1 was repeated using the (2S,3S)-3-methoxy-2-methylbutanol obtained in Example 1 i) and 4′-(trans-4-pentylcyclohexyl)biphenyl-4-carboxylic acid, to obtain a corresponding compound of this Example.

The following Examples 32 to 43 show cases of synthesis of compounds I wherein n is 0 (zero).

Example 32

Preparation of 4′-octyloxy-4-biphenyl ester of monomethyl (2R,3R)-2,3-dimethoxysuccinate (a compound of the general formula I″ in which $R_1$ is n-$C_8H_{17}$, $R_2$ and $R_3$ are both $OCH_3$, $R_4$ is $CH_3$, $Q_1$ and $Q_2$ are both -O-, $Q_3$ are $-\overset{O}{\overset{\|}{C}}-O-$, X is a single bond, and

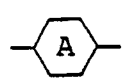 and 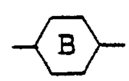 are

both 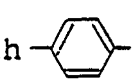 ).

(i) Preparation of monomethyl (2R,3R)-2,3-dimethoxysuccinate

The optically active carboxylic acid can be prepared through the following scheme.

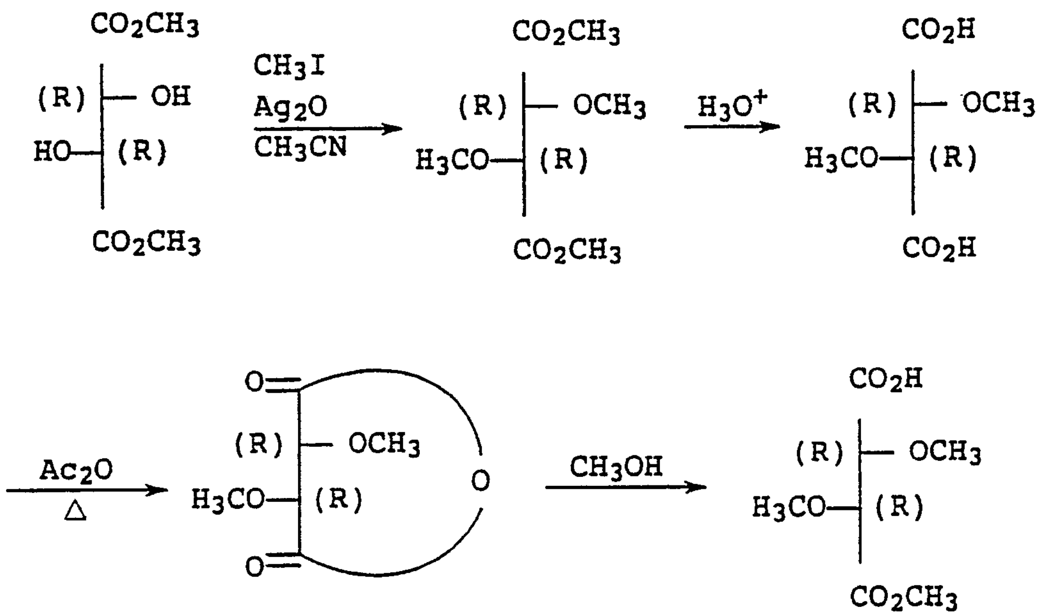


Dimethyl (2R,3R)-2,3-dihydroxysuccinate, 50.0 g, was dissolved in 1 ℓ of acetonitrile, to which 200 g of silver oxide ($Ag_2O$) and 185 ml of methyl iodide were added with stirring at room temperature. After stirring at room temperature for 3 days, insoluble materials were filtered off, and the filtrate was concentrated under reduced pressure. The residue was distilled under reduced pressure (b.p. 125-127 °C/4 mmHg), to give 52.3 g of a dimethyl ether derivative as a colorless oil.

$^1$H-NMR (90 MHz, $CDCl_3$)

δ: 3.47 (6H, s, $OCH_3$), 4.82 (6H, s, $CO_2CH_3$), 4.24 (2H, s, CH)

IR $\nu_{max}^{neat}$ $cm^{-1}$ : 1755, 1735

The product, 10.18 g, was dissolved in 50 ml of concentrated hydrochloric acid, and heated at 80 °C for 2 hours with stirring. The reaction mixture was concentrated under reduced pressure, and the residue was mixed with toluene and concentrated again under reduced pressure. This procedure was repeated several times, to give a crude dicarboxylic acid as a white crystal. The dicarboxylic acid was submitted to the next procedure, the intramolecular acid anhydride preparation, without being purified. The crude dicarboxylic acid was dissolved in 100 ml of acetic anhydride, and heated at 80 °C for one hour with stirring. The reaction mixture was concentrated under reduced pressure, the residue was mixed with toluene and concentrated again under reduced pressure. The procedure was repeated several times, to give an acid anhydride as a crystal. Recrystallization from benzene gave a purified acid anhydride as a colorless crystal, mp 130.5 - 131.5 °C.

$^1$H-NMR (90 MHz, $CDCl_3$)

δ: 3.57 (6H, s, $OCH_3$), 4.93 (2H, s, CH)

IR $\nu_{max}^{KBr}$ $cm^{-1}$ : 1790

Elemental analysis

| | | |
|---|---|---|
| Calcd. for $C_6H_8O_5$ (161.127): | C, 45.01; | H, 5.04 |
| Found : | C, 44.70; | H, 5.02 |

The acid anhydride, 11.54 g, was dissolved in 90 ml of methanol, and stirred at room temperature for 2.5 hours. The reaction mixture was concentrated under reduced pressure, the residue was distilled under reduced pressure (b.p. 127.5-129 °C/1 mmHg), to give 12.88 g of monomethyl (2R,3R)-2,3-dimethoxysuccinate as a syrup.

$^1$H-NMR (90 MHz, $CDCl_3$)
δ: 3.50 (6H, s, $OCH_3$), 3.83 (3H, s, $CO_2CH_3$)

IR $\nu_{max}^{KBr}$ $cm^{-1}$ : 2700-2400 ($CO_2H$), 1760

(ii) Esterification

The monomethyl (2R,3R)-2,3-dimethoxysuccinate obtained in (i), 1.053 g, was dissolved in 20 ml of toluene, to which 2.0 ml of thionyl chloride was added, and the mixture was heated at 70°C for 1.5 hours with stirring. The reaction mixture was concentrated under reduced pressure, the residue was mixed with toluene, and concentrated again under reduced pressure. The procedure was repeated several times, to give a crude acid chloride as a colorless oil.

IR $\nu_{max}^{neat}$ $cm^{-1}$ : 1790

The solution of the acid chloride in benzene (50 ml) was added to a solution of 1.01 g of 4′-octyloxy-4-biphenol and 0.96 g of pyridine in benzene (50 ml) at 0°C with stirring. After stirring at 0°C for 10 minutes after completion of the dropwise addition, stirring was continued at room temperature for 2 days. Insoluble materials were filtered off, the filtrate was concentrated under reduced pressure, and the residue was subjected to column chromatography on a silica gel (80 g) using n-hexane-ethyl acetate (2:1). From the relevant fractions, 0.62 g of the title compound (recrystallized from isopropanol) was obtained as a colorless powdery crystal.

The IR and $^1$H-NMR spectral data and elemental analysis of the title compound are shown in the following.

IR $\nu_{max}^{neat}$ $cm^{-1}$ : 1700, 1760, 1605, 1495, 1465
$^1$H-NMR 90 MHz, $CDCl_3$)
δ: 0.890 (3H, t, J=6Hz, $CH_2CH_3$), 1.15-2.0 (m), 3.565 (3H, s, $OCH_3$), 5.570 (3H, s, $OCH_3$), 3.841 (3H, s, $CO_2CH_3$), 3.993 (2H, T, J=6.4Hz, $OCH_2CH_2$), 4.41 (1H, d, J=3.5Hz, CH), 4.48 (1H, d, J=3.5Hz, CH), 6.85-7.65 (8H, m, aromatic H)
Elemental analysis

| | | |
|---|---|---|
| Calcd. for $C_{27}H_{36}O_7$ (472.579): | C, 68.62; | H, 7.68 |
| Found : | C, 68.50; | H, 7.68 |

Example 33

Preparation of 4′-octyloxy-4-biphenyl ester of monobutyl (2R,3R)-2,3-dimethoxysuccinate (a compound of the general formula I″ in which $R_1$ is n-$C_8H_{17}$, $R_2$ and $R_3$ are both $OCH_3$, $R_4$ is $C_4H_9$, $Q_1$ and $Q_2$ are -O-, $Q_3$ is

$-\overset{O}{\overset{\|}{C}}-O-$, X is a single bond, and

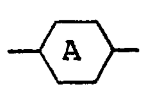 and 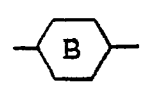 are

both 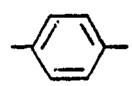).

(i) Preparation of monobutyl (2R,3R)-2,3-dimethoxysuccinate

In a similar manner to that in Example 32-(i), (2R,3R)-2,3-dimethoxysuccinic acid anhydride was allowed to react with n-butanol, to give the title compound as a syrup:

IR $\nu_{max}^{neat}$ $cm^{-1}$ : 2700-2400, 1745.

(ii) Esterification

The monobutyl (2R,3R)-2,3-dimethoxysuccinate obtained in (i), 3.0 g, was dissolved in 50 ml of benzene, to which 10 ml of thionyl chloride was added, and the mixture was heated at 80 °C for 2 hours with stirring. The reaction mixture was concentrated under reduced pressure, and the residue was mixed with toluene and concentrated again under reduced pressure. The procedure was repeated several times, to give a crude acid chloride as a colorless oil.

IR $\nu_{max}^{neat}$ $cm^{-1}$ : 1790

The solution of the acid chloride in benzene (100 ml) was added to a solution of 2.15 g of 4′-octyloxy-4-biphenol and 3.5 ml of pyridine in ethyl acetate (100 ml) at room temperature with stirring. After stirring at room temperature for 3 days, insoluble materials were filtered off, the filtrate was concentrated under reduced pressure, and the residue was subjected to column chromatography on a silica gel (150 g) using n-hexane-ethyl acetate (3:1). From the relevant fractions, 2.58 g of the title compound was obtained as a colorless oil.

The IR and $^1$H-NMR spectra data and elemental analysis of the title compound are shown in the following.

IR $\nu_{max}^{neat}$ $cm^{-1}$ : 1775, 1755, 1605, 1495, 1465

$^1$H-NMR (90 MHz, $CDCl_3$)

δ: 0.75-1.1 (6H, m, $CH_2CH_3$), 1.15-2.0 (m), 3.56 (6H, s, $OCH_3$), 3.98 (2H, t, J = 6Hz, $OCH_2CH_2$), 4.26 (2H, t, J = 6Hz, $OCH_2CH_2$), 4.38 (1H, d, J = 3Hz, CH), 4.48 (1H, d, J = 3Hz, CH), 6.85-7.75 (8H, m, aromatic H)

Elemental analysis

| | | |
|---|---|---|
| Calcd. for $C_{30}H_{42}O_7$ (514.659): | C, 70.01; | H, 8.23 |
| Found : | C, 69.98; | H, 8.43 |

Example 34

Preparation of 4-(4′-octyloxy-4-biphenylcarbonyloxy)phenyl ester of monomethyl (2R,3R)-2,3-dimethoxysuccinate (a compound of the general formula I′ in which $R_1$ is n-$C_8H_{17}$, $R_2$ and $R_3$ are both $OCH_3$, $R_4$ is $CH_3$, $Q_1$ and $Q_2$ are both -O-, $Q_3$ is

O
||
- C -O-, X is a single bond, Y is
O
||
- C -O-, and

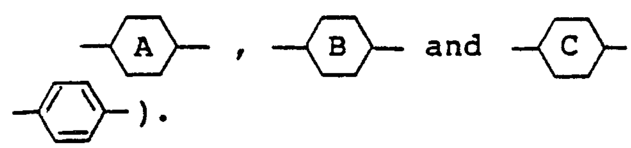
, and

are all ).

The monomethyl (2R,3R)-2,3-dimethoxysuccinate obtained in Example 32 (i), 1.053 g, was dissolved in 50 ml of benzene, to which 3.8 ml of thionyl chloride was added, and the mixture was heated at 80 °C for 3 hours with stirring. The reaction mixture was concentrated under reduced pressure, and the residue was mixed with benzene and concentrated again under reduced pressure. The procedure was repeated several times, to give a crude acid chloride as a residue. The solution of the acid chloride in benzene (60 ml) was added to a solution of 1.6 g of 4-(4′-octyloxy-4-biphenylcarbonyloxy)phenol and 1.5 ml of pyridine in dry tetrahydrofuran (80 ml) at room temperature with stirring. After stirring at room temperature overnight, insoluble materials were filtered off, the filtrate was concentrated under reduced pressure, and the residue

was subjected to column chromatography on a silica gel (150 g) using chloroformether (15:1). From the relevant fractions, 0.67 g of the title compound (recrystallized from EtOH-n-hexane) was obtained as colorless needles.

The IR and $^1$H-NMR spectral data and elemental analysis of the title compound are shown in the following.

IR $\nu_{max}^{KBr}$ $cm^{-1}$ : 1775, 1765, 1735, 1605, 1505

$^1$H-NMR (90 MHz, $CDCl_3$)

δ: 0.75-1.05 (3H, m, $CH_2CH_3$), 1.15-2.0 (m), 3.561 (3H, s, $OCH_3$), 3.568 (3H, s, $OCH_3$), 3.846 (3H, s, $CO_2CH_3$), 4.019 (2H, t, J=6Hz, $OC\underline{H}_2CH_2$), 4.40 (1H, d, J=3.5Hz, CH), 4.48 (1H, d, J=3.5Hz, CH), 6.9-8.3 (12H, m, aromatic H)

Elemental analysis

| | | |
|---|---|---|
| Calcd. for $C_{34}H_{40}O_9$ (592.68): | C, 68.90; | H, 6.80 |
| Found : | C, 69.08; | H, 6.77 |

Example 35

Preparation of 4-(4′-octyloxy-4-biphenylcarbonyloxy)phenyl ester of monobutyl (2R,3R)-2,3-dimethoxysuccinate (a compound of the general formula I′ in which $R_1$ is n-$C_8H_{17}$, $R_2$ and $R_3$ are both $OCH_3$, $R_4$ is $C_4H_9$, $Q_1$ and $Q_2$ are both -O-, $Q_3$ is

$-\overset{O}{\overset{\|}{C}}-O-$, X is a single bond, Y is

$-\overset{O}{\overset{\|}{C}}-O-$, and

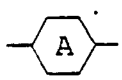, 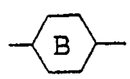 and 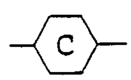

are all 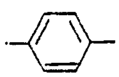).

The monobutyl (2R,3R)-2,3-dimethoxysuccinate obtained in Example 33-(i), 2.036 g, was dissolved in 40 ml of benzene, to which 4.0 ml of thionyl chloride was added, and the mixture was heated at 80° C for 2 hours with stirring. The reaction mixture was concentrated under reduced pressure, and the residue was mixed with toluene and concentrated again under reduced pressure. The procedure was repeated several times, to give an acid chloride as a residue. The solution of the acid chloride in benzene (60 ml) was added to a solution of 2.0 g of 4-(4′-octyloxy-4-biphenylcarbonyloxy)phenol and 4.0 ml of pyridine in dry tetrahydrofuran (100 ml) at room temperature with stirring. After stirring at room temperature overnight, insoluble materials were filtered off, the filtrate was concentrated under reduced pressure, and the residue was subjected to column chromatography on a silica gel (150 g) using chloroform-ether (20:1). The relevant fractions were again subjected to column chromatography on a silica gel (150 g) using n-hexane-ethyl acetate (2:1) for separation and purificatioin. From the relevant fractions, 1.37 g of the title compound (recrystallized from ethanol) was obtained as a colorless powdery crystal.

The IR and $^1$H-NMR spectral data and elemental analysis of the title compound are shown in the following.

IR $\nu_{max}^{KBr}$ $cm^{-1}$ : 1775, 1760, 1730, 1605, 1505

$^1$H-NMR (90 MHz, $CDCl_3$)

δ: 0.8-1.1 (6H, m, $CH_2CH_3$), 1.15-2.0 (m), 3.565 (6H, s, $OCH_3$), 4.017 (2H, t, J=6Hz, $OCH_2$), 4.27 (2H, t, J=6Hz, $OC\underline{H}_2CH_2$), 4.39 (1H, d, J=3Hz, CH), 4.49 (1H, d, J=3Hz, CH), 6.9-8.3 (12H, m, aromatic H)

Elemental analysis

| | | |
|---|---|---|
| Calcd. for $C_{37}H_{46}O_9$ (634.767): | C, 70.01; | H, 7.30 |
| Found : | C, 69.95; | H, 7.34 |

Example 36

Preparation of 4-(4′-octyloxy-4-biphenyloxycarbonyl)phenyl ester of monomethyl (2R,3R)-2,3-dimethoxysuccinate (a compound of the general formula I′ in which $R_1$ is n-$C_8H_{17}$, $R_2$ and $R_3$ are both $OCH_3$, $R_4$ is $CH_3$, $Q_1$ and $Q_2$ are -O-, $Q_3$ is

$-\overset{O}{\overset{\|}{C}}$ -O-, X is a single bond, Y is

-O- $\overset{O}{\overset{\|}{C}}$ -, and

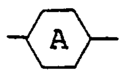 , 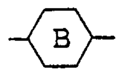 and 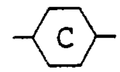 are all

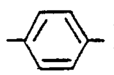 ).

To 2.63 g of the monomethyl (2R,3R)-2,3-dimethoxysuccinate obtained in Example 32-(i) was added 15 ml of thionyl chloride, and the mixture was heated at 80°C for 1.5 hours with stirring. The reaction mixture was concentrated under reduced pressure, and the residue was mixed with toluene and concentrated again under reduced pressure. The procedure was repeated several times, to give a crude acid chloride as a residue. The solution of the acid chloride in dry tetrahydrofuran (30 ml) was added to a solution of 2.88 g of 4-(4′-octyloxy-4-biphenylcarbonyloxy)phenol and 5.1 ml of pyridine in dry tetrahydrofuran (30 ml) at room temperature with stirring. After stirring at room temperature for 3 days, insoluble materials were filtered off, the filtrate was concentrated under reduced pressure, and the residue was subjected to column chromatography on a silica gel (200 g) using chloroform-ether (30:1). From the relevant fractions, 3.05 g of the title compound (recrystallized from ethanol) was obtained as colorless needles.

The IR and $^1$H-NMR spectral data and elemental analysis of the title compound are shown in the following.

IR $\nu_{max}^{KBr}$ cm$^{-1}$ : 1780, 1770, 1740, 1605, 1505

$^1$H-NMR (90 MHz, $CDCl_3$)

δ: 0.893 (3H, t, J=6Hz, $CH_2$-$CH_3$), 1.1-2.1 (12H, m, $CH_2$), 3.570 (6H, s, $OCH_3$), 3.851 (3H, s, $CO_2CH_3$), 4.00 (2H, t, J=5.93, $CH_2O$), 4.41 (1H, d, J=3.73Hz, CH), 4.50 (1H, d, J=3.73Hz, CH), 6.8-8.4 (12H, m, aromatic H)

Elemental analysis

| | | |
|---|---|---|
| Calcd. for $C_{34}H_{40}O_9$ (592.686): | C, 68.90; | H, 6.80 |
| Found : | C, 68.79; | H, 6.87 |

Example 37

Preparation of 4-(4′-octyloxy-4-biphenylcarbonyloxy)phenyl ester of monomethyl (2S,3R)-2,3-dimethoxysuccinate (a compound of the general formula I′ in which $R_1$ is n-$C_8H_{17}$, $R_2$ and $R_3$ are both $OCH_3$, $R_4$ is $C_4H_9$, $Q_1$ and $Q_2$ are both -O-, $Q_3$ is

$-\overset{O}{\overset{\|}{C}}$ -O-, X is a single bond, Y is

$-\overset{O}{\overset{\|}{C}}$ -O-, and

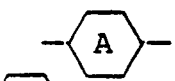 , 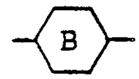 and 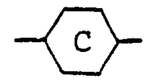

are all 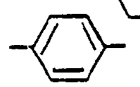 ).

(i) Preparation of monomethyl (2S,3R)-2,3-dimethoxysuccinate

The optically active compound was prepared through the following scheme.

Benzyl ester of D-erythro-hexo-2-enalo-1,4-lactone, 55.8 g, and 40.0 g of calcium carbonate were suspended in 1 ℓ of water and stirred for 30 minutes, to which 40 ml of 30% hydrogen peroxide solution was added dropwise at 0 °C. The mixture was stirred at room temperature overnight. To the reaction mixture was added 8.0 g of activated carbon and the mixture was heated at 80 to 90 °C for one hour. Insoluble materials were filtered off, and the filtrate was concentrated to give 31.2 g of a half ester calcium salt as a colorless powder. This calcium salt, 73.2 g of sodium hydrogen-carbonate, and 72.4 ml of methyl iodide were suspended in 200 ml of dimethylacetamide (DMA), and the mixture

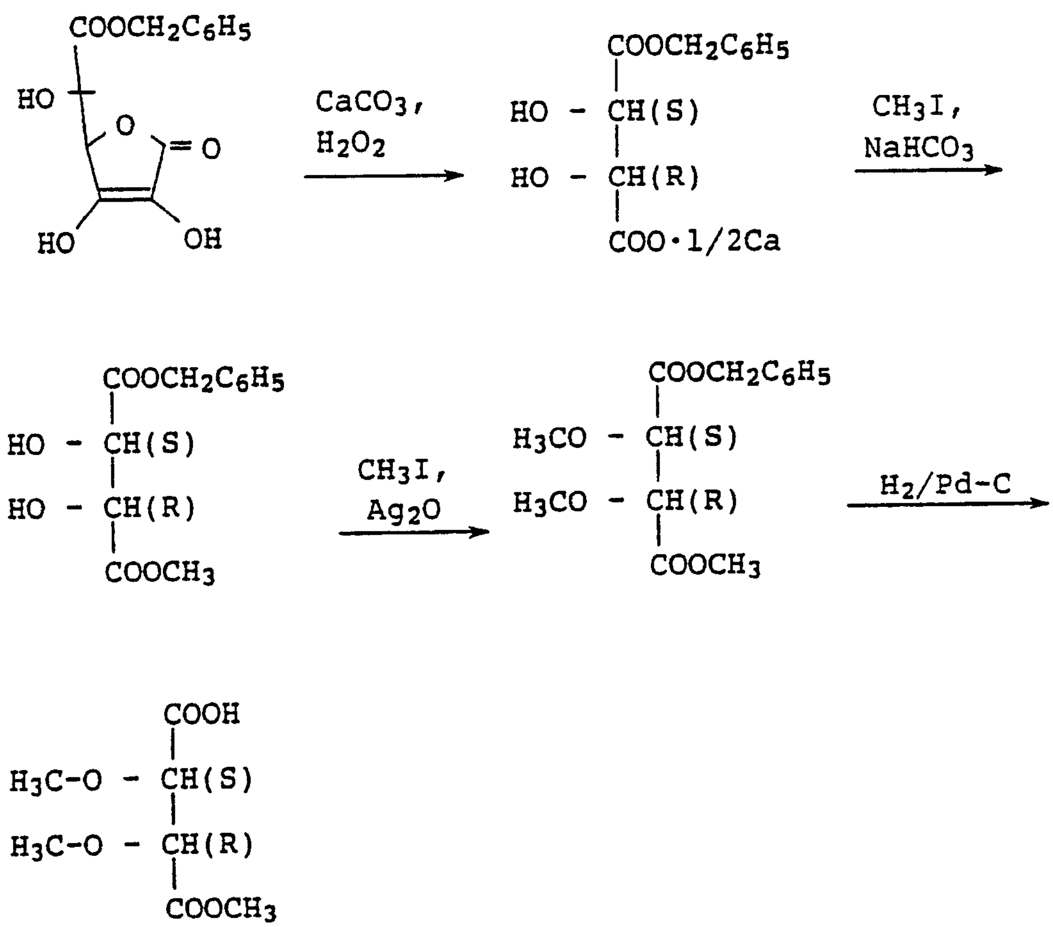


was stirred at room temperature for 2 days. The reaction mixture was added to 1 ℓ of ethyl acetate, insoluble materials were filtered off, and the filtrate was concentrated. The residue was subjected to column chromatography (silica gel, developing solvent: chloroform-ethyl acetate (3:1)) for separation and purification, to give 11.7 g of a methyl ester. This methyl ester, 10.0 g, was dissolved in 200 ml of acetonitrile, to which 27.3 g of silver oxide and 13.1 ml of methyl iodide were added at 0 °C with stirring, and the mixture was stirred at room temperature overnight. Insoluble materials were filtered off, the filtrate was concentrated, and the residue was distilled under reduced pressure (4 mmHg, 170 °C), to give 6.4 g of a methyl ether. Finally, catalytic hydrogenation (5%Pd-C 0.6 g, reaction solvent: ethyl acetate) gave 4.0 g of monoethyl (2S,3R)-2,3-dimethoxysuccinate.

The IR and $^1$H-NMR spectral data of this compound are shown in the following.

IR $\nu_{max}^{neat}$ cm$^{-1}$ : 1740
$^1$H-NMR (90 MHz, $CDCl_3$)
δ: 3.6-3.7 (6H, s), 3.8 (3H, s), 4.2-4.3 (2H, d, d), 8.6 (1H, br, s)

(ii) Esterification

To 1.0 g of monomethyl (2S,3R)-2,3-dimethoxysuccinate obtained in (i) was added 7.2 ml of thionyl chloride, and the mixture was heated at 80° C for 3 hours with stirring. Excess thionyl chloride was removed by taking advantage of azeotropy with toluene to give a corresponding acid chloride. The solution of the acid chloride in dry tetrahydrofuran (a small amount) was added dropwise to a solution of 1.9 g of 4-(4′-octyloxy-4-biphenylcarbonyloxy)phenol and 1.2 ml of pyridine in dry tetrahydrofuran (40 ml) at room temperature with stirring, and the mixture was stirred at room temperature overnight. Insoluble materials were filtered off, the filtrate was concentrated under reduced pressure, and the residue was subjected to column chromatography (silica gel, developing solvent: chloroform-ethyl acetate (20:1)) for separation and purification. The crude crystal obtained was recrystallized from ethanol to give 1.2 g of the title compound.

The IR and $^1$H-NMR spectral data and elemental analysis of the title compound are shown in the following.

IR $\nu_{max}^{KBr}$ cm$^{-1}$ : 1760, 1730, 1600, 1500
$^1$H-NMR (90 MHz, $CDCl_3$)
δ: 0.8-2.0 (15H, m), 3.6-3.7 (6H, s-s), 3.8 (3H, s), 4.0 (2H, t), 4.4-4.5 (2H, d-d), 6.9-8.3 (12H, m)
Elemental analysis

| | | |
|---|---|---|
| Calcd. for $C_{34}H_{40}O_9$: | C, 68.90; | H, 6.80 |
| Found : | C, 68.99; | H, 6.80 |

Example 38

Preparation of 4-(4′-octyloxy-4-biphenyloxycarbonyl)phenyl ester of monomethyl (2S,3R)-2,3-dimethoxysuccinate (a compound of the general formula I′ in which $R_1$ is $C_8H_{17}$, $R_2$ and $R_3$ are both $OCH_3$, $R_4$ is n-$CH_3$, $Q_1$ and $Q_2$ are -O-, $Q_3$ is

$-\overset{O}{\overset{\|}{C}}-O-$, X is a single bond, Y is

$-O-\overset{O}{\overset{\|}{C}}-$, and

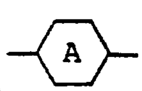 , 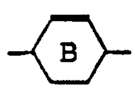 and 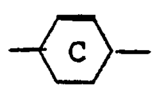 are all

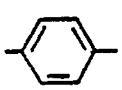 ).

(i) Preparation of monomethyl (2S,3R)-2,3-dimethoxysuccinate

The method for preparation of this optically active compound was in accordance with the method described in Example 37-(i).

(ii) Esterification

An acid chloride was synthesized according to the method described in Example 37-(ii) from 1.0 g of monomethyl (2S,3R)-2,3-dimethoxysuccinate. The acid chloride was added dropwise to a solution of 1.9 g of 4-(4′-octyloxy-4-biphenyloxycarbonyl)phenol and 1.2 ml of pyridine in dry tetrahydrofuran (40 ml) at room temperature with stirring. Insoluble materials were filtered off, the filtrate was concentrated under reduced pressure, and the residue was subjected to column chromatography (silica gel, developing solvent: chloroform) for separation and purification. Two times of recrystallization from ethanol gave 1.0 g of the title compound.

The IR and $^1$H-NMR spectral data and elemental analysis of the title compound are shown in the following.

$^1$H-NMR (90 MHz, $CDCl_3$)

δ: 0.8-2.0 (15H, M), 3.6-3.7 (6H, s), 3.8 (3H, s), 4.0 (2H, t), 4.4-4.5 (2H, d, d), 6.8-8.3 (12H, m)

IR $\nu_{max}^{KBr}$ cm$^{-1}$ : 1730, 1600, 1500

Elemental analysis

| | | |
|---|---|---|
| Calcd. for $C_{34}H_{40}O_9$: | C, 68.90; | H, 6.80 |
| Found : | C, 68.85; | H, 6.81 |

Example 39

Preparation of 4′-(4-n-octyloxyphenyloxycarbonyl)-4-biphenyl ester of monobutyl (2R,3R)-2,3-dimethoxysuccinate (a compound of the general formula I′ in which $R_1$ is n-$C_8H_{17}$, $R_2$ and $R_3$ are both $OCH_3$, $R_4$ is n-$C_4H_9$, $Q_1$ and $Q_2$ are both -O-, $Q_3$ are

O
‖
- C -O-, X is a single bond, and

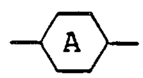 , 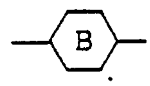 and

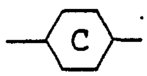 are all 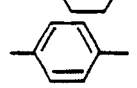 ).

The monobutyl (2R,3R)-2,3-dimethoxysuccinate obtained in Example 33-(i), 3.0 g, was dissolved in 50 ml of benzene, to which 10 ml of thionyl chloride was added, and the mixture was heated at 80 °C for 2 hours with stirring. The reaction mixture was concentrated under reduced pressure, and the residue was mixed with toluene and concentrated again under reduced pressure. The procedure was repeated several times, to give an acid chloride as a colorless oily residue

IR $\nu_{max}^{neat}$ cm$^{-1}$ : 1790

The acid chloride, 1.26 g, and 1.05 g of 4-octyloxyphenyl 4′-hydroxy-4-biphenylcarboxylate were dissolved in 20 ml of dry tetrahydrofuran, to which 0.49 g of pyridine was added at room temperature with stirring. After stirring at room temperature overnight, insoluble materials were filtered off, the filtrate was concentrated under reduced pressure, and the residue was subjected to column chromatography (silica gel, developing solvent: chloroform) for separation and purification. Recrystallization from hot methanol gave 0.88 g of the title compound.

The IR and $^1$H-NMR spectral data and elemental analysis of the title compound are shown in the following.

IR $\nu_{max}^{KBr}$ cm$^{-1}$ : 1780, 1760, 1730, 1605, 1505

$^1$H-NMR (90 MHz, $CDCl_3$)

δ: 0.75-1.1 (6H, m), 1.15-2.0 (14H, m), 3.58 (6H, s), 3.8-4.1 (2H, t), 4.15-4.6 (4H, m), 6.8-8.35 (12H, m)

Elemental analysis

| | | |
|---|---|---|
| Calcd. for $C_{37}H_{46}O_9$ (634.76): | C, 70.01; | H, 7.30 |
| Found : | C, 70.24; | H, 7.38 |

Example 40

Preparation of 4′-(4-n-octyloxyphenylcarbonyloxy)-4-biphenyl ester of monobutyl (2R,3R)-2,3-dimethoxysuccinate (a compound of the general formula I′ in which $R_1$ is n-$C_8H_{17}$, $R_2$ and $R_3$ are both $OCH_3$, $R_4$ is n-$C_4H_9$, $Q_1$ and $Q_2$ are both -O-, $Q_3$ is
$-\overset{\overset{O}{\|}}{C}-O-$, X is a single bond, and

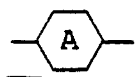 , 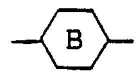 and

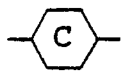 are all 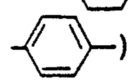).

From 1.26 g of the acid chloride of monobutyl (2R,3R)-2,3-dimethoxysuccinate obtained in Example 33-(i) and 1.05 g of 4′-hydroxy-4-biphenyl 4-octyloxybenzoate, 1.10 g of the title compound was obtained according to the same method as described in Example 39.

The results of elemental analysis of this compound are shown in the following.
Elemental analysis

| | | |
|---|---|---|
| Calcd. for $C_{37}H_{46}O_9$ (634.76): | C, 70.01; | H, 7.30 |
| Found : | C, 69.94; | H, 7.34 |

Example 41

Preparation of 4-(4′-n-octyloxy-4-biphenyl)oxycarbonylphenyl ester of monobutyl (2R,3R)-2,3-dimethoxysuccinate (a compound of the general formula I′ in which $R_1$ is n-$C_8H_{17}$, $R_2$ and $R_3$ are both $OCH_3$, $R_4$ is n-$C_4H_9$, $Q_1$ and $Q_2$ are both -O-, $Q_3$ is
$-\overset{\overset{O}{\|}}{C}-O-$, X is a single bond, and

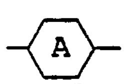 , 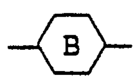 and 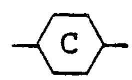 are all

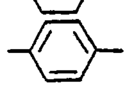).

From 1.26 g of the acid chloride of monobutyl (2R,3R)-2,3-dimethoxysuccinate obtained in Example 33-(i) and 1.05 g of 4′-octyloxy-4-biphenyl 4-hydroxybenzoate, 0.82 g of the title compound was obtained according to the same method as described in Example 39.

The results of elemental analysis of this compound are shown in the following.
Elemental analysis

| | | |
|---|---|---|
| Calcd. for $C_{37}H_{46}O_9$ (634.76): | C, 70.01; | H, 7.30 |
| Found : | C, 70.33; | H, 7.33 |

Example 42

Preparation of 4-(4′-octyloxy-4-biphenyl)oxycarbonylphenyl ester of (2R,3R)-2,3-dimethoxybutyric acid (a compound of the general formula I′ in which $R_1$ is n-$C_8H_{17}$, $R_2$ is $OCH_3$, $R_3$ is $CH_3$ $R_4$ is $CH_3$, $Q_1$ and $Q_2$ are both -O-, $Q_3$ is -O-, X is a single bond, and

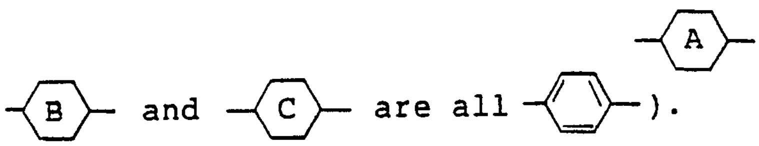

, and are all ).

(i) Preparation of (2S,3R)-2,3-dimethoxybutyric acid

The optically active carboxylic acid was prepared through the following scheme.

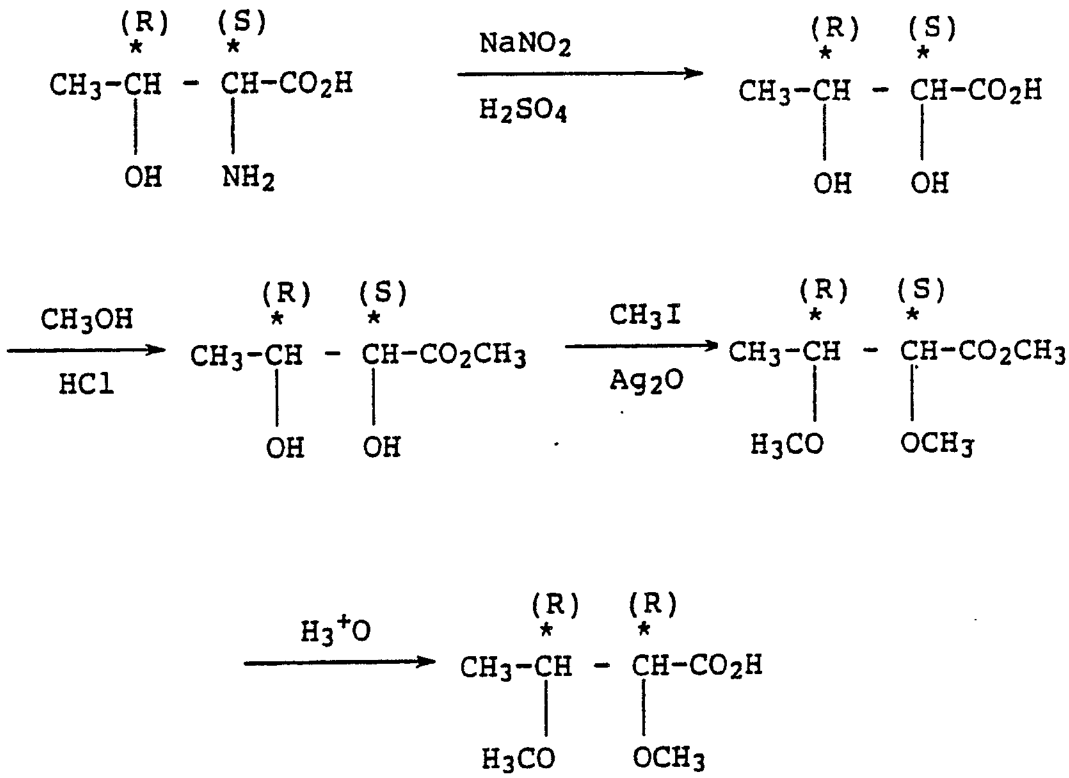


(2S,3R)-2-Amino-3-hydroxybutyric acid, 119.1 g, was dissolved in 1.5 ℓ of 1N sulfuric acid and diazotized by a known method using sodium nitrite. After completion of the reaction, the solvent was evaporated off under reduced pressure. The residue was mixed with 3 ℓ of methanol, insoluble materials were filtered off, and the filtrate was refluxed for 5 hours. The reaction mixture was concentrated under reduced pressure. The residue was distilled under reduced pressure (b.p. 98-102°C/8 mmHg), to give 53.3 g of methyl (2S,3R)-2,3-dihydroxybutyrate. This methyl ester, 13.4 g, was dissolved in 500 ml of acetonitrile, to which 142 g of methyl iodide and 69.5 g of silver oxide were added, and the mixture was stirred at room temperature for 5 days. Insoluble materials were filtered off, and the filtrate was concentrated under reduced pressure. Distillation under reduced pressure of the residue (b.p. 91-95°C/25 mmHg) gave 14.0 g of methyl (2R,3R)-2,3-dimethoxybutyrate.

This product was dissolved in 100 ml of acetic acid, to which 100 ml of 3N hydrochloric acid was added, and the mixture was heated at an inner temperature of 80-100°C for 8 hours with stirring. After completion of the reaction, the mixture was concentrated under reduced pressure. Distillation under reduced pressure of the residue (b.p. 95-100°C/4 mmHg) gave 4.3 g of (2S,3R)-2,3-dimethoxybutyric acid.

The IR and $^1$H-NMR spectral data of this compound are shown in te following.

IR $\nu_{max}^{neat}$ $cm^{-1}$ : 1740
$^1$H-NMR (90 MHz, $CDCl_3$)
δ: 1.2-1.4 (6H, m), 3.38 (3H, s), 3.50 (3H, s), 3.75-3.85 (2H, m), 8.7 (1H, broad)

(ii) Esterification

The compound obtained in (i), 1.78 g, was dissolved in 150 ml of benzene, to which was added 15.2 g of oxalyl chloride. The mixture was heated for 3 hours with stirring while the inner temperature was kept at 50°C. The reaction mixture was concentrated under reduced pressure, and the residue was mixed with benzene and concentrated again under reduced pressure. Distillation under reduced pressure (b.p. 85-90°C/30 mmHg) of the residue gave 1.2 g of a corresponding acid chloride.

IR $\nu_{max}^{neat}$ $cm^{-1}$ : 1800
The solution of the acid chloride in dry tetrahydrofuran (50 ml) was added dropwise to a solution of 2.1 g of 4-(4′-octyloxy-4-biphenyloxycarbonyl)phenol and 0.8 g of pyridine in dry tetrahydrofuran (80 ml) at room temperature with stirring, and the mixture was stirred at room temperature overnight. Insoluble materials were filtered off, the filtrate was concentrated under reduced pressure, and the residue was subjected to column chromatography (silica gel, developing solvent: chloroform) for separation and purification. The crude crystals obtained were recrystallized from hot methanol to give 0.65 g of the title compound.

The IR and 1H-NMR spectral data and elemental analysis of the title compound are shown in the following.

IR $\nu_{max}^{KBr}$ $cm^{-1}$ : 1770, 1740, 1605, 1500
$^1$H-NMR (90 MHz, $CDCl_3$)
δ: 0.75-1.0 (3H, t), 1.1-1.5 (13H, m), 1.7-1.9 (2H, t), 3.44 (3H, s), 3.58 (3H, s), 3.8-41 (4H, m), 6.8-8.3 (12H, m)
Elemental analysis

| | | |
|---|---|---|
| Calcd. for $C_{33}H_{40}O_7$ (548.67): | C, 72.24; | H, 7.35 |
| Found : | C, 72.46; | H, 7.44 |

Example 43

Preparation of 4-(4′-octyloxy-4-biphenylcarbonyloxy)phenyl ester of (2S,3R)-2,3-dimethoxybutyric acid (a compound of the general formula I′ in which $R_1$ is n-$C_8H_{17}$, $R_2$ is $OCH_3$, $R_3$ is $CH_3$, $R_4$ is $CH_3$, $Q_1$ and $Q_2$ are both -O-, $Q_3$ is -O-, X is a single bond, and

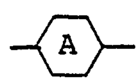 ,

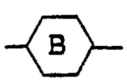 and 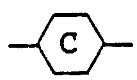 are all 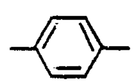).

The (2S,3R)-2,3-dimethoxybutyric acid obtained in Example 42-(i), 0.74 g, and 2.09 g of 4-(4′-octyloxy-4-biphenylcarbonyloxy)phenol were dissolved in 30 ml of dry tetrahydrofuran, to which 0.95 g of pyridine was added. Then 1.53 g of 2-chloro-1-methylpyridinium iodide was added and the mixture was refluxed for 4 hours. After cooling, 100 ml of water was added followed by extraction with 250 ml of chloroform. The organic layer was washed with water and dried with sodium sulfate, and the solvent was distilled off under reduced pressure. The residue was subjected to column chromatography (silica gel, developing solvent: chloroform) for separation and purification. Recrystallization from hot methanol gave 0.30 g of the title compound.

The results of elemental analysis of the product are shown in the following.

Elemental analysis

| | | |
|---|---|---|
| Calcd. for $C_{33}H_{40}O_7$ (548.67): | C, 72.24; | H, 7.35 |
| Found : | C, 71.75; | H, 7.37 |

Examples 44 and 45

The same esterification reaction as in Example 42 (ii) was effected using the (2S,3R)-2,3-dimethoxybutyric acid obtained in Example 42 (i) and skeletal component compounds, to obtain corresponding compounds of Examples 44 and 45.

The phase transition temperatures and spontaneous polarizations of the compounds synthesized in Examples are listed in Table 1. Observation of the phases and measurement of the phase transition temperatures were based on the texture observation with a polarizing microscope together with DSC.

Spontaneous polarization was measured by the Sowyer-Tower method described in Japanese Journal of Applied Physics, 24, 1389-1393 (1985). The values of spontaneous polarization were obtained as a temperature lower by 10 °C than the upper limit temperature of chiral smectic C phase.

The phases such as liquid crystal phase are shown by the following abbreviations.

| | |
|---|---|
| $I_{SO}$: isotropic phase<br>$S_A$ : smectic A phase<br>$Sc^*$: chiral smectic C phase | Ch: cholesteric phase<br>K : crystalline phase |

In Table 1, the mark ← in phase transition temperature column denotes phase transition during cooling.

Table 1

| Example No. | Chemical structure | Phases and phase transition temperatures (°C) | Spontaneous polarization ($nC/cm^2$) |
|---|---|---|---|
| 1 | 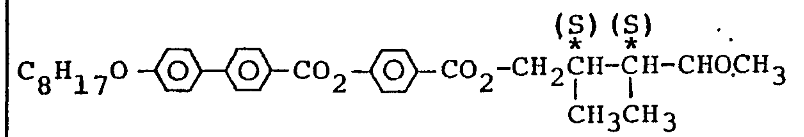 | K —63.2— Sc* —131.4— $S_A$ —167.2— Iso | 3.5 |
| 2 | 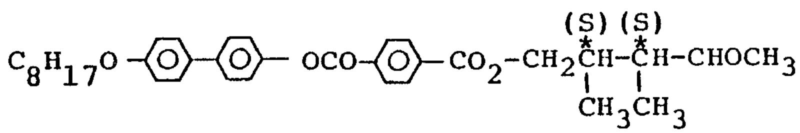 | K —74.8— S —119.0— $S_A$ —146.8— Iso | N.D. |
| 3 | 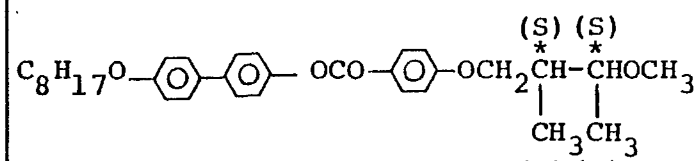 | K —117.8— Ch —133.4— Iso | N.D. |
| 8 | 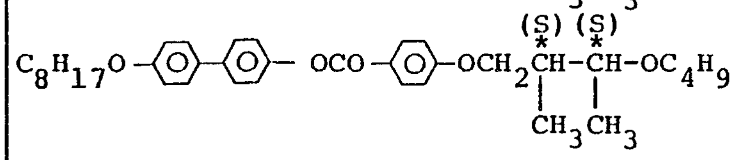 | K —105.5— Ch —129.9— Iso; Ch → Sc* 104.0 | 30 |
| 4 | 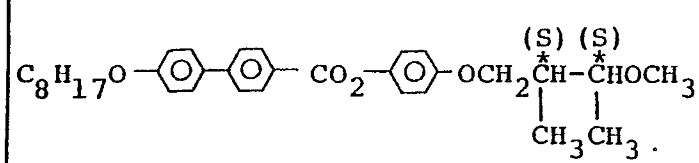 | K —59.3— S —83.1— Sc* —114.0— $S_A$ —158.8— Iso | 14 |

- Cont'd -

Table 1 (Cont'd)

| Example No. | Chemical structure | Phases and phase transition temperatures (°C) | Spontaneous polarization (nC/cm²) |
|---|---|---|---|
| 5 | 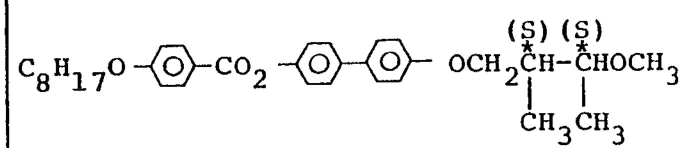 | $K \xrightarrow{75.5} Sc^{*} \xrightarrow{94.3} Ch \xrightarrow{132.5} Iso$ | 20 |
| 7 | 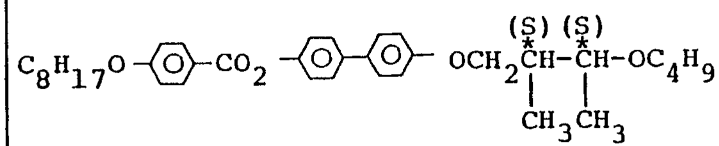 | $K \xrightarrow{43.8} S \xrightarrow{66.9} Sc^{*} \xrightarrow{104.4} Ch \xrightarrow{129.0} Iso$ | 14.5 |
| 6 | 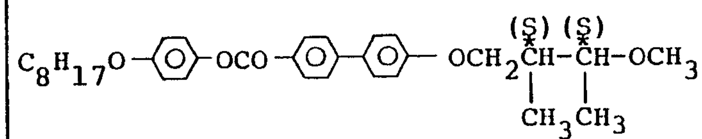 | $K \xrightarrow{75.1} Sc^{*} \xrightarrow{98.4} Ch \xrightarrow{131.4} Iso$ | 27 |
| 9 | 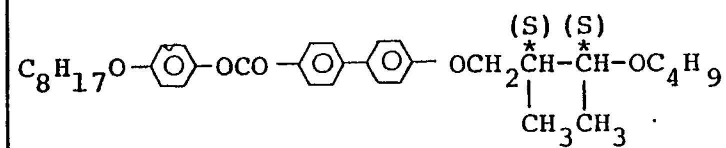 | $K \xrightarrow{54.1} Sc^{*} \xrightarrow{107.4} Ch \xrightarrow{127.6} Iso$ | 8.5 |
| 10 | 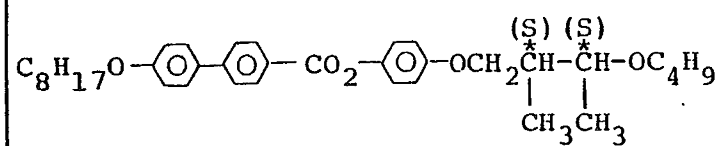 | $K \xrightarrow{33.4} S \xrightarrow{81.3} Sc^{*} \xrightarrow{92.7} S_A \xrightarrow{152.5} Iso$ | 4 |

- Cont'd -

Table 1 (Cont'd)

| Example No. | Chemical structure | Phases and phase transition temperatures (°C) | Spontaneous polarization (nC/cm2) |
|---|---|---|---|
| 11 | 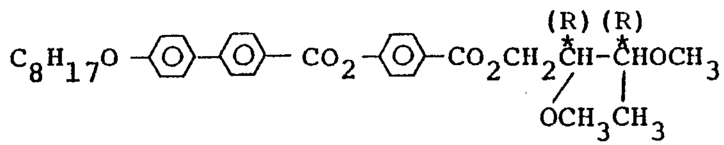 | $K \xrightarrow{67.0} Sc^* \xrightarrow{118.4} S_A \xrightarrow{155.9} Iso$ | 1 |
| 12 | 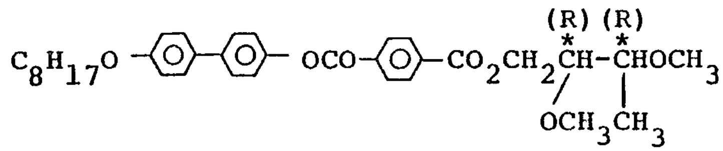 | $K \xrightarrow{75.4} S \xrightarrow{112.6} S_A \xrightarrow{135.8} Iso$ | N.D. |
| 13 | 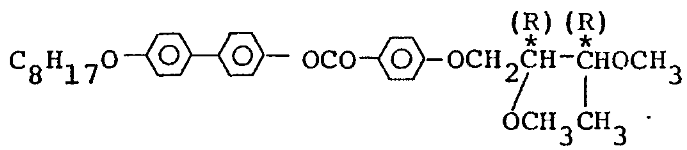 | $K \xrightarrow{99.3} Ch \xrightarrow{114.8} Iso$ | N.D. |
| 14 | 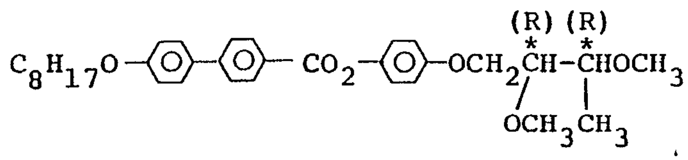 | $K \xrightarrow{29.1} S \xrightarrow{69.5} Ch \xrightarrow{117.4} Iso$ | N.D. |
| 15 | 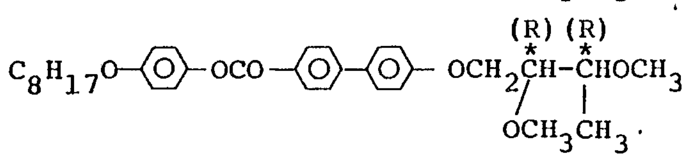 | $K \xrightarrow{39.3} Sc^* \xrightarrow{78.2} Ch \xrightarrow{106} Iso$ | 46 |

- Cont'd -

Table 1 (Cont'd)

| Example No. | Chemical structure | Phases and phase transition temperatures (°C) | Spontaneous polarization (nC/cm2) |
|---|---|---|---|
| 16 | 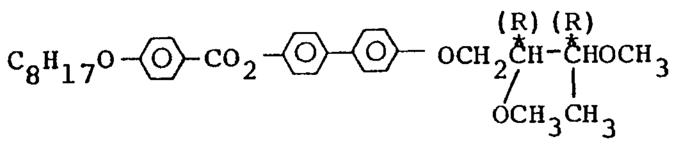 | K —77.2— S —88.5— Ch —112.8— Iso | N.D. |
| 17 | 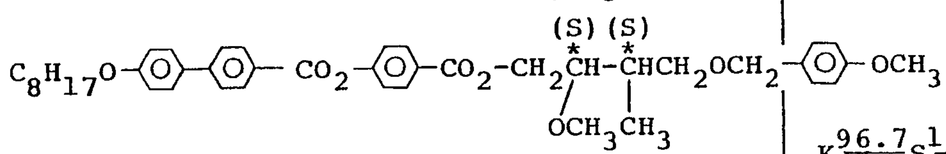 | K —96.7— S —104.7— Ch —111.1— Iso | N.D. |
| 18 | 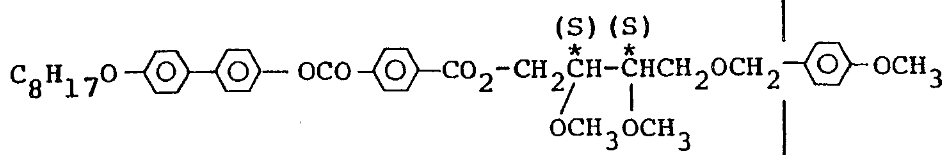 | K —107.0— Iso; Iso —102.5→ S | N.D. |
| 19 | 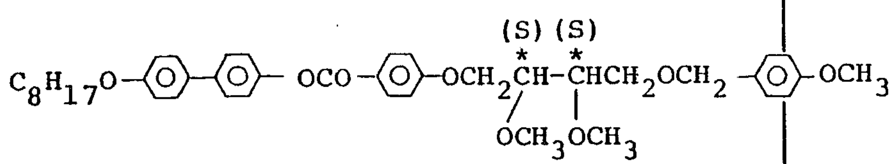 | K —51.4— Ch —71.3— Iso; Ch —40.0→ S | N.D. |

– Cont'd –

Table 1 (Cont'd)

| Example No. | Chemical structure | Phases and phase transition temperatures (°C) | Spontaneous polarization (nC/cm²) |
|---|---|---|---|
| 20 | 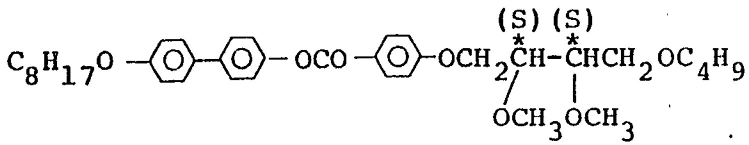 | $K \xrightarrow{79.8} Iso$ | N.D. |
| 21 | 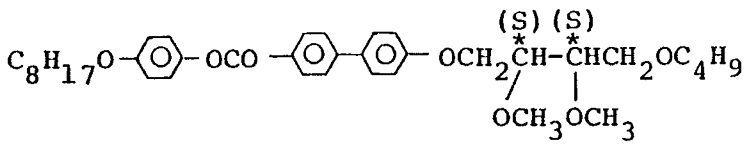 | $K \xrightarrow{40.8} Sc^* \xrightarrow{45.4} Iso$ | 13 |
| 22 | 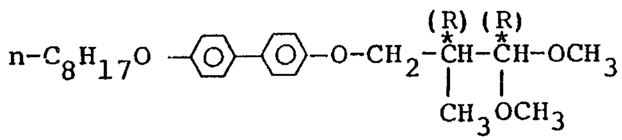 | $K \underset{33.5}{\overset{24.9}{\rightleftarrows}} Iso$ | N.D. |
| 23 | 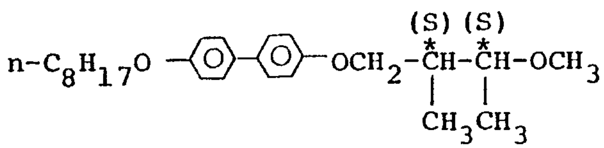 | $K \underset{53.9}{\overset{37.5}{\leftrightarrows}} Iso$ | N.D. |
| 24 | 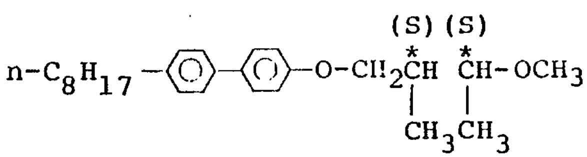 | $K \underset{40.2}{\overset{26.0}{\leftrightarrows}} Iso$ | N.D. |

- Cont'd -

Table 1 (Cont'd)

| Example No. | Chemical structure | Phases and phase transition temperatures (°C) | Spontaneous polarization (nC/cm²) |
| --- | --- | --- | --- |
| 25 | 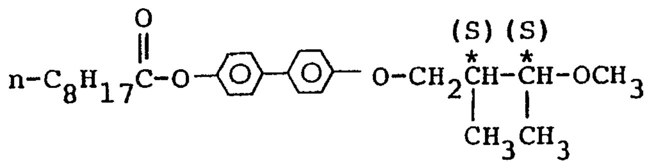 | K 27.5 47.0 Iso | N.D. |
| 26 | 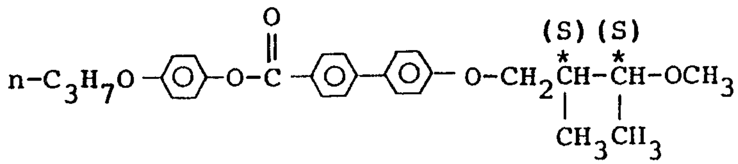 | K 133.0 Sc* 144.0 Iso 144.0 | N.D. |
| 27 | 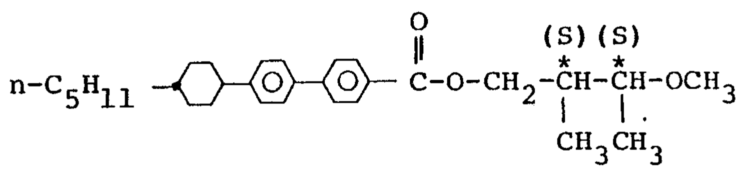 | K 34.8 $S_2$ 66.5 $S_1$ 123.3 Iso | N.D. |
| 28 | 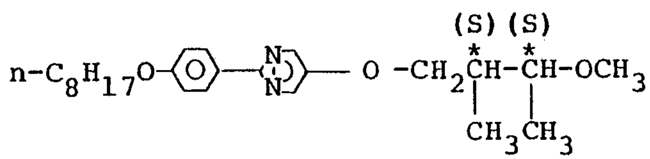 | K 27.4 58.0 Iso | N.D. |

- Cont'd -

Table 1 (Cont'd)

| Example No. | Chemical structure | Phases and phase transition temperatures (°C) | Spontaneous polarization ($nC/cm^2$) |
|---|---|---|---|
| 29 | 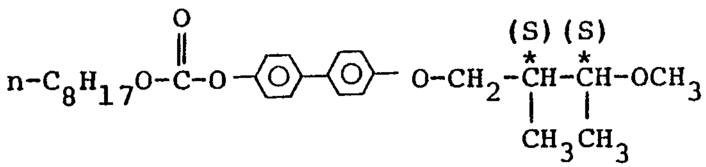 | K 17.1 / 38.9 Iso | N.D. |
| 30 | 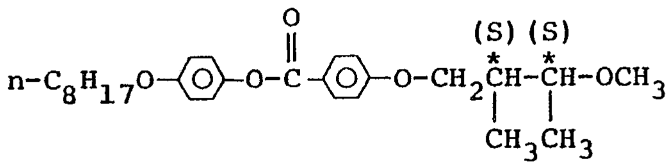 | K -4.6 $S_1$ 2.7 Iso; 34.0 | N.D. |
| 31 | 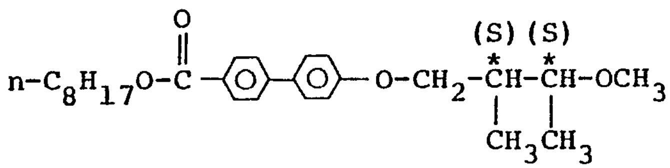 | K -3.2 / 16.1 Iso | N.D. |
| 32 | 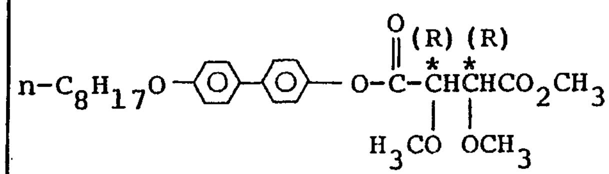 | K 60.2 Iso | N.D. |

- Cont'd -

Table 1 (Cont'd)

| Example No. | Chemical structure | Phases and phase transition temperatures (°C) | Spontaneous polarization ($nC/cm^2$) |
|---|---|---|---|
| 33 | 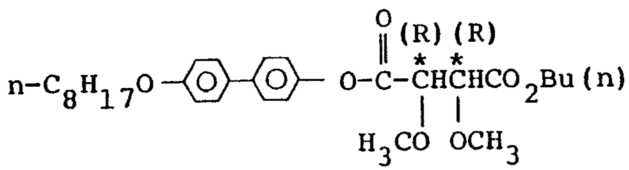 | K —— Iso | N.D. |
| 34 | 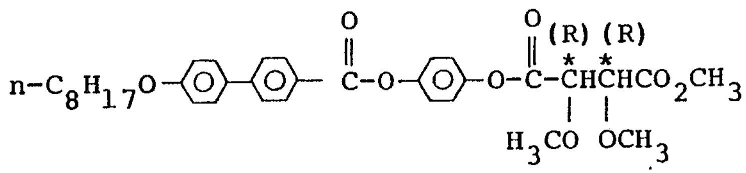 | K —113.9— $S_A$ —155.1— Ch —158.7— Iso; $S_A$ —81.9→ Sc* | 17 |
| 35 | 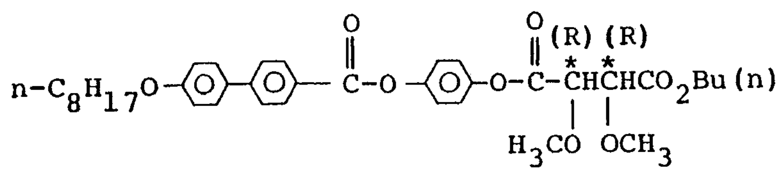 | K —63.1— Sc* —72.3— $S_A$ —134.2— Iso | 50 |
| 36 | 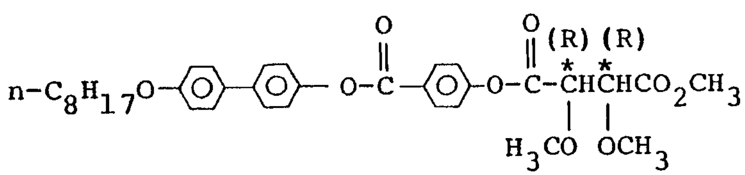 | K —131.0— Ch —151.3— Iso | N.D. |

- Cont'd -

Table 1 (Cont'd)

| Example No. | Chemical structure | Phases and phase transition temperatures (°C) | Spontaneous polarization ($nC/cm^2$) |
|---|---|---|---|
| 37 | 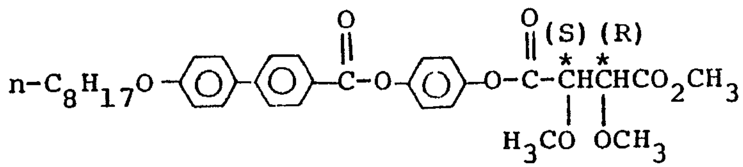 | K —71.3— Sc* —81.0— $S_A$ —137.2— Iso | 16 |
| 38 | 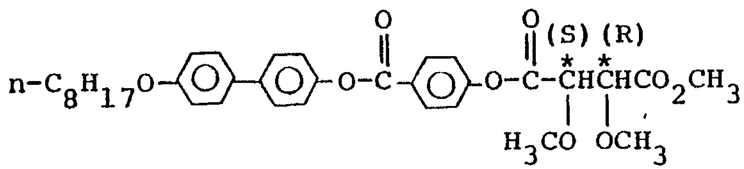 | K —83.0— S —117.4— Ch —120.6— Iso | N.D. |
| 39 | 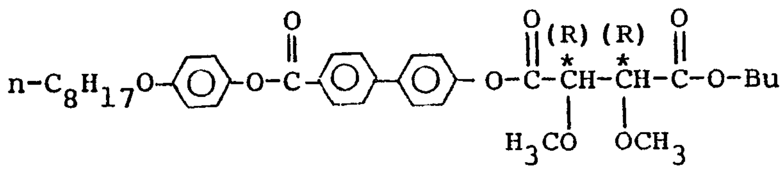 | K —62.5— Sc* —79.1— $S_A$ —81.5— Ch —120.0— Iso | 39 |
| 40 | 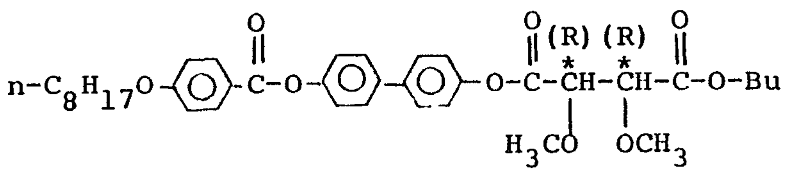 | K —88.8— Ch —129.1— Iso | N.D. |

- Cont'd -

Table 1 (Cont'd)

| Example No. | Chemical structure | Phases and phase transition temperatures (°C) | Spontaneous polarization (nC/cm²) |
|---|---|---|---|
| 41 | 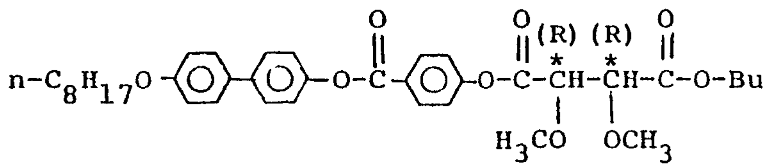 | K —104.3— Ch —132.2— Iso | N.D. |
| 42 | 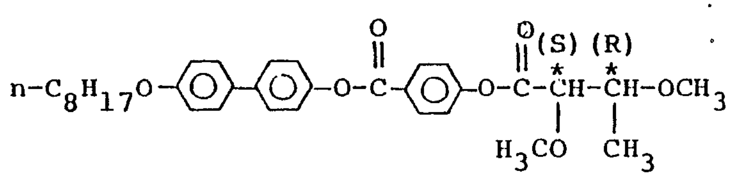 | K —99.2— S —106.2— Sc* —111.7— Ch —133.6— Iso | 40 |
| 43 | 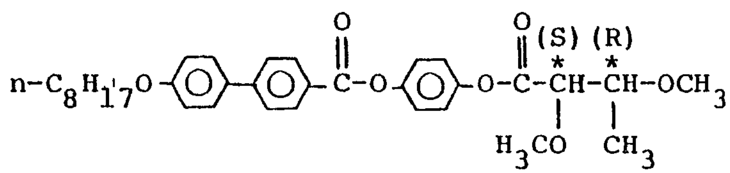 | K —90.0— S —93.8— Sc* —104.0— $S_A$ —150.5— Iso | N.D. |
| 44 | 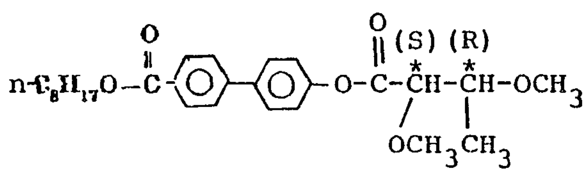 | K —29.3→ Iso | N.D. |

- Cont'd -

Table 1 (Cont'd)

| Example No. | Chemical structure | Phases and phase transition temperatures (°C) | Spontaneous polarization (nC/cm2) |
|---|---|---|---|
| 45 | 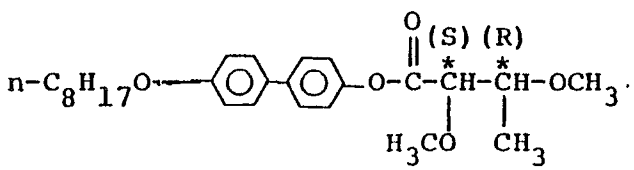 | K ⇄ Iso (30.7 ←, 39.7 →) | N.D. |

Remarks: N.D. means that the spontaneous polarization has not been determined.

Application Example

The optically active compounds of the present invention shown in Table 1 were incorporated into the known ferroelectric compounds A to D (hereinafter referred to as mother liquid crystals) shown in Table 2, in given amounts, to prepare liquid crystal compositions shown in Table 3. Each liquid crystal composition was sealed in a cell constituted by (a) two glass substrates each with a transparent electrode, obtained by spin coating of a polyimide in a film thickness of 500 Å and subsequent rubbing and (b) a spacer consisting of a polyethylene terephthalate film of 6 µm in thickness, whereby ferroelectric liquid crystal devices were prepared. A rectangular wave (10 Hz, 40 Vp-p) was applied to the liquid crystal devices at a given temperature, and their optical responses were observed by a polarizing microscope. The results are shown in Table 3. All the compositions using the optically active compounds of the present invention showed a good optical response. Incidentally, the mother liquid crystals alone showed no optical response even when the applied voltage was increased.

Table 2

| Mother liquid crystal | Chemical structure |
|---|---|
| A | 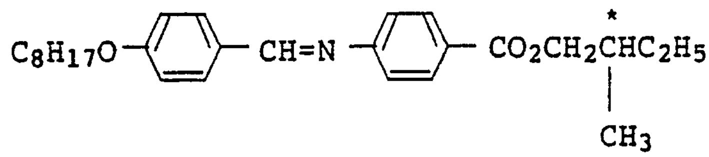<br>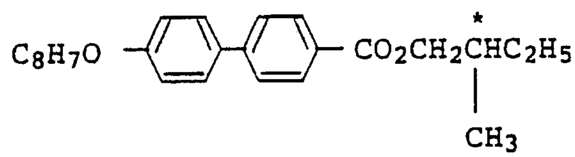<br>(Equimolar mixture) |
| B | 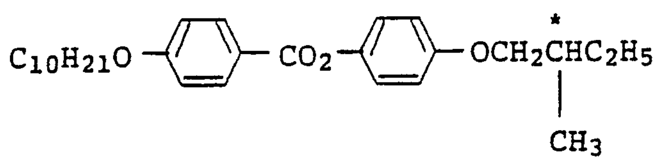 |
| C | 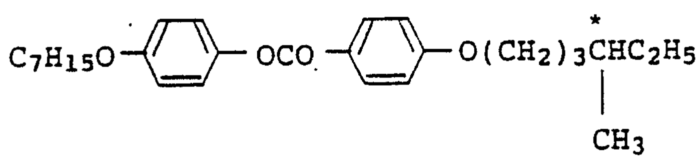<br>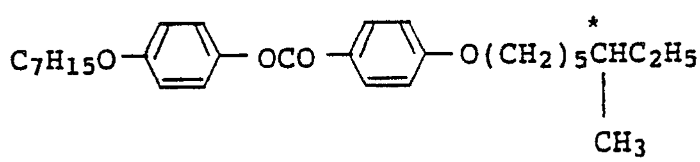<br>(Equimolar mixture) |
| D | 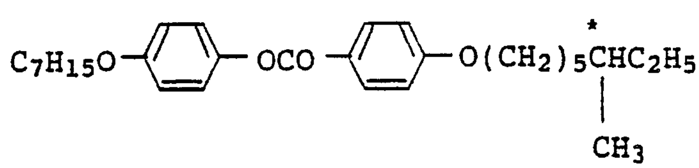 |

Table 3

| Mother liquid crystal | Optically active compound I of the present invention | | Spontaneous polarization ($nC/cm^2$) | Optical response |
|---|---|---|---|---|
| | Example No. | Amount added (wt.%) | | |
| D | 1 | 20 | - | Yes (40°C) |
| D | 8 | 10 | 5 | Yes (40°C) |
| D | 7 | 10 | - | Yes (30°C) |
| D | 6 | 20 | 7 | Yes (40°C) |
| D | 15 | 20 | 10 | Yes (25°C) |
| D | 14 | 10 | - | Yes (40°C) |
| D | 21 | 20 | - | Yes (30°C) |
| D | 19 | 20 | - | Yes (30°C) |
| D | - | - | - | No (40°C) |

Table 4

| Mother liquid crystal | Optically active compound I of the present invention | | Spontaneous polarization ($nC/cm^2$) | Optical response |
|---|---|---|---|---|
| | Example No. | Amount added (wt.%) | | |
| A | - | - | 4 | No (30°C) |
| A | 35 | 20 | -14 | Yes (30°C) |
| A | 37 | 10 | 6 | Yes (30°C) |
| A | 39 | 10 | 8 | Yes (30°C) |
| A | 42 | 20 | 12 | Yes (30°C) |
| B | - | - | 1 | No (30°C) |
| B | 34 | 20 | 8 | Yes (30°C) |
| C | - | - | <1 | No (30°C) |
| C | 39 | 10 | 9 | Yes (30°C) |
| C | 42 | 20 | 11 | Yes (30°C) |

As is clear from Tables 3 and 4, the conventionally known mother liquid crystals can increase the spontaneous polarization values by incorporating thereinto the optically active compounds of the present invention.

Incidentally, the values of spontaneous polarization shown in Tables 3 and 4 are temperatures lower by 10°C than the upper limit of chiral smectic C phase, and were measured in accordance with the Sowyer-Tower method described in Japanese Journal of Applied Physics, 24, 1389-1393 (1985).

Examples of Composition Preparation

The following liquid compositions were prepared using the compound of Example 1 or the compound of Example 15. Using these compositions, liquid crystal devices as shown in Fig. 1 were prepared. All of these devices showed a good optical response when a rectangular wave (40 Vp-p, 10 Hz) was applied at room temperature. Further, each composition was mixed with 3% by weight of an anthraquinone type or azo type dichroic dye, and guest host type liquid crystal devices were prepared in the same manner as above. All of these devices showed a good optical response of guest host type. Thus, it was found that the compounds of the present invention are useful as a component of liquid crystal compositions.

Liquid crystal composition 1

| Component | Amount (mole%) |
|---|---|
| 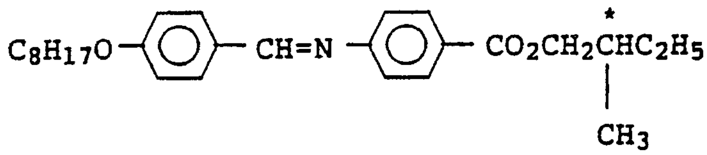 | 18 |
| 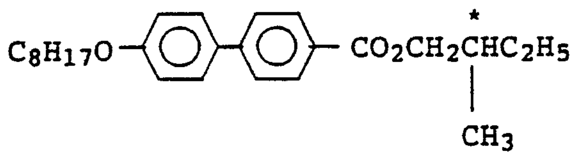 | 20 |
| 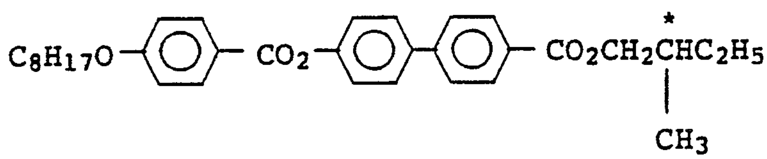 | 26 |
| 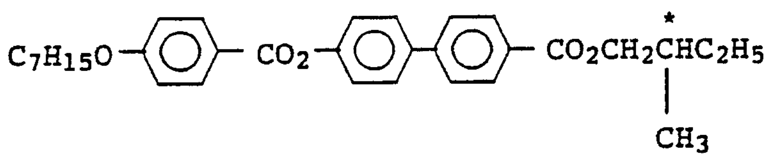 | 27 |
| Compound of Example 9 | 9 |

Liquid crystal composition 2

| Component | Amount (mole%) |
|---|---|
| 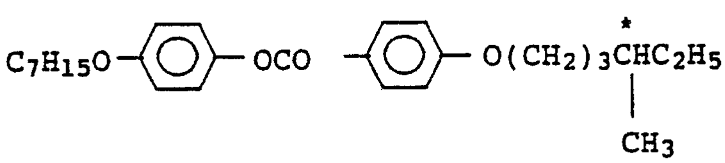 | 32 |
| 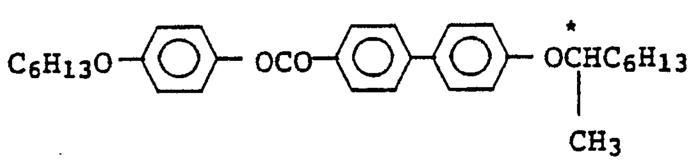 | 33 |
| 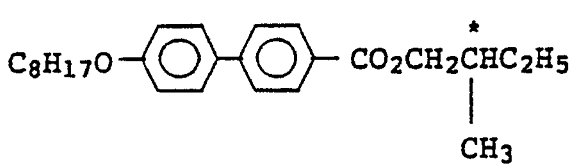 | 15 |
| Compound of Example 15 | 20 |

Liquid crystal composition 3

| Component | Amount (mole%) |
|---|---|
| 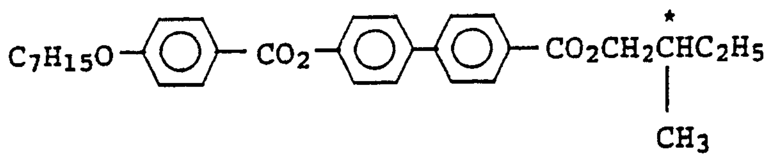 | 15 |
| Compound of Example 9 | 25 |
| 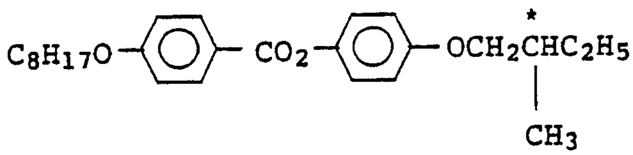 | 35 |
| 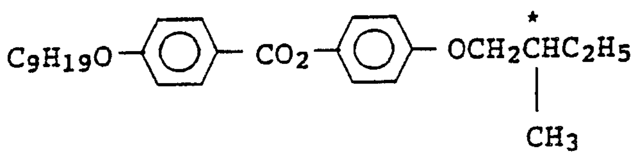 | 25 |

Liquid crystal composition 4

| Component | Amount (mole%) |
|---|---|
| 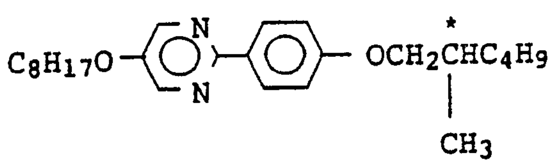 | 30 |
| 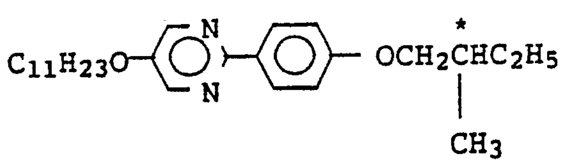 | 30 |
| 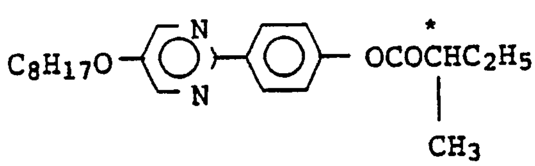 | 30 |
| Compound of Example 15 | 10 |

As is clear from Examples and Application Examples, the present invention provides liquid crystal compounds and liquid crystal compositions having a large spontaneous polarization and showing a chiral

smectic C phase. Accordingly, the optically active compounds of the present invention and the liquid crystal compositions containing these compounds are useful as a liquid crystal to be employed in optical modulators such as liquid crystal display apparatuses and can provide such apparatuses having excellent capabilities in response, etc.

## Claims

1. Optically active compounds represented by the general formula I:

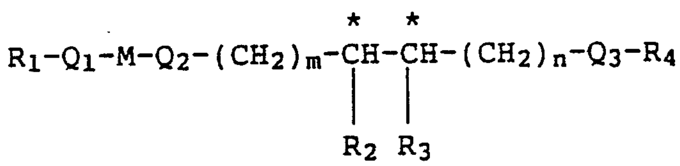

[I]

wherein $R_1$ is an alkyl group of 3-14 carbon atoms; $R_2$ and $R_3$, which may be the same or different, are independently a lower alkyl group of 1-3 carbon atoms or a lower alkoxy group of 1-3 carbon atoms; $R_4$ is an alkyl group of 1-10 carbon atoms which may be substituted with a phenyl group which may be substituted with a lower alkyl or alkoxy group of 1-4 carbon atoms; m and n are independently 0 or 1; $Q_1$ is a single bond, an ether group, a carbonyl group, a carbonyldioxy group or a carboxylic acid ester group; $Q_2$ is a single bond, an ether group, a carbonyl group or a carboxylic acid ester group; $Q_3$ is an ether group, a carbonyl group or a carboxylic acid ester group; M is

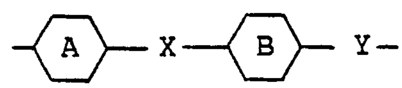

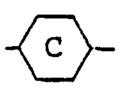

or

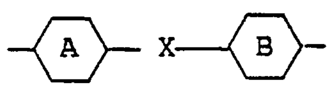

;

(X and Y are independently a single bond, a carboxylic acid ester group, a methyleneoxy group or an ethylene group, and

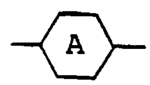

,

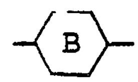

and

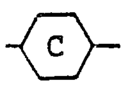

are independently a homocyclic or heterocyclic six-membered ring-1,4-diyl group which may contain 1-2 oxygen or nitrogen atoms as ring-forming atoms); when m is 0, $R_2$ is a lower alkoxy group of 1-3 carbon atoms, $R_4$ is an alkly group of 1-10 carbon atoms, and $Q_2$ is a single bond, a carbonyl group or a carboxylic acid ester group represented by

$-O-\overset{O}{\overset{\|}{C}}-$ ; the carbon atoms with the asterisk (*) denote asymmetric carbon atoms.

2. Optically active compounds according to Claim 1, wherein $R_2$ is a lower alkoxy group of 1-3 carbon atoms; $R_4$ is an alkyl group of 1-10 carbon atoms; m and n are both 0; $Q_1$ is a single bond, an ether group, a carbonyl group or a carboxylic acid ester group; $Q_2$ is a carbonyl group or a carboxylic acid ester group represented by

$-O-\overset{O}{\overset{\|}{C}}-$ ; M is

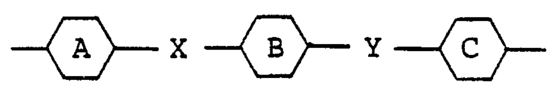

or

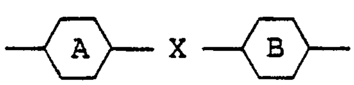

(X and Y are independently a single bond, a carboxylic acid ester group, a methyleneoxy group or an ethylene group, and

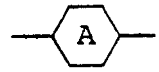 ,

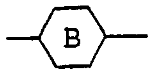 and 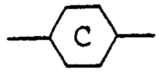

are independently a homocyclic or heterocyclic six-membered ring-1,4-diyl group which may contain 1-2 oxygen or nitrogen atoms as ring-forming atoms).

3. Optically active compounds according to Claim 1, wherein m is 1, n is 0 or 1, $Q_2$ and $Q_3$ which may be the same or different, are independently an ether group, a carbonyl group or a carboxylic acid ester group, and M is

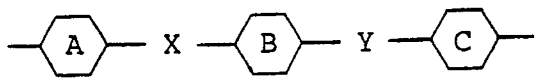 or

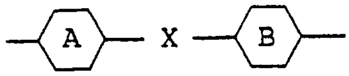

(X and Y are independently a single bond, a carboxylic acid ester group, a methyleneoxy group or an ethylene group, and

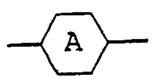 , 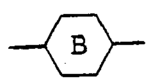 and 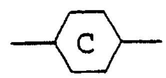

are independently a homocyclic or heterocyclic six-membered ring-1,4-diyl group which may contain 1-2 oxygen or nitrogen atoms as ring-forming atoms).

4. Optically active compounds according to any of Claims 1-3, wherein

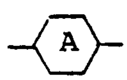 , 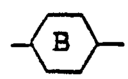 and 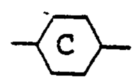

in M, which may be the same or different, are independently p-phenylene, 1,4-cyclohexylene, 2,5-(1,3-dioxane)diyl, 2,5-pyridinediyl, 2,5-pyrimidinediyl, 2,5-(1,4-pyrazine)diyl or 3,6-(1,2-pyridazine)diyl.

5. Optically active compounds according to Claim 1, wherein $R_3$ is a lower alkyl or alkoxy group of 1-3 carbon atoms, $Q_2$ is a carboxylic acid ester group represented by $-O-\overset{O}{\overset{\|}{C}}-$, $Q_3$ is an ether group when $R_3$ is a lower group, and is a carboxylic acid ester group when $R_3$ is a lower alkoxy group.

6. Optically active compounds according to Claim 1 or 3, wherein $R_2$ is a lower alkyl or alkoxy group of 1-3 carbon atoms, $R_3$ is an alkyl group of 1-3 carbon atoms, and $Q_3$ is an ether group.

7. Optically active compounds according to Claim 1 or 3, wherein $R_2$ and $R_3$ are independently a lower alkoxy group of 1-3 carbon atoms, n is 1, and $Q_3$ is an ether group.

8. Optically active compounds according to any of Claims 1-4, wherein M is

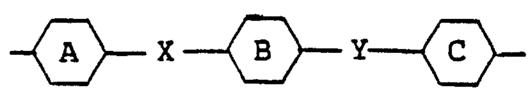 .

9. Optically active compounds according to Claim 8, wherein one of X and Y in M is a single bond, the other is a carboxylic acid ester group, and all of

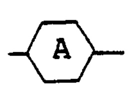 , 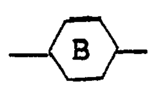 and 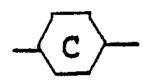

are p-phenylene.

10. Optically active compounds according to any of Claims 1-3, wherein M is

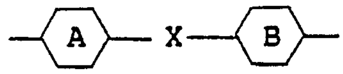 .

11. Optically active compounds according to Claim 10, wherein X is a single bond and both of

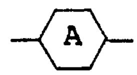 and

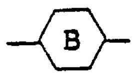

are p-phenylene.

12. Liquid crystal compositions comprising at least one of the compounds according to any of Claims 1-11.

13. Liquid crystal optical modulators containing at least one of the liquid crystal compositions according to Claim 12.

14. Process for preparing compounds according to anyone of claims 1 to 11, **characterized** in that - for preparing compounds of formula I, wherein $Q_2$ is a carboxylic ester group,
a compound of formula (II)
$R_1$-$Q_1$-M-COOH (II)
is reacted in a manner known per se with a compound of formula (III)

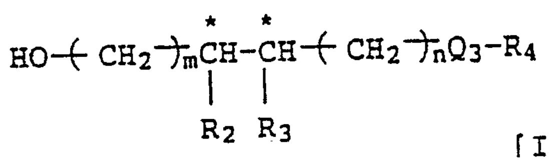

[III]

wherein $R_1$ to $R_4$, m,n,$Q_1$,$Q_3$ and M are defined as in claim 1, in an organic solvent in presence of a proton acid, - for preparing compounds of formula I, wherein $Q_2$ is an ether group, a compound of formula (IV)
$R_1$-$Q_1$-M-OH (IV)
is reacted in a manner known per se with a compound of formula (III)

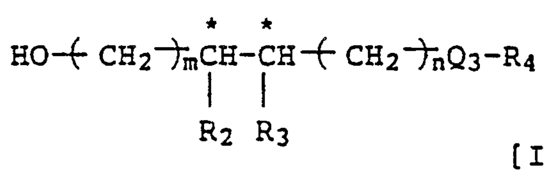

[III]

- for preparing compounds of formula (I), wherein $Q_2$ is a carboxylic ester group, a compound of formula (IV)
$R_1$-$Q_1$-M-OH (IV)
is reacted in a manner known per se with a compound of formula (V)

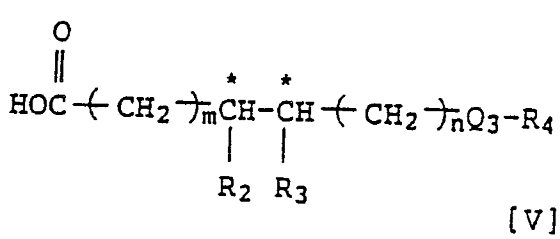

[V]

in an organic solvent in presence of a proton acid, - for preparing compounds of formula (I), wherein $Q_2$ is a carbonyl group, a compound of formula (VI)

$R_1$-$Q_1$-M-H (VI)

is reacted in a manner known per se with a compound of formula (V)

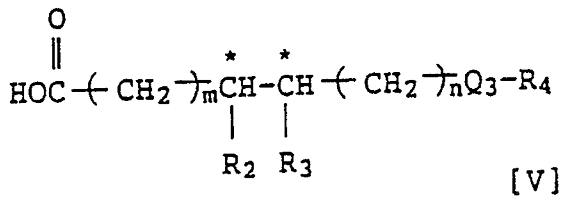

[V]

in the presence of a proton acid.

FIG. 1

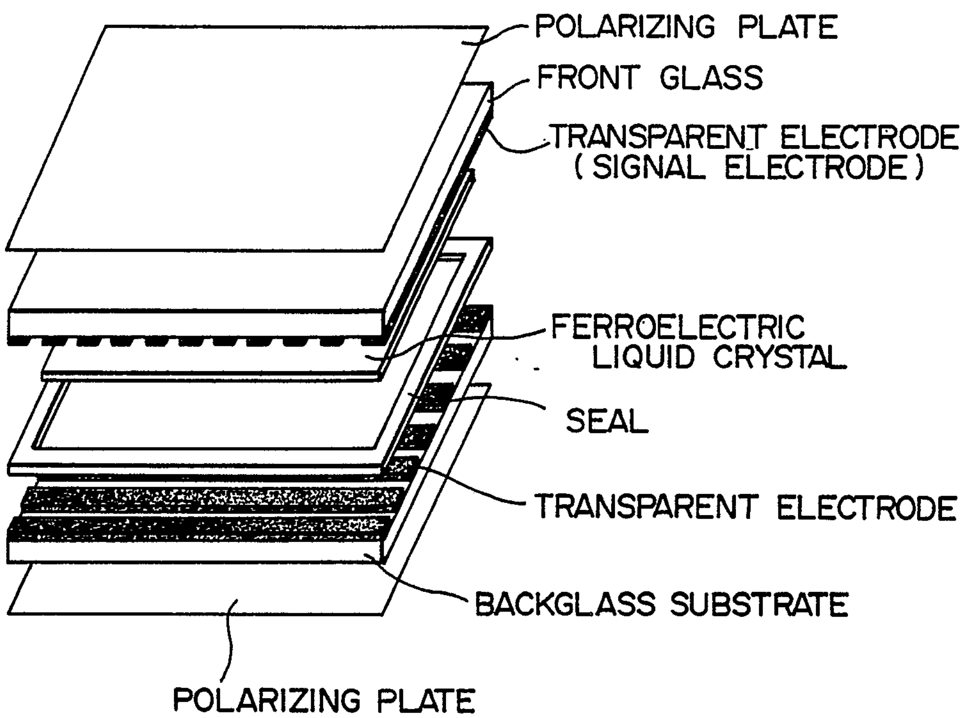


EXAMPLE OF LIQUID CRYSTAL DEVICE
(SCHEMATIC ILLUSTRATION)